# EUROPEAN PATENT SPECIFICATION

(11) **EP 4 218 754 B1**
(45) Date of publication and mention of the grant of the patent: **25.03.2026**
(21) Application number: 23154663.1
(22) Date of filing: 13.11.2019
(51) Int. Cl.: A61K 31/404, A61K 45/06, A61K 31/47, A61P 11/00

(54) **VANZACAFTOR FOR USE IN THE TREATMENT OF CYSTIC FIBROSIS**
VANZACAFTOR ZUR VERWENDUNG BEI DER BEHANDLUNG VON MUKOVISZIDOSE
VANZACAFTOR POUR LE TRAITEMENT DE LA MUCOVISCIDOSE

(30) Priority: 14.11.2018 US 201862767202 P
(43) Date of publication of application: 02.08.2023
(62) Divisional of application: 19817037.5
(73) Proprietor: Vertex Pharmaceuticals Incorporated, Boston, MA 02210 (US)
(72) Inventor: ALTSHULER, David M., Boston, 02210 (US); ANDERSON, Corey Don, Boston, 02210 (US); CHEN, Weichao George, Boston, 02210 (US); CLEMENS, Jeremy J, Boston, 02210 (US); CLEVELAND, Thomas, Boston, 02210 (US); COON, Timothy Richard, Boston, 02210 (US); FRIEMAN, Bryan, Boston, 02210 (US); GROOTENHUIS, Peter, deceased (US); HADIDA RUAH, Sara Sabina, Boston, 02210 (US); HARE, Brian J., Boston, 02210 (US); KEWALRAMANI, Reshma, Boston, 02210 (US); MCCARTNEY, Jason, Boston, 02210 (US); MILLER, Mark Thomas, Boston, 02210 (US); PARASELLI, Prasuna, Boston, 02210 (US); PIERRE, Fabrice, Boston, 02210 (US); ROBERTSON, Sarah M., Boston, 02210 (US); SOSNAY, Patrick R., Boston, 02210 (US); SWIFT, Sara E., Boston, 02210 (US); ZHOU, Jinglan, Boston, 02210 (US); BOREK, Bartlomiej, Boston, 02210 (US); VAN GOOR, Fredrick, Boston, 02210 (US); YOUNG, Tim, Boston, 02210 (US)
(74) Representative: Carpmaels & Ransford LLP

(56) References cited:
- WO-A1-2019/018353
- WO-A1-2019/018395
- WO-A1-2019/152940
- US-A1- 2019 248 809
- ANONYMOUS: "Symdeko in Cystic Fibrosis Patients", CLINICALTRIALS.GOV, 23 April 2018 (2018-04-23), Internet, pages 1 - 9, XP055661778, Retrieved from the Internet <URL:https://clinicaltrials.gov/ct2/show/record/NCT03506061> [retrieved on 20200124]
- SCOTT H. DONALDSON ET AL: "Tezacaftor/Ivacaftor in Subjects with Cystic Fibrosis and F508del / F508del-CFTR or F508del / G551D-CFTR", AMERICAN JOURNAL OF RESPIRATORY AND CRITICAL CARE MEDICINE., vol. 197, no. 2, 20 September 2017 (2017-09-20), US, pages 214 - 224, XP055498649, ISSN: 1073-449X, DOI: 10.1164/rccm.201704-0717OC
- CLINICAL TRIALS US: "A Study to Evaluate the Safety and Efficacy of VX-121 Combination Therapy in Subjects With Cystic Fibrosis -Full Text View -ClinicalTrials Study Details Tabular View Results Submitted Disclaimer How to Read a Study Record A Study to Evaluate the Safety and Efficacy of VX-121 Combination Therapy in S", 30 April 2019 (2019-04-30), XP055903330, Retrieved from the Internet <URL:https://classic.clinicaltrials.gov/ct2/show/record/NCT03912233> [retrieved on 20220321]

## Description

This application claims priority to United States provisional application 62/767,202, filed November 14, 2018.

Disclosed herein is a modulator of Cystic Fibrosis Transmembrane Conductance Regulator (CFTR), pharmaceutical compositions containing the modulator, methods of treatment of cystic fibrosis, and a process for making the modulator.

Cystic fibrosis (CF) is a recessive genetic disease that affects approximately 70,000 children and adults worldwide. Despite progress in the treatment of CF, there is no cure.

In patients with CF, mutations in CFTR endogenously expressed in respiratory epithelia lead to reduced apical anion secretion causing an imbalance in ion and fluid transport. The resulting decrease in anion transport contributes to enhanced mucus accumulation in the lung and accompanying microbial infections that ultimately cause death in CF patients. In addition to respiratory disease, CF patients typically suffer from gastrointestinal problems and pancreatic insufficiency that, if left untreated, result in death. In addition, the majority of males with cystic fibrosis are infertile, and fertility is reduced among females with cystic fibrosis.

Sequence analysis of the CFTR gene has revealed a variety of disease-causing mutations (Cutting, G. R. et al. (1990) Nature 346:366-369; Dean, M. et al. (1990) Cell 61:863:870; and Kerem, B-S. et al. (1989) Science 245:1073-1080; Kerem, B-S et al. (1990) Proc. Natl. Acad. Sci. USA 87:8447-8451). To date, greater than 2000 mutations in the CF gene have been identified. CF mutations are listed in the "Cystic Fibrosis Mutation Database," located at http://www.genet.sickkids.on.ca/app. The most prevalent disease-causing mutation is a deletion of phenylalanine at position 508 of the CFTR amino acid sequence and is commonly referred to as the *F508del* mutation. This mutation occurs in approximately 90% of the cases of cystic fibrosis and is associated with severe disease.

The deletion of residue 508 in CFTR prevents the nascent protein from folding correctly. This results in the inability of the mutant protein to exit the endoplasmic reticulum (ER) and traffic to the plasma membrane. As a result, the number of CFTR channels for anion transport present in the membrane is far less than observed in cells expressing wild-type CFTR, i.e., CFTR having no mutations. In addition to impaired trafficking, the mutation results in defective channel gating. Together, the reduced number of channels in the membrane and the defective gating lead to reduced anion and fluid transport across epithelia. (Quinton, P. M. (1990), FASEB J. 4: 2709-2727). The channels that are defective because of the F508del mutation are still functional, albeit less functional than wild-type CFTR channels. (Dalemans et al. (1991), Nature Lond. 354: 526-528; Pasyk and Foskett (1995), J. Cell. Biochem. 270: 12347-50). In addition to F508del, other disease-causing mutations in CFTR that result in defective trafficking, synthesis, and/or channel gating could be up- or down-regulated to alter anion secretion and modify disease progression and/or severity.

CFTR is a cAMP/ATP-mediated anion channel that is expressed in a variety of cell types, including absorptive and secretory epithelia cells, where it regulates anion flux across the membrane, as well as the activity of other ion channels and proteins. In epithelial cells, normal functioning of CFTR is critical for the maintenance of electrolyte transport throughout the body, including respiratory and digestive tissue. CFTR is composed of 1480 amino acids that encode a protein which is made up of a tandem repeat of transmembrane domains, each containing six transmembrane helices and a nucleotide binding domain. The two transmembrane domains are linked by a large, polar, regulatory (R)-domain with multiple phosphorylation sites that regulate channel activity and cellular trafficking.

Chloride transport takes place by the coordinated activity of ENaC and CFTR present on the apical membrane and the Na⁺-K⁺-ATPase pump and Cl- channels expressed on the basolateral surface of the cell. Secondary active transport of chloride from the luminal side leads to the accumulation of intracellular chloride, which can then passively leave the cell via Cl⁻ channels, resulting in a vectorial transport. Arrangement of Na⁺/2Cl⁻/K⁺ co-transporter, Na⁺-K⁺-ATPase pump and the basolateral membrane K⁺ channels on the basolateral surface and CFTR on the luminal side coordinate the secretion of chloride via CFTR on the luminal side. Because water is probably never actively transported itself, its flow across epithelia depends on tiny transepithelial osmotic gradients generated by the bulk flow of sodium and chloride.

A number of CFTR modulating compounds have recently been identified. However, compounds that can treat or reduce the severity of the cystic fibrosis and other CFTR mediated diseases, and particularly the more severe forms of these diseases, are still needed.
NCT03506061 relates to a clinical trial of Trikafta in Cystic Fibrosis Patients.
SCOTT H. DONALDSON ET AL : "Tezacaftor/Ivacaftor in Subjects with Cystic Fibrosis and F508del / F508del-CFTR or F508del / G551D-CFTR", AMERICAN JOURNAL OF RESPIRATORY AND CRITICAL CARE MEDICINE., vol. 197, no. 2, 20 September 2017 (2017-09-20), pages 214-224, relates to studies to evaluate the safety and efficacy of tezacaftor monotherapy and of tezacaftor/ivacaftor combination therapy in subjects with cystic fibrosis homozygous for *F508del* or compound heterozygous for *F508del* and *G551D.*
WO 2019/018353 A1 relates to methods of treating cystic fibrosis comprising administering at least 'Compound [I] of the fomula' disclosed therein, pharmaceutical compositions containing a pharmaceutically acceptable salt of at least 'Compound [I]' disclosed therein, and methods of treating cystic fibrosis comprising administering a pharmaceutically acceptable salt of at least 'Compound [I]' disclosed therein.
US 2019/248809 A1 relates to compounds of 'Formula (I)' disclosed therein, pharmaceutically acceptable salts thereof, deuterated derivatives of any of the foregoing, and metabolites of any of the foregoing. It also relates to pharmaceutical compositions comprising the same, methods of treating cystic fibrosis using the same, and methods for making the same.
WO 2019/152940 A1 relates to a pharmaceutical composition comprising 'Compound [I]' disclosed therein, and methods of treating cystic fibrosis comprising administering one or more of such pharmaceutical compositions to a patient.
WO 2019/018395 A1 relates to 'Compound I of the formula [I]' disclosed therein, and/or pharmaceutically acceptable salt(s) of 'Compound I' comprised in a pharmaceutical composition and methods of using the same to treat cystic fibrosis.
NCT03912233 relates to a clinical trial to evaluate the safety and efficacy of VX-121 Combination Therapy in Subjects with Cystic Fibrosis

Thus, one aspect of the disclosure provides a CFTR modulating compound, (14S)-8-[3-(2-{Dispiro[2.0.2.1]heptan-7-yl}ethoxy)-1H-pyrazol-1-yl]-12,12-dimethyl-2λ6-thia-3,9,11,18,23-pentaazatetracyclo [17.3.1.111,14.05,10]tetracosa-1(22),5,7,9,19(23),20-hexaene-2,2,4-trione (Compound **I**) and pharmaceutically acceptable salts thereof. Compound **I** can be depicted as having the following structure:

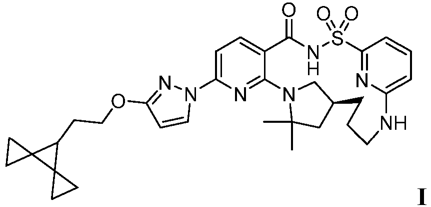

Other aspects of the disclosure provide pharmaceutical compositions comprising Compound **I** and/or at least one pharmaceutically acceptable salt thereof, which compositions may further include at least one additional active pharmaceutical ingredient and/or at least one carrier. Yet other aspects of the disclosure are methods of treating the CFTR-mediated disease cystic fibrosis comprising administering Compound **I** and/or at least one pharmaceutically acceptable salt thereof, optionally as part of a pharmaceutical composition comprising at least one additional component, to a subject in need thereof. A further aspect of the disclosure provides processes of making Compound **I** and/or pharmaceutically acceptable salts thereof is also disclosed.

One embodiment provides a method of treating the CFTR-mediated disease cystic fibrosis comprising administering (14.5)-8-[3-(2-{Dispiro[2.0.2.1]heptan-7-yl}ethoxy)-1H-pyrazol-1-yl]-12,12-dimethyl-2λ6-thia-3,9,11,18,23-pentaazatetracyclo [17.3.1.111,14.05,10]tetracosa-1(22),5,7,9,19(23),20-hexaene-2,2,4-trione (Compound **I**), alone or in combination with (R)-1-(2,2-difluorobenzo[d][1,3]dioxol-5-yl)-N-(1-(2,3-dihydroxypropyl)-6-fluoro-2-(1-hydroxy-2-methylpropan-2-yl)-1H-indol-5-yl)cyclopropanecarboxamide (Compound **II**), and/or N-[2,4-bis(1,1-dimethylethyl)-5-hydroxyphenyl]-1,4-dihydro-4-oxoquinoline-3-carboxamide (Compound **III**) or *N*-(2-(*tert*-butyl)-5-hydroxy-4-(2-(methyl-d3)propan-2-yl-1,1,1,3,3,3-d6)phenyl)-4-oxo-1,4-dihydroquinoline-3-carboxamide (Compound III-d). In certain embodiments, the method of treating the CFTR-mediated disease cystic fibrosis comprises administering Compound I in combination with Compound **III or III-d** and 3-(6-(1-(2,2-difluorobenzo[d][1,3]dioxol-5-yl)cyclopropanecarboxamido)-3-methylpyridin-2-yl)benzoic acid (Compound IV). In some embodiments, Compound I is administered in the same composition with Compound II and Compound III. In some embodiments, Compound I is administered in the same composition with Compound II and Compound III-d. In some embodiments, Compound I is administered in the same composition with Compound III and Compound IV. In some embodiments, Compound I is administered in the same composition with Compound **III-d** and Compound IV. In some embodiments, a composition comprising Compound I is co-administered with a separate composition comprising Compound II and/or Compound III. In some embodiments, a composition comprising Compound I is co-administered with a separate composition comprising Compound II and/or Compound III-d. In some embodiments, a composition comprising Compound I is co-administered with a separate composition comprising Compound III and Compound IV. In some embodiments, a composition comprising Compound I is co-administered with a separate composition comprising Compound **III-d** and Compound IV.

### Definitions

"Compound I" as used throughout this disclosure refers to (14*S*)-8-[3-(2-{Dispiro[2.0.2.1]heptan-7-yl}ethoxy)-1H-pyrazol-1-yl]-12,12-dimethyl-2À6-thia-3,9,11,18,23-pentaazatetracyclo[17.3.1.111,14.05,10]tetracosa-1(22),5,7,9,19(23),20-hexaene-2,2,4-trione, which can be depicted as having the following structure: 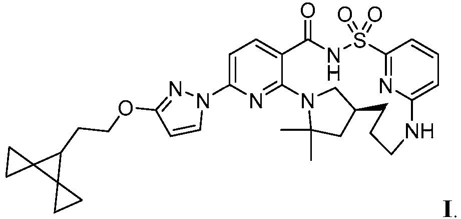 Compound I may be in the form of an isomeric mixture or enantioenriched (e.g., >90% ee, >95% ee, > 98% ee) isomers. Compound I may be in the form of a pharmaceutically acceptable salt.

"Compound **II"** as used herein, refers to (R)-1-(2,2-difluorobenzo[d][1,3]dioxol-5-yl)-*N*-(1-(2,3-dihydroxypropyl)-6-fluoro-2-(1-hydroxy-2-methylpropan-2-yl)-1H-indol-5-yl)cyclopropanecarboxamide, which can be depicted with the following structure: 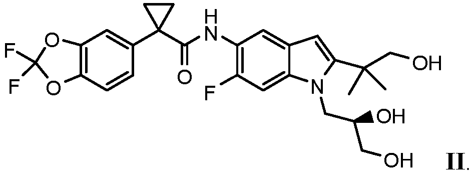 Compound **II** may be in the form of a pharmaceutically acceptable salt.

"Compound **III"** as used throughout this disclosure refers to *N*-(5-hydroxy-2,4-di-tert-butyl-phenyl)-4-oxo-1H-quinoline-3-carboxamide which is depicted by the structure: 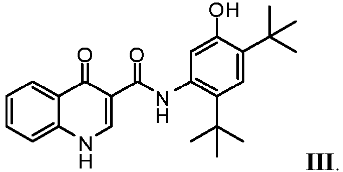 Compound **III** may also be in the form of a pharmaceutically acceptable salt. In some embodiments, a deuterated derivative of Compound **III** (Compound **III-d**) is employed in the compositions and methods disclosed herein. A chemical name for Compound **III-d** is *N*-(2-(*tert*-butyl)-5-hydroxy-4-(2-(methyl-d3)propan-2-yl-1,1,1,3,3,3-d6)phenyl)-4-oxo-1,4-dihydroquinoline-3-carboxamide, as depicted by the structure: 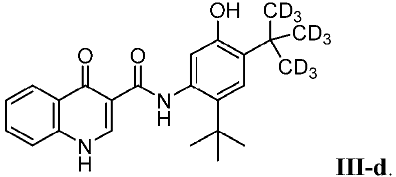 Compound **III-d** may be in the form of a pharmaceutically acceptable salt.

"Compound **IV"** as used herein, refers to 3-(6-(1-(2,2-difluorobenzo[d][1,3]dioxol-5-yl)cyclopropanecarboxamido)-3-methylpyridin-2-yl)benzoic acid, which is depicted by the chemical structure: 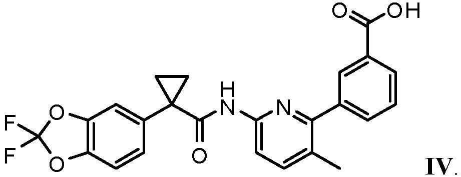 Compound **IV** may be in the form of a pharmaceutically acceptable salt.

As used herein, "CFTR" means cystic fibrosis transmembrane conductance regulator.

As used herein, "mutations" can refer to mutations in the *CFTR* gene or the CFTR protein. A *"CFTR* gene mutation" refers to a mutation in the *CFTR* gene, and a "CFTR protein mutation" refers to a mutation in the CFTR protein. A genetic defect or mutation, or a change in the nucleotides in a gene in general results in a mutation in the CFTR protein translated from that gene, or a frame shift(s).

The term "F508del" refers to a mutant CFTR protein which is lacking the amino acid phenylalanine at position 508.

As used herein, a patient who is "homozygous" for a particular gene mutation has the same mutation on each allele.

As used herein, a patient who is "heterozygous" for a particular gene mutation has the particular mutation on one allele, and a different mutation on the other allele.

As used herein, the term "modulator" refers to a compound that increases the activity of a biological compound such as a protein. For example, a CFTR modulator is a compound that increases the activity of CFTR. The increase in activity resulting from a CFTR modulator includes but is not limited to compounds that correct, potentiate, stabilize and/or amplify CFTR.

As used herein, the term "CFTR corrector" refers to a compound that facilitates the processing and trafficking of CFTR to increase the amount of CFTR at the cell surface. Compounds **I** and **II** disclosed herein are CFTR correctors.

As used herein, the term "CFTR potentiator" refers to a compound that increases the channel activity of CFTR protein located at the cell surface, resulting in enhanced ion transport. Compound **III** and **III-d** disclosed herein are CFTR potentiators. It will be appreciated that when a description of a combination of Compound I and other specified CFTR modulating agents is provided herein, reference to "Compound **III** or **III-d"** in connection with the combination means that either Compound **III** or Compound **III-d,** but not both, is included in the combination.

As used herein, the term "active pharmaceutical ingredient" or "therapeutic agent" ("API") refers to a biologically active compound.

As used herein, the term "pharmaceutically acceptable salt" refers to a salt form of a compound of this disclosure wherein the salt is nontoxic. Pharmaceutically acceptable salts of the compounds of this disclosure include those derived from suitable inorganic and organic acids and bases. Pharmaceutically acceptable salts are well known in the art. For example, S. M. Berge, et al. describes pharmaceutically acceptable salts in detail in J. Pharmaceutical Sciences, 1977, 66, 1-19.

As used herein, the term "amorphous" refers to a solid material having no long-range order in the position of its molecules. Amorphous solids are generally supercooled liquids in which the molecules are arranged in a random manner so that there is no well-defined arrangement, e.g., molecular packing, and no long-range order. Amorphous solids are generally isotropic, i.e. exhibit similar properties in all directions and do not have definite melting points. For example, an amorphous material is a solid material having no sharp characteristic crystalline peak(s) in its X-ray power diffraction (XRPD) pattern (i.e., is not crystalline as determined by XRPD). Instead, one or several broad peaks (e.g., halos) appear in its XRPD pattern. Broad peaks are characteristic of an amorphous solid. See, US 2004/0006237 for a comparison of XRPDs of an amorphous material and crystalline material. In some embodiments, a solid material may comprise an amorphous compound, and the material may, for example, be characterized by a lack of sharp characteristic crystalline peak(s) in its XRPD spectrum (i.e. the material is not crystalline, but is amorphous, as determined by XRPD). Instead, one or several broad peaks (e.g. halos) may appear in the XRPD pattern of the material. See US 2004/0006237 for a comparison of XRPDs of an amorphous material and crystalline material. A solid material, comprising an amorphous compound, may be characterized by, for example, a wider temperature range for the melting of the solid material, as compared to the range for the melting of a pure crystalline solid. Other techniques, such as, for example, Raman spectroscopy, infrared spectroscopy, and solid state NMR may be used to characterize crystalline or amorphous forms.

In some embodiments, a solid material may comprise a mixture of crystalline solids and amorphous solids. A solid material prepared to comprise an amorphous compound may also, for example, contain up to 30% of a crystalline solid. In some embodiments, a solid material prepared to comprise an amorphous compound may also, for example, contain up to 25%, 20%, 15%, 10%, 5%, or 2% of a crystalline solid. In embodiments wherein the solid material contains a mixture of crystalline solids and amorphous solids, the characterizing data, such as XRPD, may contain indicators of both crystalline and amorphous solids.

As used herein, the term "substantially amorphous" refers to a solid material having little or no long-range order in the position of its molecules. For example, substantially amorphous materials have less than 15% crystallinity (e.g., less than 10% crystallinity, less than 5% crystallinity, or less than 2% crystallinity). It is also noted that the term 'substantially amorphous' includes the descriptor, 'amorphous', which refers to materials having no (0%) crystallinity.

As used herein, the term "dispersion" refers to a disperse system in which one substance, the dispersed phase, is distributed, in discrete units, throughout a second substance (the continuous phase or vehicle). The size of the dispersed phase can vary considerably (e.g. colloidal particles of nanometer dimension, to multiple microns in size). In general, the dispersed phases can be solids, liquids, or gases. In the case of a solid dispersion, the dispersed and continuous phases are both solids. In pharmaceutical applications, a solid dispersion can include a crystalline drug (dispersed phase) in an amorphous polymer (continuous phase); or alternatively, an amorphous drug (dispersed phase) in an amorphous polymer (continuous phase). In some embodiments, a solid dispersion includes the polymer constituting the dispersed phase, and the drug constitute the continuous phase. Or, a solid dispersion includes the drug constituting the dispersed phase, and the polymer constituting the continuous phase.

The terms "patient" and "subject" are used interchangeably and refer to an animal including humans.

As used herein, the terms "treatment," "treating," and the like generally mean the improvement of CF or its symptoms or lessening the severity of CF or its symptoms in a subject. "Treatment," as used herein, includes, but is not limited to, the following: increased growth of the subject, increased weight gain, reduction of mucus in the lungs, improved pancreatic and/or liver function, reduction of chest infections, and/or reductions in coughing or shortness of breath. Improvements in or lessening the severity of any of these symptoms can be readily assessed according to standard methods and techniques known in the art.

As used herein, the term "in combination with," when referring to two or more compounds, agents, or additional active pharmaceutical ingredients, means the administration of two or more compounds, agents, or active pharmaceutical ingredients to the patient prior to, concurrently with, or subsequent to each other.

The terms "about" and "approximately", when used in connection with doses, amounts, or weight percent of ingredients of a composition or a dosage form, include the value of a specified dose, amount, or weight percent or a range of the dose, amount, or weight percent that is recognized by one of ordinary skill in the art to provide a pharmacological effect equivalent to that obtained from the specified dose, amount, or weight percent. The terms "about" and "approximately" may refer to an acceptable error for a particular value as determined by one of skill in the art, which depends in part on how the values is measured or determined. In some embodiments, the terms "about" and "approximately" mean within 20%, 15%, 10%, 5%, 4%, 3%, 2%, 1%, or 0.5% of a given value or range.

One of ordinary skill in the art would recognize that, when an amount of "a compound or a pharmaceutically acceptable salt thereof" is disclosed, the amount of the pharmaceutically acceptable salt form of the compound is the amount equivalent to the concentration of the free base of the compound. It is noted that the disclosed amounts of the compounds or their pharmaceutically acceptable salts thereof herein are based upon their free base form. For example, "100 mg of at least one compound chosen from Compound **I** and pharmaceutically acceptable salts thereof" includes 100 mg of Compound **I** and a concentration of a pharmaceutically acceptable salt of Compound **I** equivalent to 100 mg of Compound **I.**

Suitable pharmaceutically acceptable salts are, for example, those disclosed in S. M. Berge, et al. J. Pharmaceutical Sciences, 1977, 66, 1-19. For example, Table 1 of that article provides the following pharmaceutically acceptable salts:

**Table 1:**

| | | |
|---|---|---|
| Acetate | Iodide | Benzathine |
| Benzenesulfonate | Isethionate | Chloroprocaine |
| Benzoate | Lactate | Choline |
| Bicarbonate | Lactobionate | Diethanolamine |
| Bitartrate | Malate | Ethylenediamine |
| Bromide | Maleate | Meglumine |
| Calcium edetate | Mandelate | Procaine |
| Camsylate | Mesylate | Aluminum |
| Carbonate | Methylbromide | Calcium |
| Chloride | Methylnitrate | Lithium |
| Citrate | Methylsulfate | Magnesium |
| Dihydrochloride | Mucate | Potassium |
| Edetate | Napsylate | Sodium |
| Edisylate | Nitrate | Zinc |
| Estolate | Pamoate (Embonate) | |
| Esylate | Pantothenate | |
| Fumarate | Phosphate/diphosphate | |
| Gluceptate | Polygalacturonate | |
| Gluconate | Salicylate | |
| Glutamate | Stearate | |
| Glycollylarsanilate | Subacetate | |
| Hexylresorcinate | Succinate | |
| Hydrabamine | Sulfate | |
| Hydrobromide | Tannate | |
| Hydrochloride | Tartrate | |
| Hydroxynaphthoate | Teociate | |
| | Triethiodide | |

Non-limiting examples of pharmaceutically acceptable acid addition salts include: salts formed with inorganic acids, such as hydrochloric acid, hydrobromic acid, phosphoric acid, sulfuric acid, or perchloric acid; salts formed with organic acids, such as acetic acid, oxalic acid, maleic acid, tartaric acid, citric acid, succinic acid or malonic acid; and salts formed by using other methods used in the art, such as ion exchange. Non-limiting examples of pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, p-toluenesulfonate, undecanoate, and valerate salts. Pharmaceutically acceptable salts derived from appropriate bases include alkali metal, alkaline earth metal, ammonium, and N⁺(C₁₋₄alkyl)₄ salts. This disclosure also envisions the quaternization of any basic nitrogen-containing groups of the compounds disclosed herein. Suitable non-limiting examples of alkali and alkaline earth metal salts include sodium, lithium, potassium, calcium, and magnesium. Further non-limiting examples of pharmaceutically acceptable salts include ammonium, quaternary ammonium, and amine cations formed using counterions such as halide, hydroxide, carboxylate, sulfate, phosphate, nitrate, lower alkyl sulfonate and aryl sulfonate. Other suitable, non-limiting examples of pharmaceutically acceptable salts include besylate and glucosamine salts.

### Combination Therapies

One aspect disclosed herein provides methods of treating cystic fibrosis and other CFTR mediated diseases with Compound I in combination with other pharmaceutically active agents, including CFTR modulating agents. In some embodiments, Compound **I** (and/or at least one pharmaceutically acceptable salt thereof) can be administered in combination with at least one additional active pharmaceutical ingredient, such as, e.g., a CFTR modulating agent. In some embodiments, the at least one additional active pharmaceutical ingredient is chosen from (a) Compound **II** and pharmaceutically acceptable salts thereof; and (b) Compound **III** or Compound **III-d** and pharmaceutically acceptable salts of Compound **III** or Compound **III-d.** Thus, in some embodiments, the methods of treatment provided herein comprise administering to a patient in need thereof at least one compound chosen from Compound **I** and pharmaceutically acceptable salts thereof; and at least one compound chosen from Compound **II,** (Compound **III** or **III-d),** and pharmaceutically acceptable salts thereof. In some embodiments, the methods of treatment provided herein comprise administering to a patient in need thereof at least one compound chosen from Compound **I** and pharmaceutically acceptable salts thereof; and at least one compound chosen from (Compound **III** or **III-d),** Compound **IV,** and/or pharmaceutically acceptable salts thereof.

In some embodiments, at least one compound chosen from Compound **I** and pharmaceutically acceptable salts thereof is administered in combination with at least one compound chosen from Compound **II** and pharmaceutically acceptable salts thereof. In some embodiments, at least one compound chosen from Compound **I** and pharmaceutically acceptable salts thereof is administered in combination with at least one compound chosen from Compound **III** and pharmaceutically acceptable salts thereof. In some embodiments, at least one compound chosen from Compound **I** and pharmaceutically acceptable salts thereof is administered in combination with at least one compound chosen from Compound **III-d** and pharmaceutically acceptable salts thereof. In some embodiments, at least one compound chosen from Compound **I** and pharmaceutically acceptable salts thereof is administered in combination with Compound **II** or a pharmaceutically acceptable salt thereof and at least one compound chosen from Compound **III** and pharmaceutically acceptable salts thereof. In some embodiments, at least one compound chosen from Compound **I** and pharmaceutically acceptable salts thereof is administered in combination with at least one compound chosen from Compound **II** and pharmaceutically acceptable salts thereof and at least one compound chosen from Compound **III-d** and pharmaceutically acceptable salts thereof.

Each of Compounds **I, II,** and **III** or **III-d,** and their pharmaceutically acceptable salts thereof independently can be administered once daily, twice daily, or three times daily. In some embodiments, at least one compound chosen from Compound **I** and pharmaceutically acceptable salts thereof is administered once daily. In some embodiments, at least one compound chosen from Compound **I** and pharmaceutically acceptable salts thereof is administered twice daily. In some embodiments, at least one compound chosen from Compound **I** and pharmaceutically acceptable salts thereof and at least one compound chosen from Compound **II** and pharmaceutically acceptable salts thereof are administered once daily. In some embodiments, at least one compound chosen from Compound **I** and pharmaceutically acceptable salts thereof and at least one compound chosen from Compound **II** and pharmaceutically acceptable salts thereof are administered twice daily. In some embodiments, at least one compound chosen from Compound **I** and pharmaceutically acceptable salts thereof and at least one compound chosen from Compound **III** or **III-d** and pharmaceutically acceptable salts thereof are administered once daily. In some embodiments, at least one compound chosen from Compound **I** and pharmaceutically acceptable salts thereof and at least one compound chosen from Compound **III** or **III-d** and pharmaceutically acceptable salts thereof are administered twice daily.

In some embodiments, at least one compound chosen from Compound **I** and pharmaceutically acceptable salts thereof, at least one compound chosen from Compound **II** and pharmaceutically acceptable salts thereof, and at least one compound chosen from Compound **III** or **III-d** and pharmaceutically acceptable salts thereof are administered once daily. In some embodiments, at least one compound chosen from Compound **I** and pharmaceutically acceptable salts thereof, at least one compound chosen from Compound **III** or **III-d** and pharmaceutically acceptable salts thereof, and at least one compound chosen from Compound **IV** and pharmaceutically acceptable salts thereof, are administered once daily. In some embodiments, at least one compound chosen from Compound **I** and pharmaceutically acceptable salts thereof, at least one compound chosen from Compound **II** and pharmaceutically acceptable salts thereof, and at least one compound chosen from Compound **III** or **III-d** and pharmaceutically acceptable salts thereof are administered twice daily. In some embodiments, at least one compound chosen from Compound **I** and pharmaceutically acceptable salts thereof, at least one compound chosen from Compound **III** or **III-d** and pharmaceutically acceptable salts thereof, and at least one compound chosen from Compound **IV** and pharmaceutically acceptable salts thereof, are administered twice daily.

In some embodiments, at least one compound chosen from Compound **I** and pharmaceutically acceptable salts thereof and at least one compound chosen from Compound **II** and pharmaceutically acceptable salts thereof, are administered once daily and at least one compound chosen from Compound **III-d** and pharmaceutically acceptable salts thereof, are administered twice daily. In some embodiments, at least one compound chosen from Compound **I** and pharmaceutically acceptable salts thereof and at least one compound chosen from Compound **IV** and pharmaceutically acceptable salts thereof, are administered once daily and at least one compound chosen from Compound **III-d** and pharmaceutically acceptable salts thereof, are administered twice daily.

In some embodiments, at least one compound chosen from Compound **I** and pharmaceutically acceptable salts thereof is administered in an amount of 5 mg to 20 mg. In some embodiments, at least one compound chosen from Compound **I** and pharmaceutically acceptable salts thereof is administered in an amount of 5 mg, 10 mg, 15 mg, or 20 mg daily. In some embodiments, at least one compound chosen from Compound **I** and pharmaceutically acceptable salts thereof is administered in an amount of 5 mg, 10 mg, or 20 mg once daily. In some embodiments, 5 mg, or 10 mg of Compound **I** or an equivalent amount of its pharmaceutically acceptable salts are administered twice daily.

Compounds **I, II, (III** or **III-d**), and their pharmaceutically acceptable salts thereof can be administered in a single pharmaceutical composition or separate pharmaceutical compositions. Such pharmaceutical compositions can be administered once daily or multiple times daily, such as twice daily. As used herein, the phrase that a given amount of API (e.g., Compound **I, II, (III, III-d**) or a pharmaceutically acceptable salt thereof) is administered once or twice daily or per day means that said given amount is administered per dosing once or twice daily. For example, the phrase that 50 mg of Compound **II** or a pharmaceutically acceptable salt thereof is administered twice daily or per day means that 50 mg of Compound **II** or an equivalent amount of a pharmaceutically acceptable salt thereof is administerd per dosing twice per day (e.g., 50 mg of Compound **II** or an equivalent amount of a pharmaceutically acceptable salt thereof is administerd in the morning and 50 mg of Compound **II** or an equivalent amount of a pharmaceutically acceptable salt thereof is administered in the evening).

In some embodiments, at least one compound chosen from Compound I and pharmaceutically acceptable salts thereof is administered in a first pharmaceutical composition; at least one compound chosen from Compound **II** and pharmaceutically acceptable salts thereof is administered in a second pharmaceutical composition; and at least one compound chosen from Compound **III** and pharmaceutically acceptable salts thereof is administered in a third pharmaceutical composition.

In some embodiments, at least one compound chosen from Compound I and pharmaceutically acceptable salts thereof is administered in a first pharmaceutical composition; at least one compound chosen from Compound **II** and pharmaceutically acceptable salts thereof is administered in a second pharmaceutical composition; at least one compound chosen from Compound **III-d** and pharmaceutically acceptable salts thereof is administered in a third pharmaceutical composition.

In some embodiments, at least one compound chosen from Compound **I** and pharmaceutically acceptable salts thereof is administered in a first pharmaceutical composition; at least one compound chosen from Compound **III** or **III-d** and pharmaceutically acceptable salts thereof is administered in a second pharmaceutical composition; at least one compound chosen from Compound **IV** and pharmaceutically acceptable salts thereof is administered in a third pharmaceutical composition.

In some embodiments, at least one compound chosen from Compound **I** and pharmaceutically acceptable salts thereof is administered in a first pharmaceutical composition; and at least one compound chosen from Compound **II** and pharmaceutically acceptable salts thereof and at least one compound chosen from Compound **III or III-d,** and pharmaceutically acceptable salts thereof are administered in a second pharmaceutical composition. In some embodiments, the second pharmaceutical composition comprises a half of a daily dose of said at least one compound chosen from Compound **III, III-d,** and pharmaceutically acceptable salts thereof, and the other half of said at least one compound chosen from Compound **III, III-d,** and pharmaceutically acceptable salts thereof is administered in a third pharmaceutical composition.

In some embodiments, at least one compound chosen from Compound **I** and pharmaceutically acceptable salts thereof; at least one compound chosen from Compound **II** and pharmaceutically acceptable salts thereof and at least one compound chosen from Compound **III, III-d,** and pharmaceutically acceptable salts thereof are administered in a first pharmaceutical composition. In some embodiments, the first pharmaceutical composition is administered to the patient twice daily. In some embodiments the first pharmaceutical composition is administered once daily. In some embodiments the first pharmaceutical composition is administered once daily and a second composition comprising only Compound **III** is administered once daily.

Any suitable pharmaceutical compositions known in the art can be used for Compound **I,** Compound **II,** Compound **III,** Compound **III-d,** and pharmaceutically acceptable salts thereof. Some exemplary pharmaceutical compositions for Compound **I** and its pharmaceutically acceptable salts are described in the Examples. Some exemplary pharmaceutical compositions for Compound **II** and its pharmaceutically acceptable salts can be found in WO 2011/119984 and WO 2014/014841. Some exemplary pharmaceutical compositions for Compound **III** and its pharmaceutically acceptable salts can be found in WO 2007/134279, WO 2010/019239, WO 2011/019413, WO 2012/027731, and WO 2013/130669, and some exemplary pharmaceutical compositions for Compound **III-d** and its pharmaceutically acceptable salts can be found in US 8,865,902, US 9,181,192, US 9,512,079, WO 2017/053455, and WO 2018/080591. Some exemplary pharmaceutical compositions for Compound **IV** and its pharmaceutically acceptable salts can be found in WO 2010/037066, WO 2011/127241, and WO 2014/0712 22.

In some embodiments, a pharmaceutical composition comprising at least one compound chosen from Compound **I** and pharmaceutically acceptable salts thereof is administered with a pharmaceutical composition comprising Compound **II** and Compound **III** or **III-d.** Pharmaceutical compositions comprising Compound **II** and Compound **III** are disclosed in PCT Publication No. WO 2015/160787. An exemplary embodiment is shown in Table 2.

**Table 2. Exemplary Tablet Comprising 100 mg of Compound II and 150 mg of Compound III.**

| | **Ingredient** | **Amount per tablet (mg)** |
|---|---|---|
| Intra-granular | Compound **II** SDD (spray dried dispersion) (80 wt % Compound II; 20 wt % HPMC) | 125 |
| | Compound **III** SDD (80 wt % Compound III; 19.5 wt% HPMCAS-HG; 0.5 wt% sodium lauryl sulfate) | 187.5 |
| | Microcrystalline cellulose | 131.4 |
| | Croscarmellose Sodium | 29.6 |
| | **Total** | **473.5** |
| Extra-granular | Microcrystalline cellulose | 112.5 |
| | Magnesium Stearate | 5.9 |
| | **Total** | **118.4** |
| **Total uncoated Tablet** | | **591.9** |
| Film coat | Opadry | 17.7 |
| **Total coated Tablet** | | **609.6** |

In some embodiments, a pharmaceutical composition comprising Compound **I** is administered with a pharmaceutical composition comprising Compound **III** or **III-d.** Pharmaceutical compositions comprising Compound **III** are disclosed in PCT Publication No. WO 2010/01 9239. An exemplary embodiment is shown in Table 3 below.

**Table 3: Ingredients for Exemplary Tablet of Compound III.**

| **Tablet Formulation** | **Percent Dose %Wt./Wt.** | **Dose (mg)** | **Batch (g)** |
|---|---|---|---|
| Compound **III** SDD (80 wt % Compound **III;** 19.5 wt% HPMCAS-HG; 0.5 wt% sodium lauryl sulfate) | 34.09% | 187.5 | 23.86 |
| Microcrystalline cellulose | 30.51% | 167.8 | 21.36 |
| Lactose | 30.40% | 167.2 | 21.28 |
| Sodium croscarmellose | 3.000% | 16.50 | 2.100 |
| SLS | 0.500% | 2.750 | 0.3500 |
| Colloidal silicon dioxide | 0.500% | 2.750 | 0.3500 |
| Magnesium stearate | 1.000% | 5.500 | 0.7000 |
| **Total** | **100%** | **550** | **70** |

Additional pharmaceutical compositions comprising Compound **III** are disclosed in PCT Publication No. WO 2013/130669. Exemplary mini-tablets (~(2 mm diameter, ~2 mm thickness, each mini-tablet weighing about 6.9 mg) was formulated to have approximately 50 mg of Compound **III** per 26 mini-tablets and approximately 75 mg of Compound **III** per 39 mini-tablets using the amounts of ingredients recited in Table 4.

**Table 4: Ingredients for mini-tablets for 50 mg and 75 mg potency**

| **Tablet Formulation** | **Percent Dose %Wt./Wt.** | **Dose (mg) 50 mg potency** | **Dose (mg) 75 mg potency** | **Batch (g)** |
|---|---|---|---|---|
| Compound **III** SDD (80 wt % Compound **III;** 19.5 wt% HPMCAS-HG; 0.5 wt% sodium lauryl sulfate) | 35 | 62.5 | 93.8 | 1753.4 |
| Mannitol | 13.5 | 24.1 | 36.2 | 675.2 |
| Lactose | 41 | 73.2 | 109.8 | 2050.2 |
| Sucralose | 2.0 | 3.6 | 5.4 | 100.06 |
| Croscarmellose sodium | 6.0 | 10.7 | 16.1 | 300.1 |
| Colloidal silicon dioxide | 1.0 | 1.8 | 2.7 | 50.0 |
| Magnesium stearate | 1.5 | 2.7 | 4.0 | 74.19 |
| **Total** | **100** | **178.6** | **268** | **5003.15** |

In some embodiments, a pharmaceutical composition comprising Compound **I** is administered with a pharmaceutical composition comprising Compound **III** and Compound **IV.** Pharmaceutical compositions comprising Compound **III** and Compound **IV** are disclosed in PCT Publication No. WO 2014/071222.. An exemplary embodiment is shown in Table 5 below.

**Table 5: Ingredients for Exemplary Tablet of Compound III and Compound IV**

| **Tablet Formulation** | **Percent Dose %Wt./Wt.** | **Dose (mg) 200 mg potency** |
|---|---|---|
| Compound **IV** Form I | 35 | 200 |
| Compound **III** SDD (80 wt % Compound **III;** 19.5 wt% HPMCAS-HG; 0.5 wt% sodium lauryl sulfate) | 28 | 156 |
| Microcrystalline cellulose | 26 | 150 |
| Croscarmellose sodium | 6 | 34 |
| Sodium lauryl sulfate | 1 | 4 |
| Polyvinylpyrrolidone | 3 | 15 |
| Magnesium stearate | 1 | 6 |

In some embodiments, the pharmaceutical compositions employed in the combination therapies of the disclosure are tablets. In some embodiments, the tablets are suitable for oral administration. These compositions and combinations are useful for treating cystic fibrosis.

### Methods of Treatment

A CFTR mutation may affect the CFTR quantity, i.e., the number of CFTR channels at the cell surface, or it may impact CFTR function, i.e., the functional ability of each channel to open and transport ions. Mutations affecting CFTR quantity include mutations that cause defective synthesis (Class I defect), mutations that cause defective processing and trafficking (Class II defect), mutations that cause reduced synthesis of CFTR (Class V defect), and mutations that reduce the surface stability of CFTR (Class VI defect). Mutations that affect CFTR function include mutations that cause defective gating (Class III defect) and mutations that cause defective conductance (Class IV defect). Some CFTR mutations exhibit characteristics of multiple classes.

In some embodiments, disclosed herein methods of treating, lessening the severity of, or symptomatically treating cystic fibrosis in a patient comprising administering an effective amount of a compound, pharmaceutically acceptable salt thereof, or a deuterated analog of any of the foregoing; or a pharmaceutical composition, of this disclosure to a patient, such as a human, wherein said patient has cystic fibrosis. In some embodiments, the patient has an F508del/minimal function (MF) genotype, F508del/F508del genotype (homozygous for the F508del mutation), F508del/gating genotype, or F508del/residual function (RF) genotype. In some embodiments the patient is heterozygous and has one F508del mutation.

As used herein, "minimal function (MF) mutations" refer to CFTR gene mutations associated with minimal CFTR function (little-to-no functioning CFTR protein) and include, for example, mutations associated with severe defects in ability of the CFTR channel to open and close, known as defective channel gating or "gating mutations"; mutations associated with severe defects in the cellular processing of CFTR and its delivery to the cell surface; mutations associated with no (or minimal) CFTR synthesis; and mutations associated with severe defects in channel conductance.

In some embodiments, the patient is heterozygous and has an F508del mutation on one allele and a mutation on the other allele selected from Table 6:

**Table 6: CFTR Mutations**

| **Mutation** | | | | |
|---|---|---|---|---|
| Q2X | L218X | Q525X | R792X | E1104X |
| S4X | Q220X | G542X | E822X | W1145X |
| W19X | Y275X | G550X | W882X | R1158X |
| G27X | C276X | Q552X | W846X | R1162X |
| Q39X | Q290X | R553X | Y849X | S 1196X |
| W57X | G330X | E585X | R851X | W1204X |
| E60X | W401X | G673X | Q890X | L1254X |
| R75X | Q414X | Q685X | S912X | S1255X |
| L88X | S434X | R709X | Y913X | W1282X |
| E92X | S466X | K710X | Q1042X | Q1313X |
| Q98X | S489X | Q715X | W1089X | Q1330X |
| Y122X | Q493X | L732X | Y1092X | E1371X |
| E193X | W496X | R764X | W1098X | Q1382X |
| W216X | C524X | R785X | R1102X | Q1411X |
| 185+1G→T | 711+5G→A | 1717-8G→A | 2622+1G→7A | 3121-1G→A |
| 296+1G-A | 712-1G→T | 1717-1G→A | 2790-1G→C | 3500-2A→G |
| 296+1G→T | 1248+1G→A | l811+1G→7C | 3040G→7C | 3600+2insT |
| 405+1G→A | 1249-1G→A | 1811+1.6kbA→G | (G970R) | 3850-1G→7A |
| 405+3A→7C | 1341+1G→A | 1811+1643G→T | 3120G→A | 4005+IG→A |
| 406-1G→A | 1525-2A→G | 1812-1G→A | 3120+1G→A | 4374+1G→T |
| 621+1G→T | 1525-1G→A | 1898+IG→A | 3121-2A-G | |
| 711+1G→T | | 1898+1G→C | | |
| 182delT | 1078delT | 1677delTA | 2711delT | 3737delA |
| 306insA | 1119delA | 1782delA | 2732insA | 3791delC |
| 306delTAGA | 1138insG | 1824delA | 2869insG | 3821delT |
| 365-366insT | 1154insTC | 1833delT | 2896insAG | 3876delA |
| 394delTT | 1161delC | 2043delG | 2942insT | 3878delG |
| 442delA | 1213delT | 2143delT | 2957delT | 3905insT |
| 444delA | 1259insA | 2183AA→G | 3007delG | 4016insT |
| 457TAT→G | 1288insTA | 2184delA | 3028delA | 4021dupT |
| 541delC | 1343delG | 2184insA | 3171delC | 4022insT |
| 574delA | 1471delA | 2307insA | 3171insC | 4040delA |
| 663delT | 1497delGG | 2347delG | 3271delGG | 4279insA |
| 849delG | 1548delG | 2585delT | 3349insT | 4326delTC |
| 935delA | 1609del CA | 2594delGT | 3659delC | |
| CFTRdele1 | | CFTRdele16-17b | 1461ins4 | |
| CFTRdele2 | | CFTRdele17a,17b | 1924del7 | |
| CFTRdele2,3 | | CFTRdele17a-18 | 2055del9→A | |
| CFTRdele2-4 | | CFTRdelel9 | 2105-2117del13insAGAAA | |
| CFTRdele3-10,14b-16 | | CFTRdele19-21 | 2372del8 | |
| CFTRdele4-7 | | CFTRdele21 | 2721del11 | |
| CFTRdele4-11 | | CFTRdele22-24 | 2991del32 | |
| CFTR50kbdel | | CFTRdele22,23 | 3667ins4 | |
| CFTRdup6b-10 | | 124del23bp | 4010del4 | |
| CFTRdele11 | | 602del14 | 4209TGTT-AA | |
| CFTRdele13,14a | | 852del22 | | |
| CFTRdele14b-17b | | 991del5 | | |
| A46D | V520F | Y569D | N1303K | |
| G85E | A559T | L1065P | | |
| R347P | R560T | R1066C | | |
| L467P | R560S | L1077P | | |
| I507del | A561E | M1101K | | |

In some embodiments, the disclosure also is directed to methods of treatment using isotope-labelled compounds of the afore-mentioned compounds, which, in some embodiments, are referred to as Compound I', Compound **II',** Compound **III',** Compound **III-d.** In some embodiments, Compound I', Compound **II',** Compound **III',** Compound **III-d,** or pharmaceutically acceptable salts thereof, wherein the formula and variables of such compounds and salts are each and independently as described above or any other embodiments described above, provided that one or more atoms therein have been replaced by an atom or atoms having an atomic mass or mass number which differs from the atomic mass or mass number of the atom which usually occurs naturally (isotope labelled). Examples of isotopes which are commercially available and suitable for the disclosure include isotopes of hydrogen, carbon, nitrogen, oxygen, phosphorus, fluorine and chlorine, for example ²H, ³H, ¹³C, ¹⁴C, ¹⁵N, ¹⁸O, ¹⁷O, ³¹P, ³²P, ³⁵S, ¹⁸F and ³⁶Cl, respectively.

The isotope-labelled compounds and salts can be used in a number of beneficial ways. They can be suitable for medicaments and/or various types of assays, such as substrate tissue distribution assays. For example, tritium (³H)- and/or carbon-14 (¹⁴C)-labelled compounds are particularly useful for various types of assays, such as substrate tissue distribution assays, due to relatively simple preparation and excellent detectability. For example, deuterium (²H)-labelled ones are therapeutically useful with potential therapeutic advantages over the non ²H-labelled compounds. In general, deuterium (²H)-labelled compounds and salts can have higher metabolic stability as compared to those that are not isotope-labelled owing to the kinetic isotope effect described below. Higher metabolic stability translates directly into an increased in vivo half-life or lower dosages, which could be desired. The isotope-labelled compounds and salts can usually be prepared by carrying out the procedures disclosed in the synthesis schemes and the related description, in the example part and in the preparation part in the present text, replacing a non-isotope-labelled reactant by a readily available isotope-labelled reactant.

In some embodiments, the isotope-labelled compounds and salts are deuterium (²H)-labelled ones. In some specific embodiments, the isotope-labelled compounds and salts are deuterium (²H)-labelled, wherein one or more hydrogen atoms therein have been replaced by deuterium. In chemical structures, deuterium is represented as "D."

The deuterium (²H)-labelled compounds and salts can manipulate the oxidative metabolism of the compound by way of the primary kinetic isotope effect. The primary kinetic isotope effect is a change of the rate for a chemical reaction that results from exchange of isotopic nuclei, which in turn is caused by the change in ground state energies necessary for covalent bond formation after this isotopic exchange. Exchange of a heavier isotope usually results in a lowering of the ground state energy for a chemical bond and thus causes a reduction in the rate-limiting bond breakage. If the bond breakage occurs in or in the vicinity of a saddle-point region along the coordinate of a multi-product reaction, the product distribution ratios can be altered substantially. For explanation: if deuterium is bonded to a carbon atom at a non-exchangeable position, rate differences of k_{M/}k_{D} = 2-7 are typical. For a further discussion, see S. L. Harbeson and R. D. Tung, Deuterium In Drug Discovery and Development, Ann. Rep. Med. Chem. 2011, 46, 403-417.

The concentration of the isotope(s) (e.g., deuterium) incorporated into the isotope-labelled compounds and salt of the disclosure may be defined by the isotopic enrichment factor. The term "isotopic enrichment factor" as used herein means the ratio between the isotopic abundance and the natural abundance of a specified isotope. In some embodiments, if a substituent in a compound of the disclosure is denoted deuterium, such compound has an isotopic enrichment factor for each designated deuterium atom of at least 3500 (52.5% deuterium incorporation at each designated deuterium atom), at least 4000 (60% deuterium incorporation), at least 4500 (67.5% deuterium incorporation), at least 5000 (75% deuterium incorporation), at least 5500 (82.5% deuterium incorporation), at least 6000 (90% deuterium incorporation), at least 6333.3 (95% deuterium incorporation), at least 6466.7 (97% deuterium incorporation), at least 6600 (99% deuterium incorporation), or at least 6633.3 (99.5% deuterium incorporation).

When discovering and developing therapeutic agents, the person skilled in the art attempts to optimize pharmacokinetic parameters while retaining desirable in vitro properties. It may be reasonable to assume that many compounds with poor pharmacokinetic profiles are susceptible to oxidative metabolism.

### Pharmaceutical Compositions

Another aspect of the disclosure provides pharmaceutical compositions comprising Compound I or a pharmaceutically acceptable salt thereof and at least one additional active pharmaceutical ingredient. In some embodiments, the at least one additional active pharmaceutical ingredient is a CFTR modulator. In some embodiments, the at least one additional active pharmaceutical ingredient is a CFTR corrector. In some embodiments, the at least one additional active pharmaceutical ingredient is a CFTR potentiator. In some embodiments, the pharmaceutical composition comprises Compound I and at least two additional active pharmaceutical ingredients, one of which is a CFTR corrector and one of which is a CFTR potentiator.

In some embodiments, at least one additional active pharmaceutical ingredient is selected from mucolytic agents, bronchodilators, antibiotics, anti-infective agents, and anti-inflammatory agents.

In some embodiments, the disclosure provides a pharmaceutical composition comprising at least one compound chosen from Compound I and pharmaceutically acceptable salts thereof, and at least one pharmaceutically acceptable carrier.

In some embodiments, the disclosure provides a pharmaceutical composition comprising 5 mg to 20 mg of at least one compound chosen from Compound I and pharmaceutically acceptable salts thereof, at least one compound chosen from Compound II and pharmaceutically acceptable salts thereof, and at least one pharmaceutically acceptable carrier.

In some embodiments, the disclosure provides a pharmaceutical composition comprising 5 mg to 20 mg of at least one compound chosen from Compound I and pharmaceutically acceptable salts thereof, at least one compound chosen from Compound **III, III-d,** and pharmaceutically acceptable salts thereof, and at least one pharmaceutically acceptable carrier.

In some embodiments, the disclosure provides a pharmaceutical composition comprising 5 mg to 20 mg of at least one compound chosen from Compound I and pharmaceutically acceptable salts thereof, at least one compound chosen from Compound II and pharmaceutically acceptable salts thereof, at least one compound chosen from Compound III and pharmaceutically acceptable salts thereof, and at least one pharmaceutically acceptable carrier.

In some embodiments, the disclosure provides a pharmaceutical composition comprising 5 mg to 20 mg of at least one compound chosen from Compound I and pharmaceutically acceptable salts thereof, at least one compound chosen from Compound II and pharmaceutically acceptable salts thereof, at least one compound chosen from Compound **III-d** and pharmaceutically acceptable salts thereof, and at least one pharmaceutically acceptable carrier.

In some embodiments, the disclosure provides a pharmaceutical composition comprising 5 mg to 20 mg of at least one compound chosen from Compound I and pharmaceutically acceptable salts thereof, at least one compound chosen from Compound **III** or **III-d** and pharmaceutically acceptable salts thereof, at least one compound chosen from Compound **IV** and pharmaceutically acceptable salts thereof, and at least one pharmaceutically acceptable carrier.

In some embodiments, the disclosure provides a pharmaceutical composition comprising 5 mg to 20 mg of at least one compound chosen from Compound **I** and pharmaceutically acceptable salts thereof, and optionally comprise one or more additional CFTR modulating agents. In some embodiments, the composition comprises about 5 mg, about 10 mg, or about 20 mg of at least one compound chosen from Compound **I** and pharmaceutically acceptable salts thereof, and optionally comprise one or more additional CFTR modulating agents. In some embodiments, the composition comprises 5 mg to 20 mg of at least one compound chosen from Compound **I** and pharmaceutically acceptable salts thereof, 50 mg to 100 mg of Compound **II,** and 150 mg to 300 mg of Compound **III** or 50 mg to 150 mg of Compound **III-d.** In some embodiments the composition comprises about 5 mg to 20 mg of at least one compound chosen from Compound **I** and pharmaceutically acceptable salts thereof, 100 mg of Compound **II,** and 150 mg of Compound **III** or 150 mg of Compound **III-d.**

In some embodiments, the disclosure provides a pharmaceutical composition comprising 5 mg of at least one compound chosen from Compound **I** and pharmaceutically acceptable salts thereof, and optionally comprise one or more additional CFTR modulating agents. In some embodiments, the composition comprises about 10 mg of at least one compound chosen from Compound **I** and pharmaceutically acceptable salts thereof, and optionally comprise one or more additional CFTR modulating agents. In some embodiments, the disclosure provides a pharmaceutical composition comprising 20 mg of at least one compound chosen from Compound **I** and pharmaceutically acceptable salts thereof, and optionally comprise one or more additional CFTR modulating agents. In some embodiments, the composition comprises 5 mg, 10 mg, or 20 mg of at least one compound chosen from Compound **I** and pharmaceutically acceptable salts thereof, 50 mg or 100 mg of Compound **II,** and 150 mg or 300 mg of Compound **III** or 50 mg, 75 mg, 100 mg, 125 mg, or 150 mg of Compound **III-d.**

A pharmaceutical composition may further comprise at least one pharmaceutically acceptable carrier. In some embodiments, the at least one pharmaceutically acceptable carrier is chosen from pharmaceutically acceptable vehicles and pharmaceutically acceptable adjuvants. In some embodiments, the at least one pharmaceutically acceptable is chosen from pharmaceutically acceptable fillers, disintegrants, surfactants, binders, lubricants.

The pharmaceutical compositions described herein are useful for treating cystic fibrosis and other CFTR mediated diseases.

As described above, pharmaceutical compositions disclosed herein may optionally further comprise at least one pharmaceutically acceptable carrier. The at least one pharmaceutically acceptable carrier may be chosen from adjuvants and vehicles. The at least one pharmaceutically acceptable carrier, as used herein, includes any and all solvents, diluents, other liquid vehicles, dispersion aids, suspension aids, surface active agents, isotonic agents, thickening agents, emulsifying agents, preservatives, solid binders, and lubricants, as suited to the particular dosage form desired. Remington: The Science and Practice of Pharmacy, 21st edition, 2005, ed. D.B. Troy, Lippincott Williams & Wilkins, Philadelphia, and Encyclopedia of Pharmaceutical Technology, eds. J. Swarbrick and J. C. Boylan, 1988-1999, Marcel Dekker, New York discloses various carriers used in formulating pharmaceutical compositions and known techniques for the preparation thereof. Except insofar as any conventional carrier is incompatible with the compounds of this disclosure, such as by producing any undesirable biological effect or otherwise interacting in a deleterious manner with any other component(s) of the pharmaceutical composition, its use is contemplated to be within the scope of this disclosure. Non-limiting examples of suitable pharmaceutically acceptable carriers include, but are not limited to, ion exchangers, alumina, aluminum stearate, lecithin, serum proteins (such as human serum albumin), buffer substances (such as phosphates, glycine, sorbic acid, and potassium sorbate), partial glyceride mixtures of saturated vegetable fatty acids, water, salts, and electrolytes (such as protamine sulfate, disodium hydrogen phosphate, potassium hydrogen phosphate, sodium chloride, and zinc salts), colloidal silica, magnesium trisilicate, polyvinyl pyrrolidone, polyacrylates, waxes, polyethylene-polyoxypropylene-block polymers, wool fat, sugars (such as lactose, glucose and sucrose), starches (such as corn starch and potato starch), cellulose and its derivatives (such as sodium carboxymethyl cellulose, ethyl cellulose and cellulose acetate), powdered tragacanth, malt, gelatin, talc, excipients (such as cocoa butter and suppository waxes), oils (such as peanut oil, cottonseed oil, safflower oil, sesame oil, olive oil, corn oil and soybean oil), glycols (such as propylene glycol and polyethylene glycol), esters (such as ethyl oleate and ethyl laurate), agar, buffering agents (such as magnesium hydroxide and aluminum hydroxide), alginic acid, pyrogen-free water, isotonic saline, Ringer's solution, ethyl alcohol, phosphate buffer solutions, non-toxic compatible lubricants (such as sodium lauryl sulfate and magnesium stearate), coloring agents, releasing agents, coating agents, sweetening agents, flavoring agents, perfuming agents, preservatives, and antioxidants.

Exemplary embodiments of the disclosure include:
1. A method of treating cystic fibrosis comprising administering to a patient in need thereof:
   (A) 5 mg to 20 mg of at least one compound chosen from Compound I 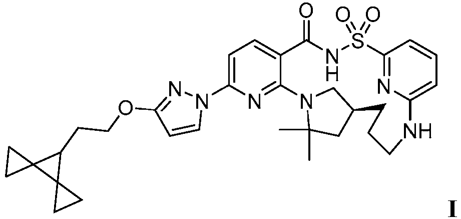 and pharmaceutically acceptable salts thereof daily; and
   (B) at least one compound chosen from:
      (i) 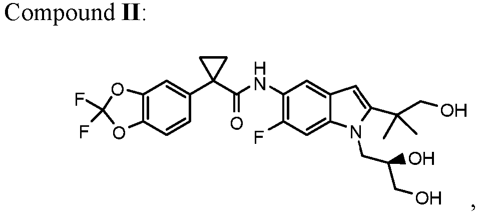 and pharmaceutically acceptable salts thereof, and
      (ii)
         (a) 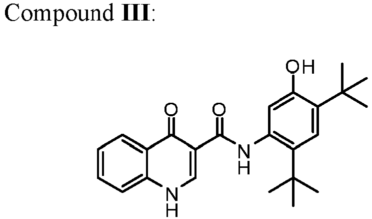 and pharmaceutically acceptable salts thereof or
         (b) 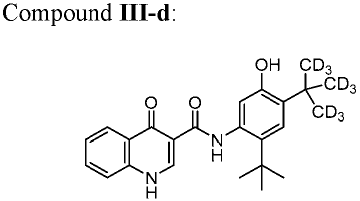 and pharmaceutically acceptable salts thereof.
2. The method according to embodiment 1, comprising administering to said patient:
   (A) at least one compound chosen from Compound I and pharmaceutically acceptable salts thereof;
   (B) at least one compound chosen from Compound **II** and pharmaceutically acceptable salts thereof; and
   (C) at least one compound chosen from (i) Compound **III** and pharmaceutically acceptable salts thereof, or (ii) Compound **III-d** and pharmaceutically acceptable salts thereof.
3. The method according to embodiment 1, comprising administering to said patient:
   (A) at least one compound chosen from Compound **I** and pharmaceutically acceptable salts thereof;
   (B) at least one compound chosen from Compound **II** and pharmaceutically acceptable salts thereof; and
   (C) at least one compound chosen from Compound **III** and pharmaceutically acceptable salts thereof.
4. The method according to embodiment 1, comprising administering to said patient:
   (A) at least one compound chosen from Compound **I** and pharmaceutically acceptable salts thereof;
   (B) at least one compound chosen from Compound **II** and pharmaceutically acceptable salts thereof; and
   (C) at least one compound chosen from Compound **III-d** and pharmaceutically acceptable salts thereof.
5. The method according to embodiment 1, wherein the at least one compound chosen from Compound **I** and pharmaceutically acceptable salts thereof, is administered in a single composition with the at least one compound chosen from Compound **II,** Compound **III,** Compound **III-d,** and pharmaceutically acceptable salts thereof.
6. The method according to embodiment 1, wherein the at least one compound chosen from Compound **I** and pharmaceutically acceptable salts thereof, and the at least one compound chosen from Compound **II,** Compound **III,** Compound **III-d,** and pharmaceutically acceptable salts thereof, are administered in one or more separate compositions.
7. The method according to embodiment 1, wherein a first pharmaceutical composition comprising at least one compound chosen from Compound **I** and pharmaceutically acceptable salts thereof is administered in combination with a second pharmaceutical composition comprising at least one compound chosen from Compound **II,** Compound **III,** Compound **III-d,** and pharmaceutically acceptable salts thereof.
8. The method according to embodiment 1, wherein a first pharmaceutical composition comprising at least one compound chosen from Compound **I** and pharmaceutically acceptable salts thereof is administered in combination with a second pharmaceutical composition comprising (a) at least one compound chosen from Compound **II** and pharmaceutically acceptable salts thereof; and (b) at least one compound chosen from Compound **III,** Compound **III-d,** and pharmaceutically acceptable salts thereof.
9. The method according to embodiment 1, wherein a first pharmaceutical composition comprising at least one compound chosen from Compound **I** and pharmaceutically acceptable salts thereof is administered in combination with a second pharmaceutical composition comprising Compound **II** and Compound **III-d.**
10. The method according to embodiment 1, comprising administering a single composition comprising Compound **I** or a pharmaceutically acceptable salt thereof, Compound **II,** and Compound **III-d.**
11. The method according to any one of embodiments 1-10, wherein 5 mg, 10 mg, or 20 mg of Compound **I** or an equivalent amount of a pharmaceutically acceptable salt thereof is administered daily.
12. The method according to embodiment 11, wherein Compound **I** or a pharmaceutically acceptable salt thereof is administered as a single dose, once daily.
13. The method according to embodiment 11, wherein Compound **I** or a pharmaceutically acceptable salt thereof is administered in two doses daily.
14. The method according to any one of embodiments 1-11, wherein 20 mg of Compound **I** or an equivalent amount of a pharmaceutically acceptable salt thereof is administered in one to four doses daily.
15. The method according to any one of embodiments 1-14, wherein 50 mg to 150 mg of Compound **II** or an equivalent amount of a pharmaceutically acceptable salt thereof is administered daily.
16. The method according to embodiment 15, wherein 50 mg of Compound **II** or or an equivalent amount of a pharmaceutically acceptable salt thereof per dosing is administered once daily or twice daily.
17. The method according to any one of embodiments 1-14, wherein 100 mg of Compound **II** or or an equivalent amount of a pharmaceutically acceptable salt thereof is administered daily.
18. The method according to embodiment 17, wherein 100 mg of Compound **II** or or an equivalent amount of a pharmaceutically acceptable salt thereof is administered as a single dose, once daily.
19. The method according to embodiment 17, wherein the 100 mg of Compound **II** or an equivalent amount of a pharmaceutically acceptable salt thereof is administered in two doses daily.
20. The method according to any one of embodiments 1-17, wherein:
   (a) 150 mg to 300 mg of Compound **III** or an equivalent amount of a pharmaceutically acceptable salt thereof is administered daily; or
   (b) 50 mg to 150 mg of Compound **III-d** or an equivalent amount of a pharmaceutically acceptable salt thereof is administered daily.
21. The method according to embodiment 18, wherein 150 mg to 300 mg of Compound **III** or an equivalent amount of a pharmaceutically acceptable salt thereof is administered daily.
22. The method according to embodiment 18, wherein 50 mg to 150 mg of Compound **III-d** or an equivalent amount of a pharmaceutically acceptable salt thereof is administered daily.
23. The method according to embodiment 20, wherein:
   (a) 150 mg of Compound **III** or an equivalent amount of a pharmaceutically acceptable salt thereof is administered twice daily; or
   (b) 150 mg of Compound **III-d** or an equivalent amount of a pharmaceutically acceptable salt thereof is administered daily.
24. The method according to embodiment 20, wherein the 150 mg daily amount of Compound **III-d** or an equivalent amount of a pharmaceutically acceptable salt thereof is administered in one, two, or three doses daily.
25. The method according to embodiment 20, wherein the 300 mg daily amount of Compound **III** or an equivalent amount of a pharmaceutically acceptable salt thereof is administered in one, two, three, or four doses.
26. A method of treating cystic fibrosis comprising administering daily to a patient in need thereof:
   (a) 5 mg to 20 mg of Compound **I** or an equivalent amount of a pharmaceutically acceptable salt thereof;
   (b) 50 mg to 100 mg of Compound **II** or an equivalent amount of a pharmaceutically acceptable salt thereof; and
   (c)
      (i)150 mg to 300 mg of Compound **III** or an equivalent amount of a pharmaceutically acceptable salt thereof; or
      (ii) 50 mg to 150 mg of Compound **III-d** or an equivalent amount of a pharmaceutically acceptable salt thereof.
27. The method of embodiment 26, wherein the method comprises administering:
   (a) 5 mg, 10 mg, or 20 mg of Compound **I** or an equivalent amount of a pharmaceutically acceptable salt thereof;
   (b) 100 mg of Compound **II** or an equivalent amount of a pharmaceutically acceptable salt thereof; and
   (c)
      (i) 300 mg of Compound **III** or an equivalent amount of a pharmaceutically acceptable salts thereof; or
      (ii) 150 mg of Compound **III-d** or an equivalent amount of a pharmaceutically acceptable salt thereof.
28. The method of embodiment 26, wherein the method comprises administering:
   (a) 5 mg of Compound **I** or an equivalent amount of a pharmaceutically acceptable salt thereof;
   (b) 100 mg of Compound **II** or an equivalent amount of a pharmaceutically acceptable salt thereof; and
   (c) 300 mg of Compound **III** or an equivalent amount of a pharmaceutically acceptable salt thereof.
29. The method of embodiment 26, wherein the method comprises administering:
   (a) 10 mg of Compound **I** or an equivalent amount of a pharmaceutically acceptable salt thereof;
   (b) 100 mg of Compound **II** or an equivalent amount of a pharmaceutically acceptable salt thereof; and
   (c) 300 mg of Compound **III** or an equivalent amount of a pharmaceutically acceptable salt thereof.
30. The method of embodiment 26, wherein the method comprises administering:
   (a) 20 mg of Compound **I** or an equivalent amount of a pharmaceutically acceptable salt thereof;
   (b) 100 mg of Compound **II** or an equivalent amount of a pharmaceutically acceptable salt thereof; and
   (c) 300 mg of Compound **III** or an equivalent amount of a pharmaceutically acceptable salts thereof.
31. The method of embodiment 26, wherein the method comprises administering:
   (a) 5 mg of Compound **I** or an equivalent amount of a pharmaceutically acceptable salt thereof;
   (b) 100 mg of Compound **II** or an equivalent amount of a pharmaceutically acceptable salt thereof; and
   (c) 150 mg of Compound **III-d** or an equivalent amount of a pharmaceutically acceptable salt thereof.
32. The method of embodiment 26, wherein the method comprises administering:
   (a) 10 mg of Compound **I** or an equivalent amount of a pharmaceutically acceptable salt thereof;
   (b) 100 mg of Compound **II** or an equivalent amount of a pharmaceutically acceptable salt thereof; and
   (c) 150 mg of Compound **III-d** or an equivalent amount of a pharmaceutically acceptable salts thereof.
33. The method of embodiment 26, wherein the method comprises administering:
   (a) 20 mg of Compound **I** or an equivalent amount of a pharmaceutically acceptable salt thereof;
   (b) 100 mg of Compound **II** or an equivalent amount of a pharmaceutically acceptable salt thereof; and
   (c) 150 mg of Compound **III-d** or an equivalent amount of a pharmaceutically acceptable salt thereof.
34. A method of treating cystic fibrosis comprising daily administering to a patient in need thereof:
   (a) a first pharmaceutical composition comprising 5 mg to 20 mg of Compound **I** or an equivalent amount of a pharmaceutically acceptable salt thereof; and
   (b) a second pharmaceutical composition daily comprising
      (i) 50 to 100 mg of Compound **II** or an equivalent amount of a pharmaceutically acceptable salt thereof; and
      (ii) either (1)150 mg to 300 mg of Compound **III** or an equivalent amount of a pharmaceutically acceptable salt thereof or (2) 50 mg to 150 mg of Compound **III-d** or an equivalent amount of a pharmaceutically acceptable salt thereof.
35. The method of embodiment 34, wherein the method comprises:
   (a) administering daily to a patient in need thereof a first pharmaceutical composition comprising 5 mg, 10mg, or 20 mg of Compound **I** or an equivalent amount of a pharmaceutically acceptable salt thereof; and
   (b) administering daily to the patient a second pharmaceutical composition comprising (i) 100 mg of Compound **II** or an equivalent amount of a pharmaceutically acceptable salt thereof; and (ii) 150 mg of Compound **III** or an equivalent amount of a pharmaceutically acceptable salt thereof; and
   (c) administering daily to the patient a third pharmaceutical composition comprising 150 mg of Compound **III** or an equivalent amount of a pharmaceutically acceptable salt thereof.
36. The method of embodiment 34, wherein the method comprises administering daily to a patient in need thereof:
   (a) a first pharmaceutical composition comprising 5 mg, 10 mg, or 20 mg of Compound **I** or an equivalent amount of a pharmaceutically acceptable salt thereof; and
   (b) a second pharmaceutical composition comprising (i) 100 mg of Compound **II** or an equivalent amount of a pharmaceutically acceptable salt thereof; and (ii) 150 mg of Compound **III-d** or an equivalent amount of a pharmaceutically acceptable salt thereof.
37. A method of treating cystic fibrosis comprising administering daily to a patient in need thereof a first pharmaceutical composition comprising
   (a) 5 mg to 20 mg of Compound **I** or an equivalent amount of a pharmaceutically acceptable salt thereof,
   (b) 100 mg of Compound **II** or an equivalent amount of a pharmaceutically acceptable salt thereof, and
   (c) 150 mg of Compound **III** or an equivalent amount of a pharmaceutically acceptable salt thereof; and
   subsequently administering to the patient daily a second pharmaceutical composition comprising 150 mg of Compound **III** or an equivalent amount of a pharmaceutically acceptable salt thereof.
38. The method according to any one of embodiments 1-37, wherein the patient is heterozygous for the F508del CFTR mutation.
39. The method according to any one of embodiments 1-37, wherein the patient is homozygous for the F508del CFTR mutation.
40. The method of any one of embodiments 1-39, wherein the method comprises administering a pharmaceutically acceptable salt of Compound **I.**
41. The method of any one of embodiments 1-40, wherein the method comprises administering Compound **II.**
42. The method of any one of embodiments 1-3, 5-8, 11-21, 23, 25-30, 34, 35, and 37, wherein the method comprises administering Compound **III.**
43. The method of any one of embodiments 1, 2, 4-8, 11-20, 22-24, 26, 27, 31-34, and 36, wherein the method comprises administering Compound **III-d.**
44. A pharmaceutical composition comprising 5 mg to 20 mg of Compound **I** or an equivalent amount of a pharmaceutically acceptable salt thereof, and optionally comprising one or more additional CFTR modulating agents.
45. The pharmaceutical composition of embodiment 44 further comprising:
   (a) 50 to 100 mg of Compound **II** or an equivalent amount of a pharmaceutically acceptable salt thereof; and
   (b)
      (i)150 mg to 300 mg of Compound **III** or an equivalent amount of a pharmaceutically acceptable salt thereof or
      (ii) 50 mg to 150 mg of Compound **III-d** or an equivalent amount of a pharmaceutically acceptable salt thereof.
46. The pharmaceutical composition of embodiment 44 further comprising:
   (a) 100 mg of Compound **II** or an equivalent amount of a pharmaceutically acceptable salt thereof; and
   (b)
      (i) 150 mg of Compound **III** or an equivalent amount of a pharmaceutically acceptable salt thereof, or
      (ii) 150 mg of Compound **III-d** or an equivalent amount of a pharmaceutically acceptable salt thereof.
47. The pharmaceutical composition of embodiment 44 further comprising:
   (a) 100 mg of Compound **II** or an equivalent amount of a pharmaceutically acceptable salt thereof; and
   (b) 150 mg of Compound **III** or an equivalent amount of a pharmaceutically acceptable salt thereof.
48. The pharmaceutical composition of embodiment 44 further comprising:
   (a) 100 mg of Compound **II** or an equivalent amount of a pharmaceutically acceptable salt thereof; and
   (b) 150 mg of Compound **III-d** or an equivalent amount of a pharmaceutically acceptable salt thereof.
49. The pharmaceutical composition of any one of embodiments 44-48, wherein the composition comprises 5 mg, 10 mg, or 20 mg of Compound **I** or an equivalent amount of a pharmaceutically acceptable salt thereof.

### Methods of Preparing Compounds

### General Experimental Procedures

Reagents and starting materials were obtained by commercial sources unless otherwise stated and were used without purification. Proton and carbon NMR spectra were acquired on either of a Bruker Biospin DRX 400 MHz FTNMR spectrometer operating at a ¹H and ¹³C resonant frequency of 400 and 100 MHz respectively, or on a 300 MHz NMR spectrometer. One dimensional proton and carbon spectra were acquired using a broadband observe (BBFO) probe with 20 Hz sample rotation at 0.1834 and 0.9083 Hz/Pt digital resolution respectively. All proton and carbon spectra were acquired with temperature control at 30 °C using standard, previously published pulse sequences and routine processing parameters. Final purity of compounds was determined by reversed phase UPLC using an Acquity UPLC BEH C₁₈ column (50 × 2.1 mm, 1.7 µm particle) made by Waters (pn: 186002350), and a dual gradient run from 1-99% mobile phase B over 3.0 minutes. Mobile phase A = H₂O (0.05 % CF₃CO₂H). Mobile phase B = CH₃CN (0.035 % CF₃CO₂H). Flow rate = 1.2 mL/min, injection volume = 1.5 µL, and column temperature = 60 °C. Final purity was calculated by averaging the area under the curve (AUC) of two UV traces (220 nm, 254 nm). Low-resolution mass spectra were obtained using a single quadrupole mass spectrometer with a mass accuracy of 0.1 Da and a minimum resolution of 1000 amu across the detection range using electrospray ionization (ESI) using the hydrogen ion (H⁺). Optical purity of methyl (2S)-2,4-dimethyl-4-nitro-pentanoate was determined using chiral gas chromatography (GC) analysis on an Agilent 7890A/MSD 5975C instrument, using a Restek Rt-βDEXcst (30m x 0.25mm x 0.25um_df) column, with a 2.0 mL/min flow rate (H₂ carrier gas), at an injection temperature of 220°C and an oven temperature of 120°C, 15 minutes. Purity of Compound I was determined by reverse phase HPLC using an Poroshell 120 EC-C8 column (4.6 × 150 mm, 2.7 µm particle, and a dual gradient run from 30-95% mobile phase B over 40 minutes. Mobile phase A = 5 mM Ammonium Acetate pH 4.50 and Mobile phase B = Acetonitrile. Flow rate = 1.0 mL/min, injection volume = 5 µL, 254 nm, and column temperature = 30 °C.

Compounds II, III, **III-d,** and IV can be prepared by any suitable method in the art, for example, PCT Publication Nos. WO 2011/133751, WO 2011/133951, WO 2015/160787 and US Patent No. 8,865,902.

### Example 1: Synthesis of (14S)-8-[3-(2-{dispiro[2.0.2.1]heptan-7-yl}ethoxy)-1H-pyrazol-1-yl]-12,12-dimethyl-2λ6-thia-3,9,11,18,23-pentaazatetracyclo [17.3.1.111,14.05,10]tetracosa-1(22),5,7,9,19(23),20-hexaene-2,2,4-trione (Compound I)

### General UPLC/HPLC Analytical Methods:

Unless indicated, yields of enantiomers separated by chiral SFC are given as a percentage of the theoretical yield for a single enantiomer of the racemate.

**LC Method A:** Analytical reverse phase UPLC using an Acquity UPLC BEH C₁₈ column (30 × 2.1 mm, 1.7 µm particle) made by Waters (pn: 186002349), and a dual gradient run from 1-99% mobile phase B over 1.2 minutes. Mobile phase A = water (0.05% trifluoroacetic acid). Mobile phase B = acetonitrile (0.035% trifluoroacetic acid). Flow rate = 1.5 mL/min, injection volume = 1.5 µL, and column temperature = 60 °C.

**LC Method B:** Analytical reverse phase UPLC using an Acquity UPLC BEH C₁₈ column (50 × 2.1 mm, 1.7 µm particle) made by Waters (pn: 186002350), and a dual gradient run from 1-99% mobile phase B over 3.0 minutes. Mobile phase A = water (0.05% trifluoroacetic acid). Mobile phase B = acetonitrile (0.035% trifluoroacetic acid). Flow rate = 1.2 mL/min, injection volume = 1.5 µL, and column temperature = 60 °C.

**LC Method D:** Analytical reverse phase UPLC using an Acquity UPLC BEH C₁₈ column (50 × 2.1 mm, 1.7 µm particle) made by Waters (pn: 186002350), and a dual gradient run from 1-99% mobile phase B over 5.0 minutes. Mobile phase A = water (0.05% trifluoroacetic acid). Mobile phase B = acetonitrile (0.035% trifluoroacetic acid). Flow rate = 1.2 mL/min, injection volume = 1.5 µL, and column temperature = 60 °C.

**LC Method** F: Analytical reverse phase UPLC using an Acquity UPLC BEH C₁₈ column (50 × 2.1 mm, 1.7 µm particle) made by Waters (pn: 186002350), and a dual gradient run from 1-99% mobile phase B over 15.0 minutes. Mobile phase A = water (0.05% trifluoroacetic acid). Mobile phase B = acetonitrile (0.035% trifluoroacetic acid). Flow rate = 1.2 mL/min, injection volume = 1.5 µL, and column temperature = 60 °C.

**LC Method Q:** Merckmillipore Chromolith SpeedROD C₁₈ column (50 x 4.6 mm) and a dual gradient run from 5 - 100% mobile phase B over 12 minutes. Mobile phase A = water (0.1 % trifluoroacetic acid). Mobile phase B = acetonitrile (0.1 % trifluoroacetic acid).

### Part A: Synthesis of 2-Chloro-6-[3-(2-dispiro[2.0.2.1]heptan-7-ylethoxy)pyrazol-1-yl]pyridine-3-carboxylic acid

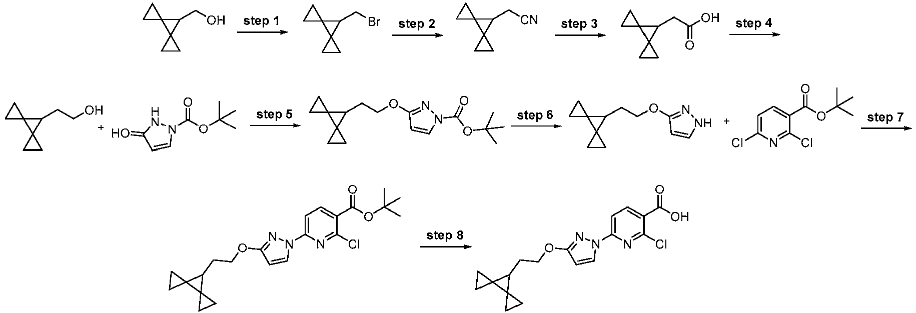

### Step 1: 7-(Bromomethyl)dispiro[2.0.2.1]heptane

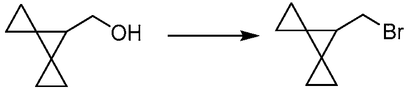

A 1000 mL, 3-neck round bottom flask was fitted with a mechanical stirrer, a cooling bath, an addition funnel, a J-Kem temperature probe and a nitrogen inlet/outlet. The vessel was charged under a nitrogen atmosphere with triphenylphosphine (102.7 mL, 443.2 mmol) and dichloromethane (1 L) which provided a clear colorless solution. Stirring was commenced and the cooling bath was charged with acetone. Dry ice was added in portions to the cooling bath until a pot temperature of -15 °C was obtained. The addition funnel was charged with a solution of bromine (22.82 mL, 443.0 mmol) in dichloromethane (220 mL, 10 mL/g) which was subsequently added dropwise over 1 h. Dry ice was added in portions to the cooling bath during the addition to maintain the pot temperature at -15 °C. After the addition of bromine was completed, the pale yellow suspension was continued to stir at -15 °C for 15 min at which point the suspension was cooled to -30 °C. The addition funnel was charged with a solution of dispiro[2.0.2.1]heptan-7-yl methanol (50 g, 402.6 mmol), pyridine (35.82 mL, 442.9 mmol) and dichloromethane (250 mL, 5 mL/g). The clear pale yellow solution was then added dropwise over 1.5 h maintaining the pot temperature at -30 °C. The resulting clear light yellow reaction mixture was allowed to gradually warm to a pot temperature of -5 °C and then continued to stir at -5 °C for 1 h. The reaction mixture then was poured into hexane (2000 mL) which resulted in the formation of a precipitate. The suspension was stirred at room temperature for 30 min and then filtered through a glass frit Buchner funnel with a 20 mm layer of celite. The clear filtrate was concentrated under reduced pressure (water bath temperature at 20 °C) to provide a yellow oil with some precipitate present. The oil was diluted with some hexane, allowed to stand at room temperature for 15 min and then filtered through a glass frit Buchner funnel with a 20 mm layer of celite. The clear filtrate was concentrated under reduced pressure (water bath temperature at 20 °C) to provide 7-(bromomethyl)dispiro[2.0.2.1]heptane (70 g, 93%) as a clear yellow oil. ¹H NMR (400 MHz, Chloroform-d) δ 3.49 (d, J = 7.5 Hz, 2H), 1.90 (t, J = 7.5 Hz, 1H), 1.06 - 0.84 (m, 4H), 0.71 (ddd, J = 9.1, 5.1, 4.0 Hz, 2H), 0.54 (dddd, J = 8.6, 4.8, 3.8, 1.0 Hz, 2H).

### Step 2: 2-Dispiro[2.0.2.1]heptan-7-ylacetonitrile

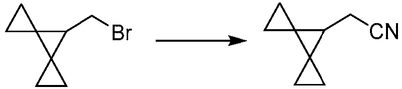

A 1000 mL, 3-neck round bottom flask was fitted with a mechanical stirrer, a cooling bath used as secondary containment, a J-Kem temperature probe and a nitrogen inlet/outlet. The vessel was charged under a nitrogen atmosphere with 7-(bromomethyl)dispiro[2.0.2.1]heptane (35 g, 187.1 mmol) and dimethyl sulfoxide (245 mL) which provided a clear amber solution. Stirring was commenced and the pot temperature was recorded at 19 °C. The vessel was then charged with sodium cyanide (11.46 g, 233.8 mmol) added as a solid in one portion which resulted in a dark solution and a gradual exotherm to 49 °C over 15 min. After a few min the pot temperature began to decrease and the mixture was continued to stir at room temperature overnight (about 15 h). The dark reaction mixture was quenched with ice cold saturated sodium carbonate solution (500 mL) and then transferred to a separatory funnel and partitioned with diethyl ether (500 mL). The organic was removed and the residual aqueous was extracted with diethyl ether (2 X 250 mL). The combined organics were washed with water (500 mL), dried over sodium sulfate (200 g) and then filtered through a glass frit Buchner funnel. The clear amber filtrate was concentrated under reduced pressure (water bath temperature 20 °C) to provide 2-dispiro[2.0.2.1]heptan-7-ylacetonitrile (21 g, 84%) as a clear dark amber oil. ¹H NMR (400 MHz, Chloroform-d) δ 2.42 (d, J = 6.6 Hz, 2H), 1.69 (t, J = 6.6 Hz, 1H), 1.02 - 0.88 (m, 4H), 0.79 - 0.70 (m, 2H), 0.66 - 0.55 (m, 2H).

### Step 3: 2-Dispiro[2.0.2.1]heptan-7-ylacetic acid

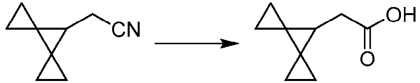

To a solution of 2-dispiro[2.0.2.1]heptan-7-ylacetonitrile (2.1 g, 14.19 mmol) in EtOH (32 mL) was added sodium hydroxide (5.12 g, 128.0 mmol) followed by water (13 mL) and the resulting solution was stirred and heated to 70 °C overnight. The mixture was then cooled to room temperature, diluted with water and extracted with diethyl ether. The aqueous phase was adjusted to pH = 1 by the addition of 6 N hydrochloric acid (resulting in a cloudy precipitate) and extracted with diethyl ether (3X). The organic phases were dried (magnesium sulfate), filtered and concentrated giving 2-dispiro[2.0.2.1]heptan-7-ylacetic acid (2.19 g, 99% yield, 98% purity) as an orange solid which was used in the next step without further purification. ¹H NMR (400 MHz, Chloroform-d) δ 2.44 (d, J = 6.9 Hz, 2H), 1.67 (t, J = 6.9 Hz, 1H), 0.91 (ddd, J = 9.0, 5.2, 3.9 Hz, 2H), 0.81 (dddd, J = 8.9, 5.2, 3.9, 0.5 Hz, 2H), 0.69 (ddd, J = 8.9, 5.2, 3.9 Hz, 2H), 0.56 - 0.44 (m, 2H).

### Step 4: 2-Dispiro[2.0.2.1]heptan-7-ylethanol

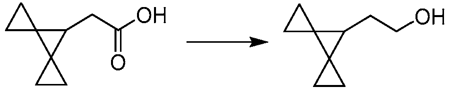

To lithium aluminum hydride (827.4 mg, 902.3 µL, 21.80 mmol) dissolved in tetrahydrofuran (33.71 mL) cooled in an ice/water bath was added 2-dispiro[2.0.2.1]heptan-7-ylacetic acid (2.552 g, 16.77 mmol) in tetrahydrofuran (7.470 mL) dropwise over 15 min keeping the reaction temperature < 20 °C. The mixture was allowed to stir a total of 18 h, gradually warming to ambient temperature. The mixture was cooled with an ice/water bath and sequentially quenched with slow addition of water (838.4 mg, 838.4 µL, 46.54 mmol), followed by sodium hydroxide (1.006 mL of 5 M, 5.031 mmol), then water (2.493 g, 2.493 mL, 138.4 mmol) affording a white, granular slurry which was filtered over celite. Washed the filtered solid with diethyl ether. The filtrate was concentrated *in vacuo* at ~ 300 mbar and 30 °C water bath. Diluted the residue with diethyl ether, dried (magnesium sulfate), filtered and concentrated *in vacuo* at ~ 300 mbar and 30 °C water bath followed by ~ 30 seconds under vacuum to give 2-dispiro[2.0.2.1]heptan-7-ylethanol (2.318 g, 100%) which was used directly in the ensuing step without further purification. ¹H NMR (400 MHz, Chloroform-d) δ 3.64 (s, 2H), 1.68 (d, J = 6.7 Hz, 2H), 1.39 (s, 1H), 1.31 (s, 1H), 0.82 (d, J = 14.0 Hz, 4H), 0.65 (s, 2H), 0.50 (d, J = 3.6 Hz, 2H).

### Step 5: tert-Butyl 3-(2-dispiro[2.0.2.1]heptan-7-ylethoxy)pyrazole-1-carboxylate

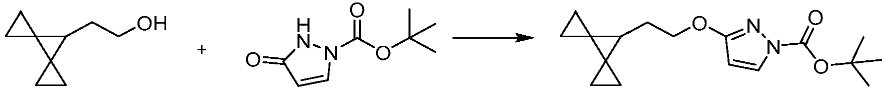

To a solution of tert-butyl 5-oxo-1H-pyrazole-2-carboxylate (2.942 g, 15.97 mmol) and 2-dispiro[2.0.2.1]heptan-7-ylethanol (2.318 g, 16.77 mmol) in tetrahydrofuran (36.78 mL) was added triphenylphosphine (4.399 g, 16.77 mmol). To the mixture was slowly added diisopropyl azodicarboxylate (3.391 g, 3.302 mL, 16.77 mmol) dropwise over 10 min (mild exotherm noted). The reaction mixture was stirred at room temperature for 30 min then at 50 °C for 30 min. The tetrahydrofuran was removed *in vacuo.* To the crude residue was added toluene (23.54 mL) and the mixture was stirred overnight as a precipitate gradually crystallized. Slurried with Celite then the precipitate was filtered off and washed with toluene (8.705 mL) and again with toluene (8.705 mL). The filtrate was concentrated *in vacuo.* The crude product was purified by silica gel chromatography using a shallow gradient from 100% hexanes to 100% ethyl acetate giving tert-butyl 3-(2-dispiro[2.0.2.1]heptan-7-ylethoxy)pyrazole-1-carboxylate (3.449 g, 71%). ESI-MS m/z calc. 304.17868, found 305.1 (M+1)+; Retention time: 0.82 min (LC Method A).

### Step 6: 3-(2-Dispiro[2.0.2.1]heptan-7-ylethoxy)-1H-pyrazole

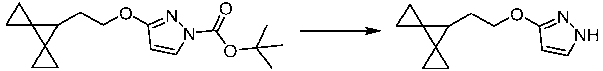

tert-Butyl 3-(2-dispiro[2.0.2.1]heptan-7-ylethoxy)pyrazole-1-carboxylate (5.304 g, 17.43 mmol) was dissolved in dichloromethane (53.04 mL) with trifluoroacetic acid (29.81 g, 20.14 mL, 261.4 mmol) and the reaction was stirred at room temperature for 120 min. The reaction was evaporated and the resulting oil was partitioned between ethyl acetate and a saturated sodium bicarbonate solution and the layers separated. The aqueous portion was extracted two additional times with ethyl acetate, then the organics were combined, washed with brine, dried over sodium sulfate, filtered and evaporated to give an oil, 3-(2-dispiro[2.0.2.1]heptan-7-ylethoxy)-1H-pyrazole (3.56 g, 100%). ESI-MS m/z calc. 204.12627, found 205.1 (M+1)+; Retention time: 0.59 min (LC Method A).

### Step 7: tert-Butyl 2-chloro-6-[3-(2-dispiro[2.0.2.1]heptan-7-ylethoxy)pyrazol-1-yl]pyridine-3-carboxylate

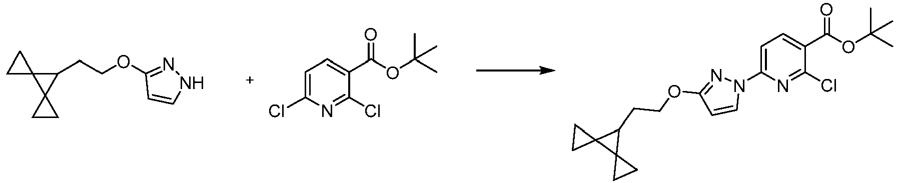

*tert*-Butyl 2,6-dichloropyridine-3-carboxylate (4.322 g, 17.42 mmol), 3-(2-dispiro[2.0.2.1]heptan-7-ylethoxy)-1H-pyrazole (3.559 g, 17.42 mmol) and potassium carbonate (2.891 g, 20.92 mmol) were combined in anhydrous dimethyl sulfoxide (71.18 mL). 1,4-Diazabicyclo[2.2.2]octane (391.1 mg, 3.487 mmol) was added and the mixture was stirred at room temperature under nitrogen for 16 h. The reaction mixture was diluted with water (136.9 mL) and stirred for 15 min. The resulting white solid was filtered and washed with water. The solid was dissolved in dichloromethane and dried over magnesium sulfate. The mixture was filtered and evaporated to give *tert*-butyl 2-chloro-6-[3-(2-dispiro[2.0.2.1]heptan-7-ylethoxy)pyrazol-1-yl]pyridine-3-carboxylate (5.69 g, 79%) as a white solid. ¹H NMR (400 MHz, Chloroform-d) δ 8.35 (d, J = 2.9 Hz, 1H), 8.18 (d, J = 8.4 Hz, 1H), 7.69 (d, J = 8.4 Hz, 1H), 5.94 (d, J = 2.9 Hz, 1H), 4.25 (s, 2H), 1.90 (d, J = 6.8 Hz, 2H), 1.62 (s, 9H), 1.49 (t, J = 6.6 Hz, 1H), 0.85 (d, J = 1.5 Hz, 4H), 0.65 (d, J = 1.5 Hz, 2H), 0.52 (d, J = 1.1 Hz, 2H). ESI-MS m/z calc. 415.16626, found 360.0 (M-tBu)+; Retention time: 2.09 min (LC Method B).

### Step 8: 2-Chloro-6-[3-(2-dispiro[2.0.2.1]heptan-7-ylethoxy)pyrazol-1-yl]pyridine-3-carboxylic acid

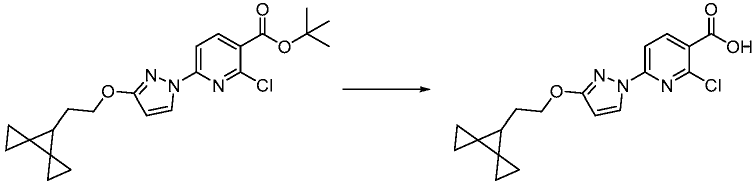

*tert*-Butyl 2-chloro-6-[3-(2-dispiro[2.0.2.1]heptan-7-ylethoxy)pyrazol-1-yl]pyridine-3-carboxylate (5.85 g, 14.07 mmol) was dissolved in dichloromethane (58.5 mL) with trifluoroacetic acid (16.26 mL, 211.1 mmol) and the reaction was stirred at room temperature for 16 h. The reaction was evaporated and to the resulting solid was added ether and then removed the ether under reduced pressure. This evaporation from ether was repeated twice more resulting in a white solid, 2-chloro-6-[3-(2-dispiro[2.0.2.1]heptan-7-ylethoxy)pyrazol-1-yl]pyridine-3-carboxylic acid (5.06 g, 100%). ¹H NMR (400 MHz, Chloroform-d) δ 8.41 (d, J = 8.5 Hz, 1H), 8.37 (d, J = 2.9 Hz, 1H), 7.75 (d, J = 8.5 Hz, 1H), 5.97 (d, J = 2.9 Hz, 1H), 4.27 (s, 2H), 1.91 (d, J = 6.7 Hz, 2H), 1.50 (s, 1H), 0.85 (d, J = 1.5 Hz, 4H), 0.71 - 0.62 (m, 2H), 0.52 (d, J = 1.1 Hz, 2H). ESI-MS m/z calc. 359.10367, found 360.2 (M+1)+; Retention time: 2.16 min (LC Method B).

### Part B: Synthesis of tert-Butyl (4S)-2,2-dimethyl-4-[3-[(6-sulfamoyl-2-pyridyl)amino]propyl]pyrrolidine-1-carboxylate

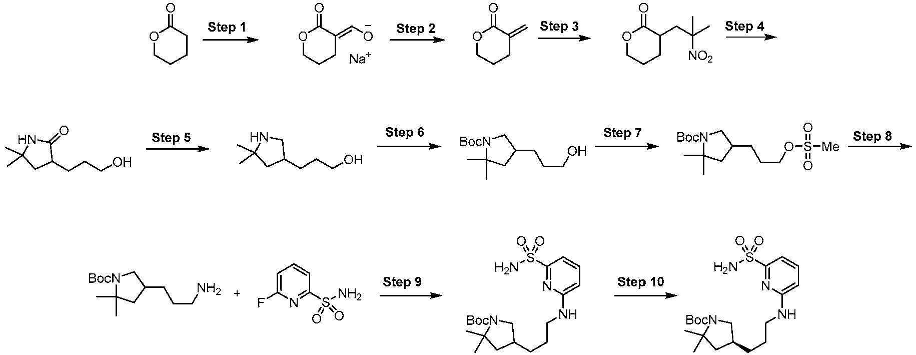

### Step 1: (E)-(2-Oxotetrahydropyran-3-ylidene)methanolate (sodium salt)

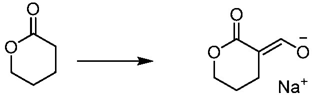

A 5 L, 3-neck round bottom flask was fitted with a mechanical stirrer, a heating mantle, an addition funnel, a J-Kem temperature probe/controller and a nitrogen inlet/outlet. The vessel was charged under a nitrogen atmosphere with sodium hydride (59.91 g of 60% w/w, 1.498 mol) followed by heptane (1.5 L) which provided a grey suspension. Stirring was commenced and the pot temperature was recorded at 19 °C. The vessel was then charged with ethyl alcohol (3.451 g, 74.91 mmol) added *via* syringe which resulted in gas evolution. The addition funnel was charged with a clear pale yellow solution of tetrahydropyran-2-one (150 g, 1.498 mol) and ethyl formate (111 g, 1.50 mol). The solution was added dropwise over 1 h which resulted in gas evolution and a gradual exotherm to 45 °C. The resulting thick white suspension was then heated to 65 °C for 2 h and then allowed to cool to room temperature. The mixture was continued to stir at room temperature overnight (about 10 h). The reaction mixture was vacuum filtered through a glass frit Buchner funnel (medium porosity) under a stream of nitrogen. The filter cake was displacement washed with heptane (2 X 250 mL) and pulled for a few min. The slightly heptane wet cake was transferred to a glass tray and dried in a vacuum oven at 45 °C for 15 h to provide a white solid (205 g, 1.36 mol, 91% yield) as the desired product, (*E*)-(2-oxotetrahydropyran-3-ylidene)methanolate (sodium salt).

### Step 2: 3-Methylenetetrahydropyran-2-one

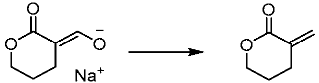

A 5 L, 3-neck round bottom flask was fitted with a mechanical stirrer, a heating mantle, an addition funnel, a J-Kem temperature probe/controller and a nitrogen inlet/outlet. The vessel was charged under a nitrogen atmosphere with (E)-(2-oxotetrahydropyran-3-ylidene)methanolate (sodium salt) (205 g, 1.366 mol) (205 g, 1.366 mol) and tetrahydrofuran (1640 mL) which provided a white suspension. Stirring was commenced and the pot temperature was recorded at 19 °C. The vessel was then charged with paraformaldehyde (136.6 g, 4.549 mol) added as a solid in one portion. The resulting suspension was heated to 63 °C and the condition was maintained for 15 h. Upon heating the reaction mixture became slightly gelatinous. The white gelatinous mixture was concentrated under reduced pressure to remove most of the tetrahydrofuran. The remaining residue was partitioned with ethyl acetate (1000 mL), saturated sodium chloride (500 mL) and saturated sodium hydrogen carbonate (500 mL) in a separatory funnel. The organic was removed and the residual aqueous was extracted with ethyl acetate (5 X 300 mL). The combined organic was dried over sodium sulfate (500 g) and then vacuum filtered through a glass frit Buchner funnel with a 20 mm layer of celite. The filter cake was displacement washed with ethyl acetate (250 mL). The clear filtrate was concentrated under reduced pressure to provide a clear pale yellow oil (135 g) as the desired crude product. The material was purified by silica gel column flash chromatography (liquid load) eluting with a gradient of 100% hexane to 60% ethyl acetate in hexane over 1 h collecting 450 mL fractions. The product was detected by TLC analysis on silica gel eluting with 3:1 hexanes/ethyl acetate and visualized under UV. The product fractions were combined and concentrated under reduced pressure to provide a clear, colorless oil (132 g, 1.18 mol, 72% yield containing 16 wt% residual ethyl acetate by NMR) as the desired product, 3-methylenetetrahydropyran-2-one. 1H NMR (400 MHz, dimethyl sulfoxide-d₆) δ 6.18 (q, J = 1.9 Hz, 1H), 5.60 (q, J = 1.9 Hz, 1H), 4.40 - 4.26 (m, 2H), 2.61 (ddt, J = 7.0, 6.3, 2.0 Hz, 2H), 1.90 - 1.75 (m, 2H).

### Step 3: 3-(2-Methyl-2-nitro-propyl)tetrahydropyran-2-one

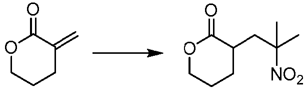

A 5000 mL, 3-neck round bottom flask was fitted with a mechanical stirrer, a cooling bath used as secondary containment, a J-Kem temperature probe, an addition funnel and a nitrogen inlet/outlet. The vessel was charged under a nitrogen atmosphere with 2-nitropropane (104.9 g, 1.177 mol) . Stirring was commenced and the pot temperature was recorded at 19 °C. The vessel was then charged with 1,8-diazabicyclo[5.4.0]undec-7-ene (22.41 g, 147.2 mmol) added neat in one portion which resulted in a clear light yellow solution. No exotherm was observed. The addition funnel was charged with a solution of 3-methylenetetrahydropyran-2-one (110 g, 981.0 mmol) in acetonitrile (1100 mL) which was added dropwise over 1 h which resulted in a clear light yellow solution and a gradual exotherm to 24 °C. The reaction mixture was continued to stir at room temperature for 3.5 h and then concentrated under reduced pressure. The remaining residue was dissolved in dichloromethane (1000 mL) and partitioned with 500 mL of a 3:2 mixture of 1 molar citric acid solution/saturated sodium chloride solution. The resulting organic phase was a clear pale blue solution and the aqueous phase was a slightly cloudy very pale blue solution. The organic was removed and the residual aqueous was extracted with dichloromethane (300 mL). The combined organic was washed with saturated sodium chloride solution (300 mL), dried over sodium sulfate (250 g) and then filtered through a glass frit Buchner funnel. The filtrate was concentrated under reduced pressure to a volume of about 200 mL. The clear pale blue dichloromethane solution was diluted with methyl tert-butyl ether (1500 mL) and the cloudy solution was concentrated under reduced pressure to a volume of about 200 mL which provided a suspension. The mixture was again diluted with methyl *tert*-butyl ether (1500 mL) and concentrated under reduced pressure to a volume of about 250 mL. The resulting suspension was allowed to stand at room temperature overnight (about 12 h). The solid was collected by vacuum filtration in a glass frit Buchner funnel and the filter cake was displacement washed with cold methyl tert-butyl ether (2 X 150 mL) and then pulled for 30 min. The material was further dried in a vacuum oven at 45 °C for 5 h to provide (160 g, 0.795 mol, 81% yield) of a white solid as the desired product, 3-(2-methyl-2-nitro-propyl)tetrahydropyran-2-one. ¹H NMR (400 MHz, dimethyl sulfoxide-d₆) δ 4.34 (ddd, J = 11.1, 9.3, 4.3 Hz, 1H), 4.20 (dt, J = 11.1, 5.1 Hz, 1H), 2.75 - 2.62 (m, 1H), 2.56 (dd, J = 14.9, 5.2 Hz, 1H), 2.01 - 1.89 (m, 2H), 1.89 - 1.67 (m, 2H), 1.55 (d, J = 6.0 Hz, 6H), 1.44 (dddd, J = 12.8, 11.5, 8.1, 6.6 Hz, 1H).

### Step 4: 3-(3-Hydroxypropyl)-5,5-dimethyl-pyrrolidin-2-one

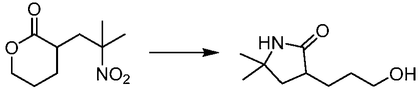

A 1000 mL, 3-neck round bottom flask was fitted with a Teflon stir bar, a heating mantle, a J-Kem temperature probe/controller and rubber septums. The vessel was charged with 3-(2-methyl-2-nitro-propyl)tetrahydropyran-2-one (25 g, 124.2 mmol) and ethyl alcohol (375 mL) which provided a white suspension. Stirring was commenced and the suspension was heated to 40 °C for 10 min which provided a clear colorless solution. The vessel was then fitted with a gas dispersion tube and the solution was degased with nitrogen for 15 min. The vessel was then charged with Raney Nickel (8.019 g of 50% w/w, 68.31 mmol) and the vessel was then fitted with the septums. The vessel was evacuated and placed under a hydrogen atmosphere. The process was repeated for three cycles. The vessel was then placed under 1 atmosphere hydrogen and the reaction mixture was gradually heated to 60 °C. The reaction was continued to stir at 60 °C for 24 h. After cooling to room temperature, the vessel was fitted with a gas dispersion tube and the reaction mixture was degased with nitrogen for 15 min. The mixture was vacuum filtered through a glass frit Buchner funnel with a 20 mm layer of celite. The filter cake was displacement washed with ethanol (2 X 100 mL) and pulled until slightly ethyl alcohol wet, then wetted with water and the used Raney nickel catalyst was discarded under water. The clear pale amber filtrate was concentrated under reduced pressure to a clear viscous light amber oil. The oil was diluted with methyl tert-butyl ether (1500 mL) and the cloudy solution was concentrated under reduced pressure to a volume of about 150 mL which provided a suspension. The mixture was again diluted with methyl tert-butyl ether (1500 mL) and concentrated under reduced pressure to a volume of about 150 mL. The resulting suspension was allowed to stand at room temperature overnight (about 12 h). The solid was collected by vacuum filtration in a glass frit Buchner funnel and the filter cake was displacement washed with cold methyl tert-butyl ether (2 X 50 mL) and then pulled for 30 min. The material was further dried in a vacuum oven at 45 °C for 3 h to provide a white solid (19 g, 0.111 mol, 89% yield) as the product, 3-(3-hydroxypropyl)-5,5-dimethyl-pyrrolidin-2-one. ¹H NMR (400 MHz, dimethyl sulfoxide-d₆) δ 7.63 (s, 1H), 3.38 (t, J = 6.5 Hz, 2H), 2.37 (tdd, J = 9.8, 8.5, 4.4 Hz, 1H), 2.02 (dd, J = 12.3, 8.6 Hz, 1H), 1.72 (tdd, J = 9.6, 7.5, 4.4 Hz, 1H), 1.52 - 1.32 (m, 3H), 1.28 - 1.03 (m, 7H).

### Step 5: 3-(5,5-Dimethylpyrrolidin-3-yl)propan-1-ol

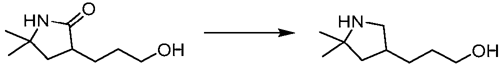

A 5 L, 3-neck round bottom flask was fitted with a mechanical stirrer, a heating mantle, an addition funnel, a J-Kem temperature probe/controller and a nitrogen inlet/outlet. The vessel was charged under a nitrogen atmosphere with lithium aluminum hydride pellets (19.39 g, 510.9 mmol). The vessel was then charged with tetrahydrofuran (500 mL, 20 mL/g). Stirring was commenced and the pot temperature was recorded at 20 °C. The mixture was allowed to stir at room temperature for 0.5 h to allow the pellets to dissolve. The pot temperature of the resulting grey suspension was recorded at 24 °C. The addition funnel was charged with a solution of 3-(3-hydroxypropyl)-5,5-dimethyl-pyrrolidin-2-one (25 g, 146.0 mmol) in tetrahydrofuran (500 mL) and the clear pale yellow solution was added dropwise over 90 min. Slight heating was required to achieve homogeneity. After the completed addition the pot temperature of the resulting greyish suspension was recorded at 24 °C. The mixture was then heated to a pot temperature of 65 °C and the condition was maintained for 72 h. Analysis of the reaction mixture at this point indicated some residual starting material still remaining and no change in product formation. The reaction was subsequently stopped at this point. The heating mantle was removed and the vessel was fitted with a cooling bath. The suspension was cooled to 0 °C with a crushed ice/water cooling bath and then quenched by the very slow dropwise addition of water (19.93 mL), followed by 15 wt% sodium hydroxide solution (19.93 mL) and then finally with water (59.79 mL). The pot temperature of the resulting white suspension was recorded at 5 °C. The cooling bath was removed and the vessel was again fitted with a heating mantle. The suspension was warmed to 60 °C and the condition was maintained for 30 min. The warm suspension was vacuum filtered through a glass frit Buchner funnel with a 20 mm layer of celite. The filter cake was then displacement washed with 60 °C tetrahydrofuran (2 X 250 mL) and then pulled for 30 min. The clear filtrate was concentrated under reduced pressure to provide (23.5 g, 0.149 mol, 99% yield) of a clear light yellow viscous oil as the desired product, 3-(5,5-dimethylpyrrolidin-3-yl)propan-1-ol. 1H NMR (400 MHz, dimethyl sulfoxide-d₆) δ 3.37 (dt, J = 8.3, 6.4 Hz, 3H), 2.95 (dd, J = 10.6, 7.6 Hz, 1H), 2.40 (dd, J = 10.7, 7.7 Hz, 1H), 2.04 (dt, J = 16.1, 8.1 Hz, 1H), 1.69 (dd, J = 12.2, 8.2 Hz, 1H), 1.50 - 1.24 (m, 5H), 1.11 - 0.94 (m, 7H).

### Step 6: tert-Butyl 4-(3-hydroxypropyl)-2,2-dimethyl-pyrrolidine-1-carboxylate

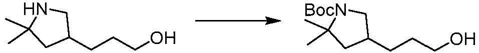

A 1 L, 3-neck round bottom flask was fitted with a mechanical stirrer, a cooling bath, an addition funnel, a J-Kem temperature probe and a nitrogen inlet/outlet. The vessel was charged under a nitrogen atmosphere with 3-(5,5-dimethylpyrrolidin-3-yl)propan-1-ol (15 g, 95.39 mmol) and dichloromethane (225 mL, 15 mL/g) which provided a clear light yellow solution. Stirring was commenced and the pot temperature was recorded at 19 °C. The cooling bath was charged with crushed ice/water and the pot temperature was lowered to 0 °C. The addition funnel was charged with triethylamine (12.55 g, 124.0 mmol) which was subsequently added neat dropwise over 5 min. No exotherm was observed. The addition funnel was then charged with di-tert-butyl dicarbonate (22.89 g, 104.9 mmol) dissolved in dichloromethane (225 mL). The clear pale yellow solution was then added dropwise over 30 min which resulted in gentle gas evolution. No exotherm was observed. The cooling bath was removed and the resulting clear light yellow solution was allowed to warm to room temperature and continue to stir at room temperature for 3 h. The reaction mixture was transferred to a separatory funnel and partitioned with water (75 mL). The organic was removed and washed with saturated sodium chloride solution (75 mL), dried over sodium sulfate (150 g) and then filtered through a glass frit Buchner funnel. The filtrate was concentrated under reduced pressure to provide (30 g) of a clear light yellow oil as the desired crude product. The material was purified by silica gel column flash chromatography (liquid load with dichloromethane) eluting with a gradient of 100% dichloromethane to 10% methyl alcohol in dichloromethane over 60 min collecting 50 mL fractions. The desired product fractions were combined and concentrated under reduced pressure to provide tert-butyl 4-(3-hydroxypropyl)-2,2-dimethyl-pyrrolidine-1-carboxylate (22 g, 0.0855 mol, 90% yield) as a clear pale yellow viscous oil. ¹H NMR (400 MHz, DMSO-d₆) δ 4.38 (td, J = 5.2, 1.4 Hz, 1H), 3.54 (dt, J = 10.3, 6.7 Hz, 1H), 3.38 (td, J = 6.6, 3.5 Hz, 2H), 2.76 (q, J = 10.3 Hz, 1H), 2.07 (td, J = 11.6, 5.7 Hz, 1H), 1.87 (ddd, J = 16.7, 12.1, 6.0 Hz, 1H), 1.37 (dd, J = 14.2, 10.4 Hz, 17H), 1.24 (s, 3H).

### Step 7: tert-Butyl 2,2-dimethyl-4-(3-methylsulfonyl oxypropyl)pyrrolidine-1-carboxylate

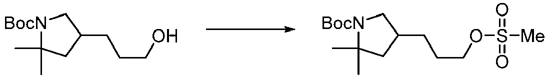

*tert-*Butyl 4-(3-hydroxypropyl)-2,2-dimethyl-pyrrolidine-1-carboxylate (50.5 g, 196.22 mmol) and triethylamine (39.711 g, 54.698 mL, 392.44 mmol) were dissolved in dichloromethane (500 mL) and the resulting solution was chilled in an ice water bath for 30 min. Mesyl chloride (24.725 g, 16.706 mL, 215.84 mmol) was added dropwise over a 30 min period, then the ice bath was removed and the mixture stirred at room temperature for one hour. The reaction was then quenched with saturated sodium bicarbonate solution (200 mL). The phases were separated and the organic phase was extracted with saturated sodium bicarbonate (200 mL) and water (2 X 100 mL). The aqueous phases were discarded and the organic phase was dried over sodium sulfate, filtered and concentrated *in vacuo* to obtain *tert*-butyl 2,2-dimethyl-4-(3-methylsulfonyl oxypropyl)pyrrolidine-1-carboxylate (64.2 g, 93%) as a pale yellow oil. ESI-MS m/z calc. 335.1766, found 336.4 (M+1)+; Retention time: 5.54 min (LC Method Q).

### Step 8: tert-Butyl 4-(3-aminopropyl)-2,2-dimethyl-pyrrolidine-1-carboxylate

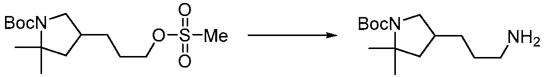

*tert*-Butyl 2,2-dimethyl-4-(3-methylsulfonyloxypropyl)pyrrolidine-1-carboxylate (64.2 g, 191.38 mmol) was dissolved in dioxane (650 mL) and then ammonium hydroxide (650 mL) was added and the resulting mixture heated to 45 °C for 18 h. After 18 h, the reaction was cooled to room temperature. The solution was diluted with 1M sodium hydroxide (200 mL) and then extracted with diethyl ether (3 X 650 mL). The aqueous phase was discarded and the combined organic phases were extracted with water (2 X 200 mL). The aqueous phases were discarded and the organic phase was dried over sodium sulfate, filtered and concentrated *in vacuo* to afford *tert*-butyl 4-(3-aminopropyl)-2,2-dimethyl-pyrrolidine-1-carboxylate (48.9 g, 95%) as a pale yellow oil. ESI-MS m/z calc. 256.2151, found 257.3 (M+1)+; Retention time: 3.70 min (LC Method Q).

### Step 9: tert-Butyl 2,2-dimethyl-4-[3-[(6-sulfamoyl-2-pyridyl)amino]propyl]pyrrolidine-1-carboxylate

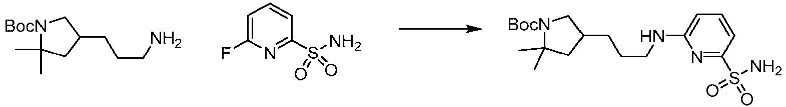

To *tert*-butyl 4-(3-aminopropyl)-2,2-dimethyl-pyrrolidine-1-carboxylate (8.91 g, 34.8 mmol) and 6-fluoropyridine-2-sulfonamide (6.13 g, 34.8 mmol) in dimethyl sulfoxide (75 mL) was added potassium carbonate (4.91 g, 35.5 mmol) and the mixture stirred at 100 °C for 12 h and then allowed to cool to ambient temperature and stirred for an additional 4 h (16 h total). The reaction mixture was slowly poured into hydrochloric acid (35 mL of 1 M, 35.00 mmol) in water (200 mL) (some foaming) and diluted with ethyl acetate (250 mL). The organic phase was separated and washed with 100 mL of brine. The organic phase was dried over magnesium sulfate, filtered over celite, and concentrated in vacuo to afford a dark yellow oil. The crude product was purified by silica gel chromatography eluting with 0% - 100% ethyl acetate in hexanes. Collected both pure (9.0 g) and impure (3 g) fractions. Purified the impure fractions by silica gel chromatography eluting with 0% - 100% ethyl acetate in hexanes affording, in total, *tert-*butyl 2,2-dimethyl-4-[3-[(6-sulfamoyl-2-pyridyl)amino]propyl]pyrrolidine-1-carboxylate (10.0 g, 69%). 1H NMR (400 MHz, dimethyl sulfoxide-d₆) δ 7.52 (dd, J = 8.5, 7.2 Hz, 1H), 7.07 (s, 2H), 6.95 (dd, J = 7.2, 0.7 Hz, 2H), 6.61 (d, J = 8.5 Hz, 1H), 3.55 (q, J = 9.1 Hz, 1H), 3.32 - 3.24 (m, 2H), 2.79 (q, J = 10.0 Hz, 1H), 2.13 (d, J = 16.1 Hz, 1H), 1.96 - 1.82 (m, 1H), 1.51 (dt, J = 18.0, 9.3 Hz, 2H), 1.37 (dd, J = 12.9, 10.6 Hz, 15H), 1.24 (s, 3H). ESI-MS m/z calc. 412.21442, found 413.1 (M+1)+; Retention time: 2.34 min (LC Method D).

### Step 10: tert-Butyl (4S)-2,2-dimethyl-4-[3-[(6-sulfamoyl-2-pyridyl)amino]propyl]pyrrolidine-1-carboxylate

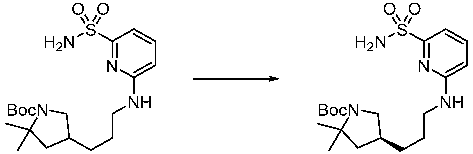

Subjected racemic *tert*-butyl 2,2-dimethyl-4-[3-[(6-sulfamoyl-2-pyridyl)amino]propyl]pyrrolidine-1-carboxylate (7 g, 16.97 mmol) to chiral separation by SFC chromatography using a ChiralPak IG (250 X 21.2 mm column, 5µm particle size) with 40% methanol/60% carbon dioxide mobile phase at 70 mL/min over 11.0 min (injection volume = 500 µL of 32 mg/mL solution in methanol) giving as the first peak to elute, *tert*-butyl (4*S*)-2,2-dimethyl-4-[3-[(6-sulfamoyl-2-pyridyl)amino]propyl]pyrrolidine-1-carboxylate (3.4481 g, 99%). ESI-MS m/z calc. 412.21442, found 413.2 (M+1)⁺; Retention time: 0.63 min (LC Method A).

### Part C: Synthesis of (14S)-8-[3-(2-{dispiro[2.0.2.1]heptan-7-yl}ethoxy)-1H-pyrazol-1-yl]-12,12-dimethyl-2λ6-thia-3,9,11,18,23-pentaazatetracyclo [17.3.1.111,14.05,10]tetracosa-1(22),5,7,9,19(23),20-hexaene-2,2,4-trione (Compound I)

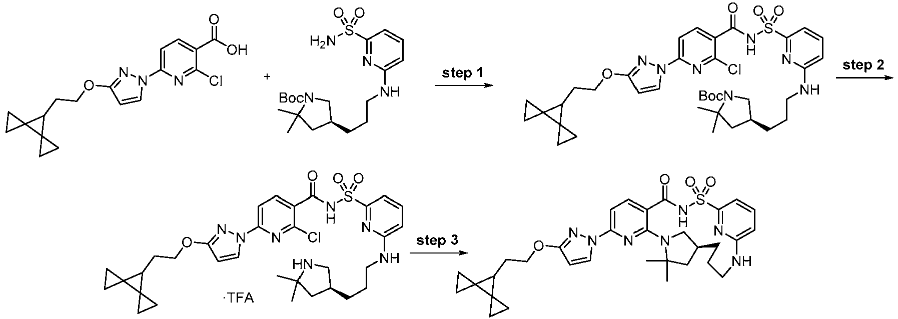

### Step 1: tert-Butyl (4S)-4-[3-[[6-[[2-chloro-6-[3-(2-dispiro[2.0.2.1]heptan-7-ylethoxy)pyrazol-1-yl]pyridine-3-carbonyl]sulfamoyl]-2-pyridyl]amino]propyl]-2,2-dimethyl-pyrrolidine-1-carboxylate

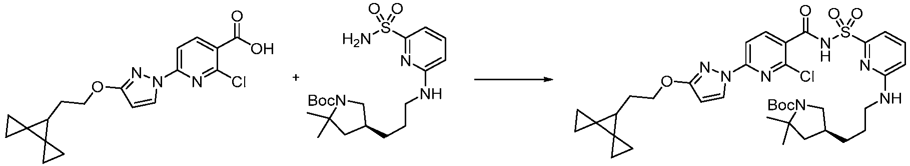

To a solution of 2-chloro-6-[3-(2-dispiro[2.0.2.1]heptan-7-ylethoxy)pyrazol-1-yl]pyridine-3-carboxylic acid (5.2 g, 14.45 mmol) in tetrahydrofuran (100 mL) was added carbonyl diimidazole (2.8 g, 16.51 mmol) and the mixture stirred at ambient temperature for 1 h. To this mixture was added *tert*-butyl (4S)-2,2-dimethyl-4-[3-[(6-sulfamoyl-2-pyridyl)amino]propyl]pyrrolidine-1-carboxylate (6.0 g, 14.54 mmol) in tetrahydrofuran (15 mL) followed by 1,8-diazabicyclo[5.4.0]undec-7-ene (6.5 mL, 43.47 mmol) and the mixture was stirred at ambient temperature for 16 h. The reaction was diluted with water (150 mL) and the mixture acidified with aqueous hydrochloric acid (15 mL of 6 M, 90.00 mmol). The mixture was extracted with ethyl acetate (300 mL) and the organic phase separated. The organic phase was washed with brine, dried over magnesium sulfate, filtered over Celite and concentrated *in vacuo* affording a white precipitate. The precipitate was slurried with acetonitrile and the solid collected by filtration using a medium glass frit and washed with acetonitrile. The filtrate was concentrated *in vacuo* affording a yellow oil. The crude oil was diluted with acetonitrile and some *N*-methyl-2-pyrrolidone and chromatographed on a 415 g reverse phase C₁₈ column eluting with 50% - 100% acetonitrile in water giving *tert*-butyl (4*S*)-4-[3-[[6-[[2-chloro-6-[3-(2-dispiro[2.0.2.1]heptan-7-ylethoxy)pyrazol-1-yl]pyridine-3-carbonyl]sulfamoyl]-2-pyridyl]amino]propyl]-2,2-dimethyl-pyrrolidine-1-carboxylate (4.5 g, 41%). ESI-MS m/z calc. 753.30756, found 754.4 (M+1)⁺; Retention time: 3.79 min (LC Method D).

### Step 2: 2-Chloro-N-[[6-[3-[(3S)-5,5-dimethylpyrrolidin-3-yl]propylamino]-2-pyridyl]sulfonyl]-6-[3-(2-dispiro[2.0.2.1]heptan-7-ylethoxy)pyrazol-1-yl]pyridine-3-carboxamide (trifluoroacetate salt)

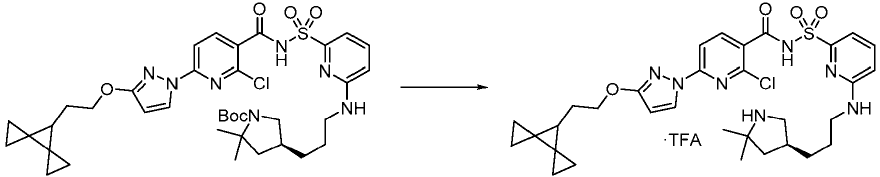

To a solution of *tert*-butyl (4*S*)-4-[3-[[6-[[2-chloro-6-[3-(2-dispiro[2.0.2.1]heptan-7-ylethoxy)pyrazol-1-yl]pyridine-3-carbonyl]sulfamoyl]-2-pyridyl]amino]propyl]-2,2-dimethyl-pyrrolidine-1-carboxylate (5.9 g, 7.821 mmol) in dichloromethane (30 mL) and toluene (15 mL) was added trifluoroacetic acid (6.0 mL, 77.88 mmol) and the mixture stirred at ambient temperature for 18 h. The solvent was removed *in vacuo* with the bath temp set at 45 °C affording a thick, yellow oil. The oil was diluted with toluene (125 mL) and the solvent removed *in vacuo* with the bath temp set at 45 °C. The oil was diluted with toluene and the solvent removed *in vacuo* affording a thick, viscous yellow oil, 2-chloro-*N*-[[6-[3-[(3*S*)-5,5-dimethylpyrrolidin-3-yl]propylamino]-2-pyridyl]sulfonyl]-6-[3-(2-dispiro[2.0.2.1]heptan-7-ylethoxy)pyrazol-1-yl]pyridine-3-carboxamide (trifluoroacetate salt) (6.0 g, 100%) which was used in the next step without further purification. ESI-MS m/z calc. 653.2551, found 654.3 (M+1)⁺; Retention time: 2.6 min (LC Method B).

### Step 3: (14S)-8-[3-(2-{Dispiro[2.0.2.1]heptan-7-yl}ethoxy)-1H-pyrazol-1-yl]-12,12-dimethyl-2λ6-thia-3,9,11,18,23-pentaazatetracyclo[17.3.1.111,14.05,10]tetracosa-1(22),5,7,9,19(23),20-hexaene-2,2,4-trione (Compound I)

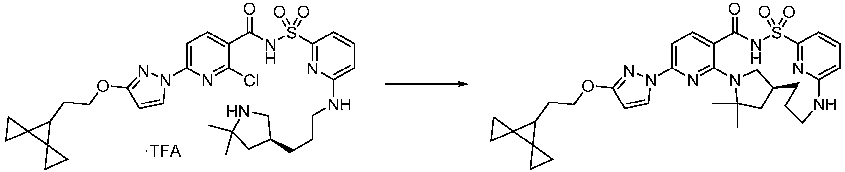

To a solution of 2-chloro-*N*-[[6-[3-[(3*S*)-5,5-dimethylpyrrolidin-3-yl]propylamino]-2-pyridyl]sulfonyl]-6-[3-(2-dispiro[2.0.2.1]heptan-7-ylethoxy)pyrazol-1-yl]pyridine-3-carboxamide (trifluoroacetate salt) (6.0 g, 7.810 mmol) in NMP (140 mL) was added potassium carbonate (5.3 g, 38.35 mmol). The mixture was purged with nitrogen for 5 min. The mixture was then heated at 150 °C for 22 h. The reaction mixture was cooled to room temperature and added to water (300 mL) affording an off-white solid precipitate. The mixture was carefully acidified with aqueous hydrochloric acid (12 mL of 6 M, 72.00 mmol) affording a foamy slurry. The solid was collected by filtration using a medium glass frit. The wet filter cake was dissolved in ethyl acetate (500 mL) and washed with 200 mL of brine. The aqueous phase was slightly cloudy so it was acidified with a small amount of 6N hydrochloric acid and returned to the organic phase. The aqueous phase was separated and the organic phase was dried over magnesium sulfate, filtered and concentrated *in vacuo* affording a light yellow oil. This crude product was diluted with acetonitrile and chromatographed on a 415 g C₁₈ reverse phase column eluting with 50% - 100% acetonitrile in water. The product was isolated as a cream colored foam. The foam was dried *in vacuo* at 45 °C for 48 h giving (14*S*)-8-[3-(2-{dispiro[2.0.2.1]heptan-7-yl}ethoxy)-1H-pyrazol-1-yl]-12,12-dimethyl-2λ6-thia-3,9,11,18,23-pentaazatetracyclo[17.3.1.111,14.05,10]tetracosa-1(22),5,7,9,19(23),20-hexaene-2,2,4-trione (Compound **I)** (3.32 g, 68%). ¹H NMR (400 MHz, dimethyl sulfoxide-d₆) δ 12.48 (s, 1H), 8.20 (d, J = 2.8 Hz, 1H), 7.81 (d, J = 8.2 Hz, 1H), 7.57 (dd, J = 8.5, 7.2 Hz, 1H), 7.05 (d, J = 7.1 Hz, 1H), 6.97 (d, J = 8.5 Hz, 1H), 6.91 (d, J = 8.2 Hz, 1H), 6.71 (d, J = 8.5 Hz, 1H), 6.08 (d, J = 2.7 Hz, 1H), 4.21 (td, J = 6.7, 1.3 Hz, 2H), 3.92 (d, J = 12.0 Hz, 1H), 3.16 (s, 1H), 2.95 (d, J = 13.3 Hz, 1H), 2.78 - 2.66 (m, 1H), 2.07 (s, 1H), 1.92 - 1.72 (m, 4H), 1.60 (s, 6H), 1.51 (s, 3H), 1.47 (t, J = 6.5 Hz, 1H), 1.31 (q, J = 12.2 Hz, 1H), 0.89 - 0.77 (m, 4H), 0.69 - 0.61 (m, 2H), 0.53 - 0.45 (m, 2H). ESI-MS m/z calc. 617.27844, found 618.4 (M+1)⁺; Retention time: 10.29 min (LC Method F).

Ca²⁺, Na⁺, and K⁺ salts of Compound **I** were made by mixing Compound **I** with Ca(OCH₃)₂, Na(OCH₃), and KOH, respectively: mixing Compound **I** (1g) and Ca(OCH₃)₂ (83 mg) in methanol (65 mL) at room temperature for 30 minutes and then at 65 °C for 30 minutes; mixing Compound **I** (0.6 g (1 mMol)) in MeOH (40 mL) with 25 wt% Na(OCH₃) in MeOH (250 mL (1 molar equiv)) at 60 °C for 20 minutes; and mixing Compound **I** (0.6 g) in acetone (11 mL) with 1N KOH (1 molar equivalent) at 50 °C for 1 hour. After filtration of the resulting hot solutions, the filtrates were evaporated to dryness to yield the desired amorphous salts, respectively (PXRD data not shown).

### Example 2: Synthesis of (R)-1-(2,2-Difluorobenzo[d][1,3]dioxol-5-yl)-N-(1-(2,3-dihydroxypropyl)-6-fluoro-2-(1-hydroxy-2-methylpropan-2-yl)-1H-indol-5-yl)cyclopropanecarboxamide (Compound II)

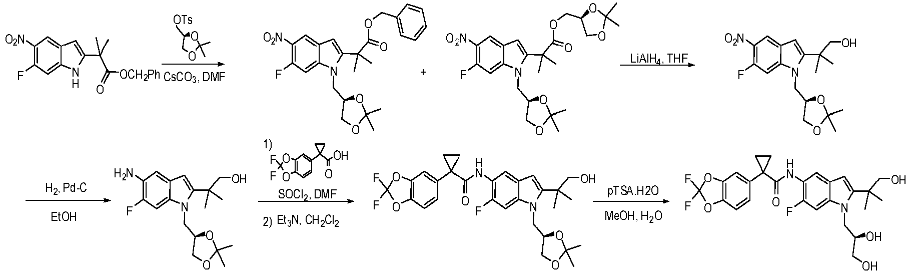

### Step 1: (R)-Benzyl 2-(1-((2,2-dimethyl-1,3-dioxolan-4-yl)methyl)-6-fluoro-5-nitro-1H-indol-2-yl)-2-methylpropanoate and ((S)-2,2-Dimethyl-1,3-dioxolan-4-yl)methyl 2-(1-(((R)-2,2-dimethyl-1,3-dioxolan-4-yl)methyl)-6-fluoro-5-nitro-1H-indol-2-yl)-2-methylpropanoate

Cesium carbonate (8.23 g, 25.3 mmol) was added to a mixture of benzyl 2-(6-fluoro-5-nitro-1H-indol-2-yl)-2-methylpropanoate (3.0 g, 8.4 mmol) and (S)-(2,2-dimethyl-1,3-dioxolan-4-yl)methyl 4-methylbenzenesulfonate (7.23 g, 25.3 mmol) in DMF (17 mL). The reaction was stirred at 80 °C for 46 hours under a nitrogen atmosphere. The mixture was then partitioned between ethyl acetate and water. The aqueous layer was extracted with ethyl acetate. The combined ethyl acetate layers were washed with brine, dried over MgSO₄, filtered and concentrated. The crude product, a viscous brown oil which contains both of the products shown above, was taken directly to the next step without further purification. (R)-Benzyl 2-(1-((2,2-dimethyl-1,3-dioxolan-4-yl)methyl)-6-fluoro-5-nitro-1H-indol-2-yl)-2-methylpropanoate, ESI-MS *m*/*z* calc. 470.2, found 471.5 (M+1)⁺. Retention time 2.20 minutes. ((S)-2,2-Dimethyl-1,3-dioxolan-4-yl)methyl 2-(1-(((R)-2,2-dimethyl-1,3-dioxolan-4-yl)methyl)-6-fluoro-5-nitro-1H-indol-2-yl)-2-methylpropanoate, ESI-MS *m*/*z* calc. 494.5, found 495.7 (M+1)⁺. Retention time 2.01 minutes.

### Step 2: (R)-2-(1-((2,2-dimethyl-1,3-dioxolan-4-yl)methyl)-6-fluoro-5-nitro-1H-indol-2-yl)-2-methylpropan-1-ol

The crude reaction mixture obtained in step (A) was dissolved in THF (42 mL) and cooled in an ice-water bath. LiAlH₄ (16.8 mL of 1 M solution, 16.8 mmol) was added drop-wise. After the addition was complete, the mixture was stirred for an additional 5 minutes. The reaction was quenched by adding water (1 mL), 15% NaOH solution (1 mL) and then water (3 mL). The mixture was filtered over Celite, and the solids were washed with THF and ethyl acetate. The filtrate was concentrated and purified by column chromatography (30-60% ethyl acetate- hexanes) to obtain (R)-2-(1-((2,2-dimethyl-1,3-dioxolan-4-yl)methyl)-6-fluoro-5-nitro-1H-indol-2-yl)-2-methylpropan-1-ol as a brown oil (2.68g, 87 % over 2 steps). ESI-MS *m*/*z* calc. 366.4, found 367.3 (M+1)⁺. Retention time 1.68 minutes. ¹H NMR (400 MHz, DMSO-d6) δ 8.34 (d, J = 7.6 Hz, 1H), 7.65 (d, J = 13.4 Hz, 1H), 6.57 (s, 1H), 4.94 (t, J = 5.4 Hz, 1H), 4.64 - 4.60 (m, 1H), 4.52 - 4.42(m, 2H), 4.16 - 4.14 (m, 1H), 3.76 - 3.74 (m, 1H), 3.63 - 3.53 (m, 2H), 1.42 (s, 3H), 1.38 - 1.36 (m, 6H) and 1.19 (s, 3H) ppm

### Step 3: (R)-2-(5-amino-1-((2,2-dimethyl-1,3-dioxolan-4-yl)methyl)-6-fluoro-1H-indol-2-yl)-2-methylpropan-1-ol

(R)-2-(1-((2,2-dimethyl-1,3-dioxolan-4-yl)methyl)-6-fluoro-5-nitro-1H-indol-2-yl)-2-methylpropan-1-ol (2.5 g, 6.82 mmol) was dissolved in ethanol (70 mL) and the reaction was flushed with N₂. Then Pd-C (250 mg, 5% wt) was added. The reaction was flushed with nitrogen again and then stirred under H₂ (atm). After 2.5 hours only partial conversion to the product was observed by LCMS. The reaction was filtered through Celite and concentrated. The residue was re-subjected to the conditions above. After 2 hours LCMS indicated complete conversion to product. The reaction mixture was filtered through Celite. The filtrate was concentrated to yield the product as a black solid (1.82 g, 79 %). ESI-MS *m*/*z* calc. 336.2, found 337.5 (M+1)⁺. Retention time 0.86 minutes. ¹H NMR (400 MHz, DMSO-d6) δ 7.17 (d, J = 12.6 Hz, 1H), 6.76 (d, J = 9.0 Hz, 1H), 6.03 (s, 1H), 4.79 - 4.76 (m, 1H), 4.46 (s, 2H), 4.37 - 4.31 (m, 3H),4.06 (dd, J = 6.1, 8.3 Hz, 1H), 3.70 - 3.67 (m, 1H), 3.55 - 3.52 (m, 2H), 1.41 (s, 3H), 1.32 (s, 6H) and 1.21 (s, 3H) ppm.

### Step 4: (R)-1-(2,2-difluorobenzo[d][1,3]dioxol-5-yl)-N-(1-((2,2-dimethyl-1,3-dioxolan-4-yl)methyl)-6-fluoro-2-(1-hydroxy-2-methylpropan-2-yl)-1H-indol-5-yl)cyclopropanecarboxamide

DMF (3 drops) was added to a stirring mixture of 1-(2,2-difluorobenzo[d][1,3]dioxol-5-yl)cyclopropanecarboxylic acid (1.87 g, 7.7 mmol) and thionyl chloride (1.30 mL, 17.9 mmol). After 1 hour a clear solution had formed. The solution was concentrated under vacuum and then toluene (3 mL) was added and the mixture was concentrated again. The toluene step was repeated once more and the residue was placed on high vacuum for 10 minutes. The acid chloride was then dissolved in dichloromethane (10 mL) and added to a mixture of (R)-2-(5-amino-1-((2,2-dimethyl-1,3-dioxolan-4-yl)methyl)-6-fluoro-1H-indol-2-yl)-2-methylpropan-1-ol (1.8 g, 5.4 mmol) and triethylamine (2.24 mL, 16.1 mmol) in dichloromethane (45 mL). The reaction was stirred at room temperature for 1 hour. The reaction was washed with 1N HCl solution, saturated NaHCO₃ solution and brine, dried over MgSO₄ and concentrated to yield the product as a black foamy solid (3g, 100%). ESI-MS *m*/*z* calc. 560.6, found 561.7 (M+1)⁺. Retention time 2.05 minutes. ¹H NMR (400 MHz, DMSO-*d*6) δ 8.31 (s, 1H), 7.53 (s, 1H), 7.42 - 7.40 (m, 2H), 7.34 - 7.30 (m, 3H), 6.24 (s, 1H), 4.51 - 4.48 (m, 1H), 4.39 - 4.34 (m,2H), 4.08 (dd, J = 6.0, 8.3 Hz, 1H), 3.69 (t, J = 7.6 Hz, 1H), 3.58 - 3.51 (m, 2H), 1.48 - 1.45 (m, 2H), 1.39 (s, 3H), 1.34 - 1.33 (m, 6H), 1.18 (s, 3H) and 1.14 - 1.12 (m, 2H) ppm

### Step 5: (R)-1-(2,2-difluorobenzo[d][1,3]dioxol-5-yl)-N-(1-(2,3-dihydroxypropyl)-6-fluoro-2-(1-hydroxy-2-methylpropan-2-yl)-1H-indol-5-yl)cyclopropanecarboxamide

(R)-1-(2,2-difluorobenzo[d][1,3]dioxol-5-yl)-N-(1-((2,2-dimethyl-1,3-dioxolan-4-yl)methyl)-6-fluoro-2-(1-hydroxy-2-methylpropan-2-yl)-1H-indol-5-yl)cyclopropanecarboxamide (3.0 g, 5.4 mmol) was dissolved in methanol (52 mL). Water (5.2 mL) was added followed by p-TsOH.H₂O (204 mg, 1.1 mmol). The reaction was heated at 80 °C for 45 minutes. The solution was concentrated and then partitioned between ethyl acetate and saturated NaHCO₃ solution. The ethyl acetate layer was dried over MgSO₄ and concentrated. The residue was purified by column chromatography (50-100 % ethyl acetate - hexanes) to yield the product as a cream colored foamy solid. (1.3 g, 47 %, ee >98% by SFC). ESI-MS *m*/*z* calc. 520.5, found 521.7 (M+1)⁺. Retention time 1.69 minutes. ¹H NMR (400 MHz, DMSO-d6) δ 8.31 (s, 1H), 7.53 (s, 1H), 7.42 - 7.38 (m, 2H), 7.33 - 7.30 (m, 2H), 6.22 (s, 1H), 5.01 (d, J = 5.2 Hz, 1H), 4.90 (t, J = 5.5 Hz, 1H), 4.75 (t, J = 5.8 Hz, 1H), 4.40 (dd, J = 2.6, 15.1 Hz, 1H), 4.10 (dd, J = 8.7, 15.1 Hz, 1H), 3.90 (s, 1H), 3.65 - 3.54 (m, 2H), 3.48 - 3.33 (m, 2H), 1.48 - 1.45 (m, 2H), 1.35 (s, 3H), 1.32 (s, 3H) and 1.14 - 1.11 (m, 2H) ppm.

### Example 3: Synthesis of N-(2,4-di-tert-butyl-5-hydroxyphenyl)-4-oxo-1,4-dihydroquinoline-3-carboxamide (Compound III)

### Part A: Synthesis of 4-oxo-1,4-dihydroquinoline-3-carboxylic acid

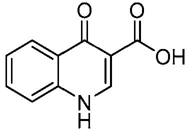

### Step 1: 2-Phenylaminomethylene-malonic acid diethyl ester

A mixture of aniline (25.6 g, 0.275 mol) and diethyl 2-(ethoxymethylene)malonate (62.4 g, 0.288 mol) was heated at 140-150 °C for 2 h. The mixture was cooled to room temperature and dried under reduced pressure to afford 2-phenylaminomethylene-malonic acid diethyl ester as a solid, which was used in the next step without further purification. ¹H NMR (DMSO-*d₆*) δ 11.00 (d, 1H), 8.54 (d, *J* = 13.6 Hz, 1H), 7.36-7.39 (m, 2H), 7.13-7.17 (m, 3H), 4.17-4.33 (m, 4H), 1.18-1.40 (m, 6H).

### Step 2: 4-Hydroxyquinoline-3-carboxylic acid ethyl ester

A 1 L three-necked flask fitted with a mechanical stirrer was charged with 2-phenylaminomethylene-malonic acid diethyl ester (26.3 g, 0.100 mol), polyphosphoric acid (270 g) and phosphoryl chloride (750 g). The mixture was heated to 70 °C and stirred for 4 h. The mixture was cooled to room temperature and filtered. The residue was treated with aqueous Na₂CO₃ solution, filtered, washed with water and dried. 4-Hydroxyquinoline-3-carboxylic acid ethyl ester was obtained as a pale brown solid (15.2 g, 70%). The crude product was used in next step without further purification.

### Step 3: 4-Oxo-1,4-dihydroquinoline-3-carboxylic acid

4-Hydroxyquinoline-3-carboxylic acid ethyl ester (15 g, 69 mmol) was suspended in sodium hydroxide solution (2N, 150 mL) and stirred for 2 h at reflux. After cooling, the mixture was filtered, and the filtrate was acidified to pH 4 with 2N HCl. The resulting precipitate was collected via filtration, washed with water and dried under vacuum to give 4-oxo-1,4-dihydroquinoline-3-carboxylic acid as a pale white solid (10.5 g, 92 %). ¹H NMR (DMSO-*d₆*) δ 15.34 (s, 1 H), 13.42 (s, 1 H), 8.89 (s, 1H), 8.28 (d, *J* = 8.0 Hz, 1H), 7.88 (m, 1H), 7.81 (d, J = 8.4 Hz, 1H), 7.60 (m, 1H).

### Part B: Synthesis of N-(2,4-di-tert-butyl-5-hydroxyphenyl)-4-oxo-1,4-dihydro quinoline-3-carboxamide

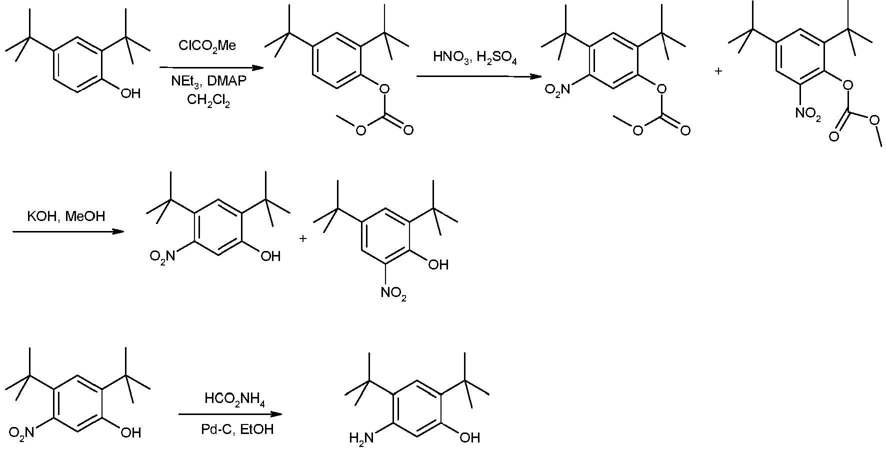

### Step 1: Carbonic acid 2,4-di-tert-butyl-phenyl ester methyl ester

Methyl chloroformate (58 mL, 750 mmol) was added dropwise to a solution of 2,4-di-*tert*-butyl-phenol (103.2 g, 500 mmol), Et₃N (139 mL, 1000 mmol) and DMAP (3.05 g, 25 mmol) in dichloromethane (400 mL) cooled in an ice-water bath to 0 °C. The mixture was allowed to warm to room temperature while stirring overnight, then filtered through silica gel (approx. 1L) using 10% ethyl acetate - hexanes (~ 4 L) as the eluent. The combined filtrates were concentrated to yield carbonic acid 2,4-di-*tert*-butyl-phenyl ester methyl ester as a yellow oil (132 g, quant.). ¹H NMR (400 MHz, DMSO-*d*₆) δ 7.35 (d, J = 2.4 Hz, 1H), 7.29 (dd, J = 8.5, 2.4 Hz, 1H), 7.06 (d, J = 8.4 Hz, 1H), 3.85 (s, 3H), 1.30 (s, 9H), 1.29 (s, 9H).

### Step 2: Carbonic acid 2,4-di-tert-butyl-5-nitro-phenyl ester methyl ester and Carbonic acid 2,4-di-tert-butyl-6-nitro-phenyl ester methyl ester

To a stirring mixture of carbonic acid 2,4-di-*tert*-butyl-phenyl ester methyl ester (4.76 g, 180 mmol) in conc. sulfuric acid (2 mL), cooled in an ice-water bath, was added a cooled mixture of sulfuric acid (2 mL) and nitric acid (2 mL). The addition was done slowly so that the reaction temperature did not exceed 50 °C. The reaction was allowed to stir for 2 h while warming to room temperature. The reaction mixture was then added to ice-water and extracted into diethyl ether. The ether layer was dried (MgSO₄), concentrated and purified by column chromatography (0 - 10% ethyl acetate - hexanes) to yield a mixture of carbonic acid 2,4-di-*tert*-butyl-5-nitro-phenyl ester methyl ester and carbonic acid 2,4-di-*tert*-butyl-6-nitro-phenyl ester methyl ester as a pale yellow solid (4.28 g), which was used directly in the next step.

### Step 3: 2,4-Di-tert-butyl-5-nitro-phenol and 2,4-Di-tert-butyl-6-nitro-phenol

The mixture of carbonic acid 2,4-di-*tert*-butyl-5-nitro-phenyl ester methyl ester and carbonic acid 2,4-di-*tert*-butyl-6-nitro-phenyl ester methyl ester (4.2 g, 14.0 mmol) was dissolved in MeOH (65 mL) before KOH (2.0 g, 36 mmol) was added. The mixture was stirred at room temperature for 2 h. The reaction mixture was then made acidic (pH 2-3) by adding conc. HCl and partitioned between water and diethyl ether. The ether layer was dried (MgSO₄), concentrated and purified by column chromatography (0 - 5 % ethyl acetate - hexanes) to provide 2,4-di-tert-butyl-5-nitro-phenol (1.31 g, 29% over 2 steps) and 2,4-di-*tert*-butyl-6-nitro-phenol. 2,4-Di-*tert*-butyl-5-nitro-phenol: ¹H NMR (400 MHz, DMSO-*d*₆) δ 10.14 (s, 1H, OH), 7.34 (s, 1H), 6.83 (s, 1H), 1.36 (s, 9H), 1.30 (s, 9H). 2,4-Di-*tert*-butyl-6-nitro-phenol: ¹H NMR (400 MHz, CDCl₃) δ 11.48 (s, 1H), 7.98 (d, J = 2.5 Hz, 1H), 7.66 (d, J = 2.4 Hz, 1H), 1.47 (s, 9H), 1.34 (s, 9H).

### Step 4: 5-Amino-2,4-di-tert-butyl-phenol

To a reluxing solution of 2,4-di-*tert*-butyl-5-nitro-phenol (1.86 g, 7.40 mmol) and ammonium formate (1.86 g) in ethanol (75 mL) was added Pd-5% wt. on activated carbon (900 mg). The reaction mixture was stirred at reflux for 2 h, cooled to room temperature and filtered through Celite. The Celite was washed with methanol and the combined filtrates were concentrated to yield 5-amino-2,4-di-*tert*-butyl-phenol as a grey solid (1.66 g, quant.). ¹H NMR (400 MHz, DMSO-*d*₆) δ 8.64 (s, 1H, OH), 6.84 (s, 1H), 6.08 (s, 1H), 4.39 (s, 2H, NH₂), 1.27 (m, 18H); HPLC ret. time 2.72 min, 10-99 % CH₃CN, 5 min run; ESI-MS 222.4 m/z [M+H]⁺.

### Step 5: N-(5-hydroxy-2,4-di-tert-butyl-phenyl)-4-oxo-1H-quinoline-3-carboxamide

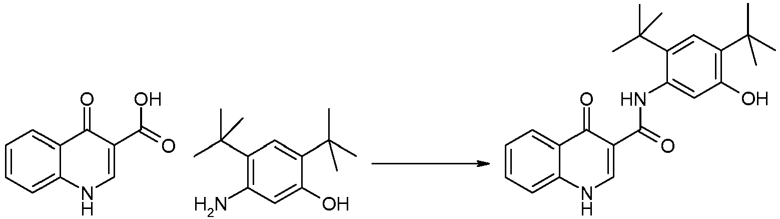

To a suspension of 4-oxo-1,4-dihydroquinolin-3-carboxylic acid (35.5 g, 188 mmol) and HBTU (85.7 g, 226 mmol) in DMF (280 mL) was added Et₃N (63.0 mL, 451 mmol) at ambient temperature. The mixture became homogeneous and was allowed to stir for 10 min before 5-amino-2,4-di-*tert*-butyl-phenol (50.0 g, 226 mmol) was added in small portions. The mixture was allowed to stir overnight at ambient temperature. The mixture became heterogeneous over the course of the reaction. After all of the acid was consumed (LC-MS analysis, MH+ 190, 1.71 min), the solvent was removed *in vacuo.* EtOH was added to the orange solid material to produce a slurry. The mixture was stirred on a rotovap (bath temperature 65 °C) for 15 min without placing the system under vacuum. The mixture was filtered and the captured solid was washed with hexanes to provide a white solid that was the EtOH crystalate. Et₂O was added to the solid obtained above until a slurry was formed. The mixture was stirred on a rotovapor (bath temperature 25 °C) for 15 min without placing the system under vacuum. The mixture was filtered and the solid captured. This procedure was performed a total of five times. The solid obtained after the fifth precipitation was placed under vacuum overnight to provide N-(5-hydroxy-2,4-di-*tert*-butyl-phenyl)-4-oxo-1H-quinoline-3-carboxamide as a white powdery solid (38 g, 52%). HPLC ret. time 3.45 min, 10-99% CH₃CN, 5 min run; ¹H NMR (400 MHz, DMSO-*d*₆) δ 12.88 (s, 1H), 11.83 (s, 1H), 9.20 (s, 1H), 8.87 (s, 1H), 8.33 (dd, J = 8.2, 1.0 Hz, 1H), 7.83-7.79 (m, 1H), 7.76 (d, J = 7.7 Hz, 1H), 7.54-7.50 (m, 1H), 7.17 (s, 1H), 7.10 (s, 1H), 1.38 (s, 9H), 1.37 (s, 9H); ESI-MS m/z calc'd 392.21; found 393.3 [M+H]⁺.

### Example 4: Synthesis of N -(2-( tert-Buty1 )-4-( tert-butyl -d,)-5- hydroxyphenyl )-4-oxo-1 ,4-dihydroquinoline-3 -carboxamide (Compound III-d)

### Step 1. 2-(tert-Butyl-d₉)-4-(tert-butyl)-6-d-phenol

To a solution of 4-tert-butyl phenol (3.43 g, 22.7 mmol) and tert-butyl alcohol-d10 (3.00 mL, 31.8 mmol, 98 atom% D, Cambridge Isotope Laboratories, Inc.) in dichloromethane (40.0 mL) was added D₂SO₄ (1.50 mL, 99.5 atom% D, Sigma-Aldrich). The reaction was stirred at room temperature for 15 hours then was diluted with water and extracted with dichloromethane (3x100 mL). The organic layers were combined, washed with saturated NaHCO₃, dried (Na₂SO₄), filtered, and concentrated in vacuo. The resulting oil was purified by column chromatography (SiO₂, 0-15% ethyl acetate/heptanes) to afford 2-(tert-Butyl-d₉)-4-(tert-butyl)-6-d-phenol (4.04 g, 83% yield) as a clear oil. ¹HNMR (d₆-DMSO, 400MHz) δ 9.04 (s, 1H), 7.12 (d, *J=2.4* Hz, 1H), 6.98 (dd, J=3.8, 2.5 Hz, 1H), 6.67 (d, J=8.3 Hz, 0.3H), 1.22 (s, 10H).

### Step 2: 2-(tert-Butyl-d₉)-4-(tert-butyl)-6-d-phenyl methyl carbonate

To a solution of 2-(tert-Butyl-d₉)-4-(tert-butyl)-6-d-phenol (4.04 g, 18.8 mmol), triethylamine (5.24 mL, 37.6 mmol) and N,N-dimethylaminopyridine (115 mg, 0.940 mmol) in CH₂Cl₂ (40.0 mL) at 0 °C was added methyl chloroformate (2.17 mL, 28.2 mmol). The reaction was stirred at room temperature for 15 hours and additional trimethylamine (1.30 mL, 9.33 mmol) and methyl chloroformate (0.550 mL, 7.15 mmol) were added. After stirring for an additional 1 hour the reaction was diluted with 10% ethyl acetate/heptanes and filtered through a silica plug. The silica plug was then rinsed with additional 10% ethyl acetate/heptanes. The filtrate was combined and concentrated in vacuo to provide 2-(tert-Butyl-d₉)-4-(tert-butyl)-6-d-phenyl methyl carbonate (4.69 g, 91% yield) as a light yellow oil which was carried forward without purification. ¹H NMR (d₆-DMSO, 400 MHz) δ 7.33 (d, *J=2.4* Hz, 1H), 7.30-7.20 (m, 1H), 7.06 (d, J=8.5 Hz, 0.3H), 3.84 (d, *J=0.7* Hz, 3H), 1.28 (s, 9H).

### Step 3: 2-(tert-Butyl-d₉)-4-(tert-butyl)-6-d-5-nitro-phenol

To a solution of 2-(tert-Butyl-d₉)-4-(tert-butyl)-6-d-phenyl methyl carbonate (4.69 g, 17.2 mmol) in sulfuric acid (2.00 mL) at 0 °C was added a 1:1 mixture of sulfuric acid and nitric acid (4.00 mL) dropwise. The reaction was then stirred at room temperature for two hours then slowly added to ice water with vigorous stirring. The resulting slurry was extracted with ethyl acetate (3x100 mL) and the combined organic layers were dried (Na₂SO₄), filtered, and concentrated to afford an amber oil containing a mixture of regio-isomers. This crude oil was then taken up in MeOH (100 mL) and KOH (3.50 g) was added. The reaction was stirred at room temperature for 2 hours then was acidified to pH=2 with concentrated HCl. The resulting solution was extracted with diethyl ether (3x100 mL), dried (MgSO₄), filtered, and concentrated. The residue was then purified via column chromatography (SiO₂, 0-5% ethyl acetate/heptanes) to afford 2-(tert-Butyl-d₉)-4-(tert-butyl)-6-d-5-nitro-phenol (1.33 g, 30%) as a light yellow solid. MS (ESI) 260.2 [(M-H)⁻].

### Step 4: 5-Amino-2-(tert-butyl-d₉)-4-(tert-butyl)-6-d-phenol

A solution of 2-(tert-Butyl-d₉)-4-(tert-butyl)-6-d-5-nitro-phenol (1.33 g, 5.11 mmol) and ammonium formate (1.29 g, 20.4 mmol) in ethanol (60.0 mL) was heated to reflux. At this time, 10% Pd/C (650 mg, 50% wet) was added in small portions and the reaction continued to stir at reflux for two hours. The reaction was then cooled to room temperature, diluted with THF, filtered through Celite^{®} and concentrated in vacuo to afford 5-Amino-2-(tert-butyl-d9)-4-(tert-butyl)-6-d-phenol (1.19 g, 100%) as a pink solid. MS (ESI) 232.3 [(M+H)⁺].

### Step 5: 5-Amino-2-(tert-butyl-d₉)-4-(tert-butyl)-phenol

5-Amino-2-(tert-butyl-d₉)-4-(tert-butyl)-6-dphenol (298 mg, 1.29 mmol) was dissolved in 5M HCl in 2-propanol (20 mL) and the reaction was stirred at room temperature for 15 hours. The reaction was then concentrated in vacuo and taken back up in 5M HCl in 2-propanol (20 mL). After stirring for an additional 15 hours at room temperature, the reaction was concentrated in vacuo and diluted with saturated aqueous sodium bicarbonate (100 mL ). The resulting aqueous solution was extracted with dichloromethane (3x50 mL). The organic layers were combined, dried (Na₂SO₄), filtered and concentrated in vacuo to afford 5-Amino-2-(tert-butyl-d₉)-4-(tert-butyl)-phenol (240 mg, 81%) as a pink solid. ¹H NMR (d₆-DMSO, 400 MHz) δ 8.62 (s, 1H), 6.83 (s, 1H), 6.08 (s, 1H), 1.27 (s, 9H).

### Step 6: N-(2-(tert-Butyl)-4-(tert-butyl-d₉)-5-hydroxyphenyl)-4-oxo-1,4-dihydroquinoline-3-carboxamide (Compound III-d)

To a solution of 5-Amino-2-(tert-butyl-d₉)-4-(tert-butyl)-phenol (240 mg, 1.04 mmol), 4-oxo-1,4-dihydroquinoline-3-carboxylic acid (purchased from Matrix Scientific, 99 mg, 0.521 mmol) and N,N-diisopropylethylamine (181 µl, 1.04 mmol) in DMF (6.00 mL) was added HATU (198 mg, 0.521 mmol). The reaction was stirred at room temperature for three hours then was diluted with saturated NaHCO₃ and extracted with ethyl acetate (3x50 mL). The combined organic extracts were washed with water (3x20 mL), dried (Na₂SO₄ ), filtered, and concentrated in vacuo. The resulting residue was purified via column chromatography (SiO₂, 0-70% ethyl acetate/heptanes) to afford N-(2-(tert-Butyl)-4-(tert-butyl-d₉)-5-hydroxyphenyl)-4-oxo-1,4-dihydroquinoline-3-carboxamide (Compound **III-d)** (80 mg, 38% Yield) as a white solid. ¹H NMR (d₆-DMSO, 400 MHz) δ 12.88 (s, 1H), 11.81 (s, 1H), 9.19 (s, 1H), 8.86 (s, 1H), 8.32 (dd, J=8.1, 1.4 Hz, 1H), 7.86-7.77 (m, 1H), 7.75 (d, J=8.2 Hz, 1H), 7.51 (s, 1H), 7.15 (s, 1H), 7.09 (s, 1H) 1.37 (s, 9H); MS (ESI) 402.3 [(M+H)⁺].

### Example 5: Synthesis of 3-(6-(1-(2,2-difluorobenzo[d][1,3]dioxol-5-yl)cyclopropanecarboxamido)-3-methylpyridin-2-yl)benzoic acid (Compound IV)

Vitride^{®} (sodium bis(2-methoxyethoxy)aluminum hydride [or NaAlH₂(OCH₂CH₂OCH₃)₂], 65 wgt% solution in toluene) was purchased from Aldrich Chemicals. 2,2-Difluoro-1,3-benzodioxole-5-carboxylic acid was purchased from Saltigo (an affiliate of the Lanxess Corporation).

### Step 1: (2,2-Difluoro-1,3-benzodioxol-5-yl)-methanol

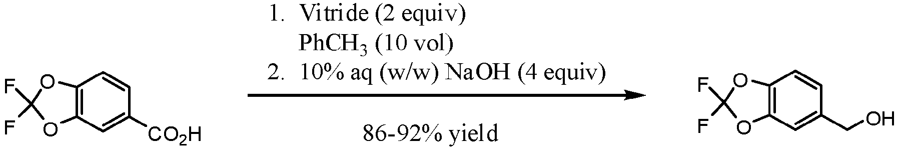

Commercially available 2,2-difluoro-1,3-benzodioxole-5-carboxylic acid (1.0 eq) was slurried in toluene (10 vol). Vitride^{®} (2 eq) was added via addition funnel at a rate to maintain the temperature at 15-25 °C. At the end of the addition, the temperature was increased to 40 °C for 2 hours (h), then 10% (w/w) aqueous (aq) NaOH (4.0 eq) was carefully added via addition funnel, maintaining the temperature at 40-50 °C. After stirring for an additional 30 minutes (min), the layers were allowed to separate at 40 °C. The organic phase was cooled to 20 °C, then washed with water (2 x 1.5 vol), dried (Na₂SO₄), filtered, and concentrated to afford crude (2,2-difluoro-1,3-benzodioxol-5-yl)-methanol that was used directly in the next step.

### Step 2: 5-Chloromethyl-2,2-difluoro-1,3-benzodioxole

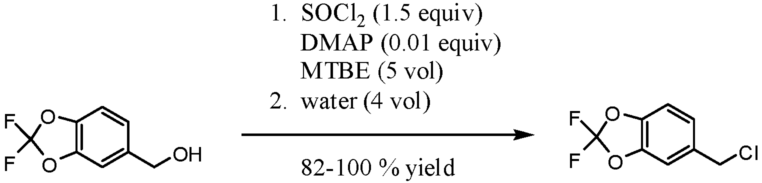

(2,2-Difluoro-1,3-benzodioxol-5-yl)-methanol (1.0 eq) was dissolved in MTBE (5 vol). A catalytic amount of 4-(N,N-dimethyl)aminopyridine (DMAP) (1 mol %) was added and SOCl₂ (1.2 eq) was added via addition funnel. The SOCl₂ was added at a rate to maintain the temperature in the reactor at 15-25 °C. The temperature was increased to 30 °C for 1 h, and then was cooled to 20 °C. Water (4 vol) was added via addition funnel while maintaining the temperature at less than 30 °C. After stirring for an additional 30 min, the layers were allowed to separate. The organic layer was stirred and 10% (w/v) aq NaOH (4.4 vol) was added. After stirring for 15 to 20 min, the layers were allowed to separate. The organic phase was then dried (Na₂SO₄), filtered, and concentrated to afford crude 5-chloromethyl-2,2-difluoro-1,3-benzodioxole that was used directly in the next step.

### Step 3: (2,2-Difluoro-1,3-benzodioxol-5-yl)-acetonitrile

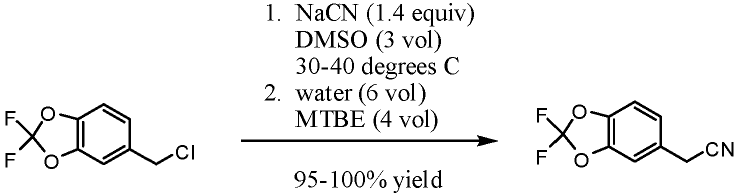

A solution of 5-chloromethyl-2,2-difluoro-1,3-benzodioxole (1 eq) in DMSO (1.25 vol) was added to a slurry of NaCN (1.4 eq) in DMSO (3 vol), while maintaining the temperature between 30-40 °C. The mixture was stirred for 1 h, and then water (6 vol) was added, followed by methyl tert-butyl ether (MTBE) (4 vol). After stirring for 30 min, the layers were separated. The aqueous layer was extracted with MTBE (1.8 vol). The combined organic layers were washed with water (1.8 vol), dried (Na₂SO₄), filtered, and concentrated to afford crude (2,2-difluoro-1,3-benzodioxol-5-yl)-acetonitrile (95%) that was used directly in the next step. ¹H NMR (500 MHz, DMSO) δ 7.44 (br s, 1H), 7.43 (d, *J* = 8.4 Hz, 1H), 7.22 (dd, *J* = 8.2, 1.8 Hz, 1H), 4.07 (s, 2H).

### Step 3b: Alternate Synthesis of (2,2-difluoro-1,3-benzodioxol-5-yl)-1-ethylacetate-acetonitrile

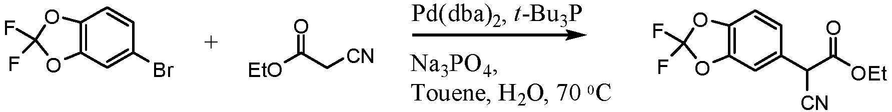

A reactor was purged with nitrogen and charged with toluene (900 mL). The solvent was degassed via nitrogen sparge for no less than 16 hours. To the reactor was then charged Na₃PO₄ (155.7 g, 949.5 mmol), followed by bis(dibenzylideneacetone) palladium (0) (7.28 g, 12.66 mmol). A 10% w/w solution of *tert*-butylphosphine in hexanes (51.23 g, 25.32 mmol) was charged over 10 minutes at 23 °C from a nitrogen purged addition funnel. The mixture was allowed to stir for 50 minutes, at which time 5-bromo-2,2-difluoro-1,3-benzodioxole (75 g, 316.5 mmol) was added over 1 minute. After stirring for an additional 50 minutes, the mixture was charged with ethyl cyanoacetate (71.6 g, 633.0 mmol) over 5 minutes, followed by water (4.5 mL) in one portion. The mixture was heated to 70 °C over 40 minutes and analyzed by HPLC every 1 to 2 hours for the percent conversion of the reactant to the product. After complete conversion was observed (typically 100% conversion after 5 to 8 hours), the mixture was cooled to 20 to 25 °C and filtered through a celite pad. The celite pad was rinsed with toluene (2 X 450 mL), and the combined organics were concentrated to 300 mL under vacuum at 60 to 65 °C. The concentrate was charged with DMSO (225mL) and concentrated under vacuum at 70 to 80 °C until active distillation of the solvent ceased. The solution was cooled to 20 to 25 °C and diluted to 900 mL with DMSO in preparation for Step 3c. ¹H NMR (500 MHz, CDCl₃) δ 7.16 - 7.10 (m, 2H), 7.03 (d, *J* = 8.2 Hz, 1H), 4.63 (s, 1H), 4.19 (m, 2H), 1.23 (t, *J* = 7.1 Hz, 3H).

### Step 3c: Alternate Synthesis of (2,2-difluoro-1,3-benzodioxol-5-yl)-acetonitrile

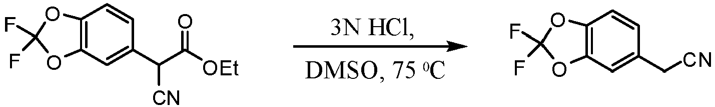

The DMSO solution of (2,2-difluoro-1,3-benzodioxol-5-yl)-1-ethylacetate-acetonitrile from above was charged with 3 N HCl (617.3 mL, 1.85 mol) over 20 minutes while maintaining an internal temperature less than 40 °C. The mixture was then heated to 75 °C over 1 hour and analyzed by HPLC every 1 to 2 hours for percent conversion. When a conversion of greater than 99% was observed (typically after 5 to 6 hours), the reaction was cooled to 20 to 25 °C and extracted with MTBE (2 X 525 mL), with sufficient time to allow for complete phase separation during the extractions. The combined organic extracts were washed with 5% NaCl (2 X 375 mL). The solution was then transferred to equipment appropriate for a 1.5 to 2.5 Torr vacuum distillation that was equipped with a cooled receiver flask. The solution was concentrated under vacuum at less than 60 °C to remove the solvents. (2,2-Difluoro-1,3-benzodioxol-5-yl)-acetonitrile was then distilled from the resulting oil at 125 to 130 °C (oven temperature) and 1.5 to 2.0 Torr. (2,2-Difluoro-1,3-benzodioxol-5-yl)-acetonitrile was isolated as a clear oil in 66% yield from 5-bromo-2,2-difluoro-1,3-benzodioxole (2 steps) and with an HPLC purity of 91.5% AUC (corresponds to a w/w assay of 95%). ¹H NMR (500 MHz, DMSO) δ 7.44 (br s, 1H), 7.43 (d, *J* = 8.4 Hz, 1H), 7.22 (dd, *J* = 8.2, 1.8 Hz, 1H), 4.07 (s, 2H).

### Step 4: (2,2-Difluoro-1,3-benzodioxol-5-yl)-cyclopropanecarbonitrile

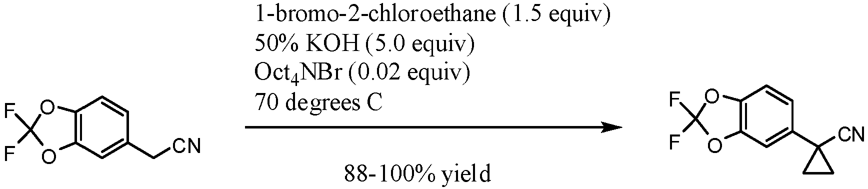

A mixture of (2,2-difluoro-1,3-benzodioxol-5-yl)-acetonitrile (1.0 eq), 50 wt % aqueous KOH (5.0 eq) 1-bromo-2-chloroethane (1.5 eq), and Oct₄NBr (0.02 eq) was heated at 70 °C for 1 h. The reaction mixture was cooled, then worked up with MTBE and water. The organic phase was washed with water and brine. The solvent was removed to afford (2,2-difluoro-1,3-benzodioxol-5-yl)-cyclopropanecarbonitrile. 1H NMR (500 MHz, DMSO) δ 7.43 (d, J = 8.4 Hz, 1H), 7.40 (d, J = 1.9 Hz, 1H), 7.30 (dd, J = 8.4, 1.9 Hz, 1H), 1.75 (m, 2H), 1.53 (m, 2H).

### Step 5: 1-(2,2-Difluoro-1,3-benzodioxol-5-yl)-cyclopropanecarboxylic acid

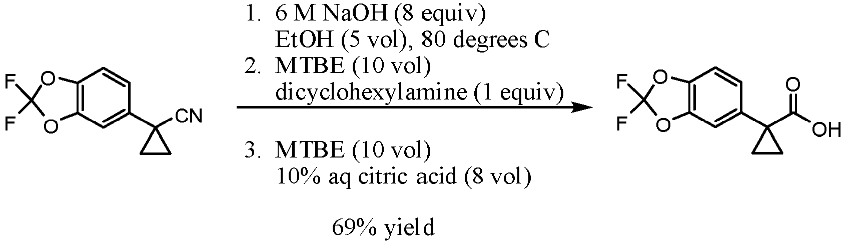

(2,2-Difluoro-1,3-benzodioxol-5-yl)-cyclopropanecarbonitrile was hydrolyzed using 6 M NaOH (8 equiv) in ethanol (5 vol) at 80 °C overnight. The mixture was cooled to room temperature and the ethanol was evaporated under vacuum. The residue was taken up in water and MTBE, 1 M HCl was added, and the layers were separated. The MTBE layer was then treated with dicyclohexylamine (DCHA) (0.97 equiv). The slurry was cooled to 0 °C, filtered and washed with heptane to give the corresponding DCHA salt. The salt was taken into MTBE and 10% citric acid and stirred until all the solids had dissolved. The layers were separated and the MTBE layer was washed with water and brine. A solvent swap to heptane followed by filtration gave 1-(2,2-difluoro-1,3-benzodioxol-5-yl)-cyclopropanecarboxylic acid after drying in a vacuum oven at 50 °C overnight. ESI-MS m/z calc. 242.04, found 241.58 (M+1)+; 1H NMR (500 MHz, DMSO) δ 12.40 (s, 1H), 7.40 (d, J = 1.6 Hz, 1H), 7.30 (d, J = 8.3 Hz, 1H), 7.17 (dd, J = 8.3, 1.7 Hz, 1H), 1.46 (m, 2H), 1.17 (m, 2H).

### Step 6: 1-(2,2-Difluoro-1,3-benzodioxol-5-yl)-cyclopropanecarbonyl chloride

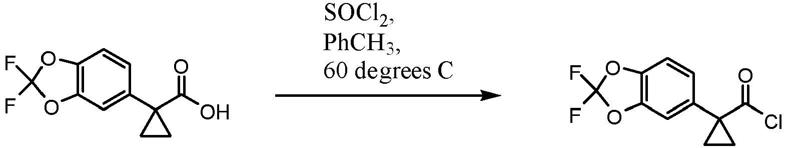

1-(2,2-Difluoro-1,3-benzodioxol-5-yl)-cyclopropanecarboxylic acid (1.2 eq) is slurried in toluene (2.5 vol) and the mixture was heated to 60 °C. SOCl₂ (1.4 eq) was added via addition funnel. The toluene and SOCl₂ were distilled from the reaction mixture after 30 minutes. Additional toluene (2.5 vol) was added and the resulting mixture was distilled again, leaving the product acid chloride as an oil, which was used without further purification.

### Step 7: tert-Butyl-3-(3-methylpyridin-2-yl)benzoate

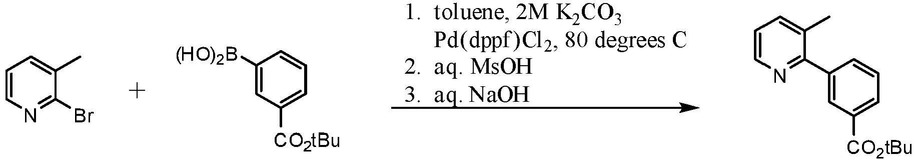

2-Bromo-3-methylpyridine (1.0 eq) was dissolved in toluene (12 vol). K₂CO₃ (4.8 eq) was added, followed by water (3.5 vol). The resulting mixture was heated to 65 °C under a stream of N₂ for 1 hour. 3-(t-Butoxycarbonyl)phenylboronic acid (1.05 eq) and Pd(dppf)Cl₂·CH₂Cl₂ (0.015 eq) were then added and the mixture was heated to 80 °C. After 2 hours, the heat was turned off, water was added (3.5 vol), and the layers were allowed to separate. The organic phase was then washed with water (3.5 vol) and extracted with 10% aqueous methanesulfonic acid (2 eq MsOH, 7.7 vol). The aqueous phase was made basic with 50% aqueous NaOH (2 eq) and extracted with EtOAc (8 vol). The organic layer was concentrated to afford crude tert-butyl-3-(3-methylpyridin-2-yl)benzoate (82%) that was used directly in the next step.

### Step 8: 2-(3-(tert-Butoxycarbonyl)phenyl)-3-methylpyridine-1-oxide

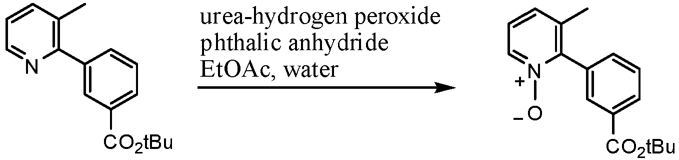

tert-Butyl-3-(3-methylpyridin-2-yl)benzoate (1.0 eq) was dissolved in EtOAc (6 vol). Water (0. 3 vol) was added, followed by urea-hydrogen peroxide (3 eq). Phthalic anhydride (3 eq) was then added portionwise to the mixture as a solid at a rate to maintain the temperature in the reactor below 45 °C. After completion of the phthalic anhydride addition, the mixture was heated to 45 °C. After stirring for an additional 4 hours, the heat was turned off. 10% w/w aqueous Na₂SO₃ (1.5 eq) was added via addition funnel. After completion of Na₂SO₃ addition, the mixture was stirred for an additional 30 min and the layers separated. The organic layer was stirred and 10% wt/wt aqueous. Na₂CO₃ (2 eq) was added. After stirring for 30 minutes, the layers were allowed to separate. The organic phase was washed 13% w/v aq NaCl. The organic phase was then filtered and concentrated to afford crude 2-(3-(tert-butoxycarbonyl)phenyl)-3-methylpyridine-1-oxide (95%) that was used directly in the next step.

### Step 9: tert-Butyl-3-(6-amino-3-methylpyridin-2-yl)benzoate

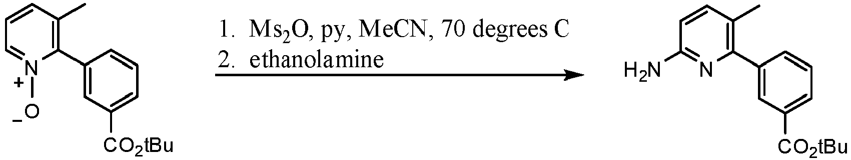

A solution of 2-(3-(tert-butoxycarbonyl)phenyl)-3-methylpyridine-1-oxide (1 eq) and pyridine (4 eq) in acetonitrile (8 vol) was heated to 70 °C. A solution of methanesulfonic anhydride (1.5 eq) in MeCN (2 vol) was added over 50 min via addition funnel while maintaining the temperature at less than 75 °C. The mixture was stirred for an additional 0.5 hours after complete addition. The mixture was then allowed to cool to ambient temperature. Ethanolamine (10 eq) was added via addition funnel. After stirring for 2 hours, water (6 vol) was added and the mixture was cooled to 10 °C. After stirring for 3 hours, the solid was collected by filtration and washed with water (3 vol), 2:1 acetonitrile/water (3 vol), and acetonitrile (2 x 1.5 vol). The solid was dried to constant weight (<1% difference) in a vacuum oven at 50 °C with a slight N₂ bleed to afford tert-butyl-3-(6-amino-3-methylpyridin-2-yl)benzoate as a red-yellow solid (53% yield).

### Step 10: 3-(6-(1-(2,2-Difluorobenzo[d][1,3]dioxol-5-yl)- cyclopropanecarboxamido)-3-methylpyridin-2-yl)-t-butylbenzoate

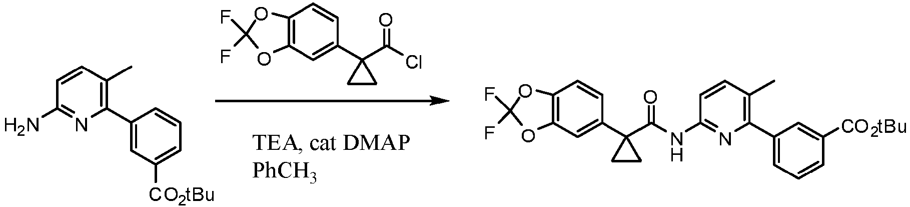

The crude acid chloride described above was dissolved in toluene (2.5 vol based on acid chloride) and added via addition funnel to a mixture of tert-butyl-3-(6-amino-3-methylpyridin-2-yl)benzoate (1 eq), DMAP, (0.02 eq), and triethylamine (3.0 eq) in toluene (4 vol based on tert-butyl-3-(6-amino-3-methylpyridin-2-yl)benzoate). After 2 hours, water (4 vol based on tert-butyl-3-(6-amino-3-methylpyridin-2-yl)benzoate) was added to the reaction mixture. After stirring for 30 minutes, the layers were separated. The organic phase was then filtered and concentrated to afford a thick oil of 3-(6-(1-(2,2-difluorobenzo[d][1,3]dioxol-5-yl) cyclopropanecarboxamido)-3-methylpyridin-2-yl)-t-butylbenzoate (quantitative crude yield). Acetonitrile (3 vol based on crude product) was added and distilled until crystallization occurs. Water (2 vol based on crude product) was added and the mixture stirred for 2 h. The solid was collected by filtration, washed with 1:1 (by volume) acetonitrile/water (2 x 1 volumes based on crude product), and partially dried on the filter under vacuum. The solid was dried to a constant weight (<1% difference) in a vacuum oven at 60 °C with a slight N₂ bleed to afford 3-(6-(1-(2,2-difluorobenzo[d][1,3]dioxol-5-yl) cyclopropanecarboxamido)-3-methylpyridin-2-yl)-t-butylbenzoate as a brown solid.

### Step 11: 3-(6-(1-(2,2-Difluorobenzo[d][1,3]dioxol-5-yl) cyclopropanecarboxamido)-3-methylpyridin-2-yl)benzoic acid • HCl salt

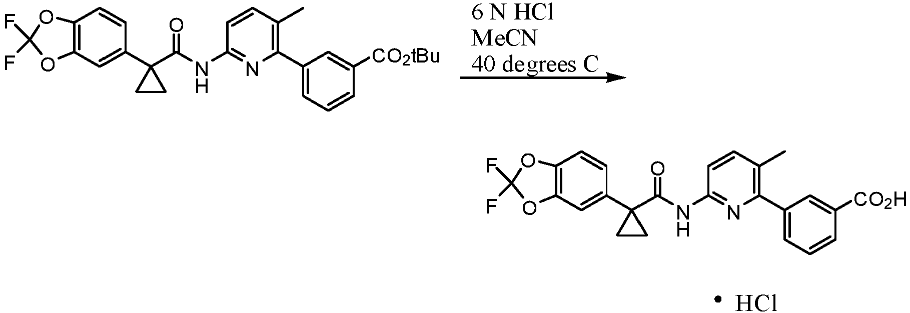

To a slurry of 3-(6-(1-(2,2-difluorobenzo[d][1,3]dioxol-5-yl) cyclopropanecarboxamido)-3-methylpyridin-2-yl)-t-butylbenzoate (1.0 eq) in MeCN (3.0 vol) was added water (0.83 vol) followed by concentrated aqueous HCl (0.83 vol). The mixture was heated to 45 ± 5 °C. After stirring for 24 to 48 h, the reaction was complete, and the mixture was allowed to cool to ambient temperature. Water (1.33 vol) was added and the mixture stirred. The solid was collected by filtration, washed with water (2 x 0.3 vol), and partially dried on the filter under vacuum. The solid was dried to a constant weight (<1% difference) in a vacuum oven at 60 °C with a slight N₂ bleed to afford 3-(6-(1-(2,2-difluorobenzo[d][1,3]dioxol-5-yl) cyclopropanecarboxamido)-3-methylpyridin-2-yl)benzoic acid • HCl as an off-white solid.

**Table 7: Physical data for Compound IV**

| Compound | LC/MS M+ 1 | LC/RT minutes | NMR |
|---|---|---|---|
| Compound **IV** | 453.3 | 1.93 | ¹HNMR (400 MHz, DMSO-d6) 9.14 (s, 1H), 7.99-7.93 (m, 3H), 7.80-7.78 (m,1H), 7.74-7.72 (m,1H), 7.60-7.55 (m,2H), 7.41-7.33 (m,2H), 2.24 (s, 3H), 1.53-1.51 (m, 2H), 1.19-1.17 (m, 2H). |

### Example 6. Preparation of a Tablet containing 5 mg of Compound I

Microcrystalline cellulose is passed through a stainless steel screen (30 mesh) and 210.1 g is charged into a 10L Bohle Bin. Compound **I** is passed through a stainless steel screen (30 mesh) and 210.0g is charged into the 10L Bohle Bin. The bin is sealed and the components are blended for 2 min at a speed of 32 RPM to yield a microcrystalline cellulose/Compound **I** blend. The microcrystalline cellulose/Compound **I** blend is discharged into a stainless steel container. The following materials are sieved through a stainless steel 30 mesh screen and added to the 10 L Bohle bin in this order: lactose (approximately half of 1022.2 g), microcrystalline cellulose (approximately half of 812 g), microcrystalline cellulose/Compound **I** blend, polyvinylpyrrolidone/vinyl acetate (210.1 g), croscarmellose sodium (133 g), microcrystalline cellulose (the remaining half portion from the 812 g amount), and lactose (the remaining half portion from the 1022.2 g amount). The bin is sealed and the components are blended for 18.5 min at a speed of 32 RPM. Sodium stearyl fumarate pruv^{®} is passed through a 60 mesh stainless steel and 53.1 g is charged into the Bohle bin. The bin is sealed and the components are blended for 4 min at a speed of 32 RPM. The bin is tested for homogeneity. The blend is added to a Piccola Tablet press and compressed into tablets weighing 67.0 mg.

**Table 8: Compound I tablet composition**

| **Component** | **% w/w tablet (approx.)** | **Tablet quantity (approx.)** |
|---|---|---|
| Compound **I** (Ca salt) | 8 | 5 mg |
| Microcrystalline cellulose (pre-blend) | 8 | 5 mg |
| polyvinylpyrrolidone/vinyl acetate | 8 | 5 mg |
| Microcrystalline cellulose (tablet-blend) | 31 | 21 mg |
| Lactose monohydrate | 38 | 26 mg |
| croscarmellose sodium | 5 | 3 mg |
| Sodium stearyl fumarate pruv^{®} | 2 | 1 mg |

### Example 7. Bioactivity Assay

### Solutions

Base medium (ADF+++) consisted of Advanced DMEM/Ham's F12, 2 mM Glutamax, 10 mM HEPES, 1µ/ml penicillin/streptomycin.

Intestinal enteroid maintenance medium (IEMM) consisted of ADF+++, 1x B27 supplement, 1x N2 supplement, 1.25 mM N-acetyl cysteine, 10 mM Nicotinamide, 50 ng/mL hEGF, 10 nM Gastrin, 1 µg/mL hR-spondin-1, 100 ng/mL hNoggin, TGF-b type 1 inhibitor A-83-01, 100 µg/mL Primocin, 10 µM P38 MAPK inhibitor SB202190.

Bath 1 Buffer consisted of 1 mM MgCl₂, 160 mM NaCl, 4.5 mM KCl, 10 mM HEPES, 10 mM Glucose, 2 mM CaCl₂.

Chloride Free Buffer consisted of 1 mM Magnesium Gluconate, 2 mM Calcium Gluconate, 4.5 mM Potassium Gluconate, 160 mM Sodium Gluconate, 10 mM HEPES, 10 mM Glucose.

Bath1 Dye Solution consisted of Bath 1 Buffer, 0.04% Pluronic F127, 20 µM Methyl Oxonol, 30 µM CaCCinh-A01, 30 µM Chicago Sky Blue.

Chloride Free Dye Solution consisted of Chloride Free Buffer, 0.04% Pluronic F127, 20 µM Methyl Oxonol, 30 µM CaCCinh-A01, 30 µM Chicago Sky Blue.

Chloride Free Dye Stimulation Solution consisted of Chloride Free Dye Solution, 10 µM forskolin, 100 µM IBMX, and 300 nM Compound **III**.

### Cell Culture

Human intestinal epithelial enteroid cells were obtained from the Hubrecht Institute for Developmental Biology and Stem Cell Research, Utrecht, The Netherlands and expanded in T-Flasks as previously described (Dekkers JF, Wiegerinck CL, de Jonge HR, Bronsveld I, Janssens HM, de Winter-de Groot KM, Brandsma AM, de Jong NWM, Bijvelds MJC, Scholte BJ, Nieuwenhuis EES, van den Brink S, Clevers H, van der Ent CK, Middendorp S and M Beekman JM. A functional CFTR assay using primary cystic fibrosis intestinal organoids. Nat Med. 2013 Jul;19(7):939-45.

### Enteroid Cell Harvesting and Seeding

Cells were recovered in cell recovery solution, collected by centrifugation at 650 rpm for 5 min at 4°C, resuspended in TryPLE and incubated for 5 min at 37°C. Cells were then collected by centrifugation at 650 rpm for 5 min at 4°C and resuspended in IEMM containing 10 µM ROCK inhibitor (RI). The cell suspension was passed through a 40 µm cell strainer and resuspended at 1x106 cells/mL in IEMM containing 10 µM RI. Cells were seeded at 5000 cells/well into multi-well plates and incubated for overnight at 37°C, 95% humidity and 5% CO₂ prior to assay.

### Membrane Potential Dye Assay

Enteroid cells were incubated with test compound in IEMM for 18-24 hours at 37°C, 95% humidity and 5% CO₂. Following compound incubations, a membrane potential dye assay was employed using a FLIPR Tetra to directly measure the potency and efficacy of the test compound on CFTR-mediated chloride transport following acute addition of 10 µM forskolin and 300 nM Compound **III.** Briefly, cells were washed 5 times in Bath 1 Buffer. Bath 1 Dye Solution was added and the cells were incubated for 25 min at room temperature. Following dye incubation, cells were washed 3 times in Chloride Free Dye Solution. Chloride transport was initiated by addition of Chloride Free Dye Stimulation Solution and the fluorescence signal was read for 15 min. The CFTR-mediated chloride transport for each condition was determined from the AUC of the fluorescence response to acute forskolin and 300 nM Compound **III** stimulation. Chloride transport was then expressed as a percentage of the chloride transport following treatment with 3 µM Compound **I, 3** µM Compound **II,** and 300 nM acute Compound **III** triple combination control (% Activity).

The following represents the data in Table 9:
Max Activity: +++ is >60%; ++ is 30-60%; + is <30%. EC50: +++ is <1 µM; ++ is 1-3 µM; + is >3 µM; and ND is "not determined."

**Table 9: Assay Data for Compound I**

| **Molecule** | **Max. Activity** | **EC50** |
|---|---|---|
| Compound **I** | +++ | +++ |

### Example 8.

### Compound I Increases Chloride Transport Alone and in Combination With Compound II and/or Compound III in F508del/F508del HBE and F508del/MF HBE

Ussing chamber studies were conducted to measure F508del CFTR mediated chloride transport in HBE cells derived from 3 F508del homozygous donors and 5 F508del/MF donors (G542X, 3 donors; E585X, 1 donor; 3905InsT, 1 donor). As a positive control, maximally effective concentrations of N-(benzenesulfonyl)-6-[3-[2-[1-(trifluoromethyl)cyclopropyl]ethoxy]pyrazol-1-yl]-2-[(4S)-2,2,4-trimethylpyrrolidin-1-yl]pyridine-3-carboxamide (see WO 2018/064632) and N-[(6-amino-2-pyridyl)sulfonyl]-6-(3-fluoro-5-isobutoxy-phenyl)-2-[(4S)-2,2,4-trimethylpyrrolidin-1-yl]pyridine-3-carboxamide (see WO 2016/057572) in combination with Compound **II**/Compound **III** were included in each experiment.

In these CF cell lines, CFTR-mediated chloride transport was low in the absence of CFTR modulators, which is consistent with little-to-no CFTR at the cell surface. Treatment with Compound **I** alone for 16 to 24 hours caused a modest increase in chloride transport in both F508del/F508del HBE and F508del/MF HBE cells. The combination of Compound **I** and Compound **II** further increased chloride transport when compared to Compound **I** alone and was similar to Compound **II**/Compound **III.** Addition of Compound **III** strongly potentiated chloride transport in the presence of Compound **I** or in combination with Compound **I**/Compound **II.** Synergy analyses showed that the effect of Compound **I** was highly synergistic with a fixed concentration of Compound **III** or Compound **II**/Compound **III** and was modestly synergistic with a fixed combination of Compound **II.** At most Compound **I** concentrations, Compound **I**/Compound **II**/Compound **III** increased chloride transport more than Compound **I**/Compound **II** or Compound **I**/Compound **III.** However, the efficacy of Compound **I**/Compound **III** and Compound **I**/Compound **II**/Compound **III** was similar at their respective EC90 values. The respective EC90 values under conditions that maximally activate CFTR for Compound **I**/Compound **III** and Compound **I**/Compound **II/** Compound **III** were 0.848 µM and 0.152 µM in F508del/F508del HBE and 1.15 µM and 0.122 µM in F508del/MF HBE.

Following a single oral administration of Compound **I** in male animals, Compound **I** mean tₘₐₓ values were 9 hours in rats, 4 hours in dogs, and 3 hours in monkeys. Mean oral bioavailability (F) was low to moderate in rats (76.9%), dogs (49.7%), and monkeys (12.9%).

### Compound I Pharmacokinetic Parameters Following a Single Oral Administration of Compound I in Male Rats, Dogs, and Monkeys

| **Species** | **Nominal Dose (mg/kg)** | **AUC_{0-∞} (µg•h/mL)** | **Cₘₐₓ (µg/mL)** | **tₘₐₓ (h)** | **t_{1/2} (h)** | **F (%)** |
|---|---|---|---|---|---|---|
| Rat | 3 | 31.9 ± 11.1 | 1.10 ± 0.337 | 9.33 ± 2.31 | 22.6 ± 2.83 | 76.9 |
| Dog | 1 | 38.5 ± 4.70 | 2.44 ± 0.178 | 4.00 ± 0.00 | 11.1 ± 1.09 | 49.7 |
| Monkey | 1 | 0.795 ± 0.233 | 0.102 ± 0.0132 | 3.33 ± 1.15 | 3.07 ± 1.16 | 12.9 |

| | | | | | | |
|---|---|---|---|---|---|---|
| Note: Data are presented as mean ± SD (n = 3). | | | | | | |

As the dose increased, systemic exposure of Compound **I** increased in a more than dose-proportional manner in rats and dogs. Dose-normalized exposure was higher in female rats than male rats. In dogs, systemic exposures to Compound **I** were similar in both sexes. Following repeated oral administration of Compound **I** for 28 days in rats and dogs, accumulation of Compound **I** exposure was observed. Systemic exposure to Compound **I** on Day 28 was higher than on Day 1 (Day 28/Day 1 AUC₀₋₂₄ₕ ratio ranged from 1.63 to 2.70 in male rats, 5.01 to 8.26 in female rats, 1.73 to 2.64 in male dogs, and 1.82 to 2.23 in female dogs).

### Example 9. Safety and Efficacy Study of Compound I

A safety analysis of an ongoing clinical study was performed for 37 subjects in Cohorts A1 to A5, 33 subjects in Cohort B, and 17 subjects in Cohort C, who were exposed to at least 1 dose of study drug (Compound **I** or placebo) as a monotherapy and as part of a triple combination with Compound **II** or Compound **III.** Compound **I** was generally safe and well-tolerated up to a dose of 60 mg qd in monotherapy and 20 mg qd in triple combination with Compound **II** and Compound **III.**

### Planned Efficacy studies

This is a Phase 2, 3-part, randomized, double-blind, placebo- and TEZ/IVA-controlled, proof-of-concept study of Compound **I.** Study parts may be conducted in parallel or sequentially. Randomization will be stratified by ppFEV₁.

### Part 1: Subjects with F508del/MF genotypes

Part 1 evaluates Compound **I** in triple combination with Compound **II** and Compound **III-d** as shown in the Table below.
The Washout Period (18 ± 3 days) is included to enable a more thorough evaluation of Compound **I** exposure-response relationships by conducting PK and PD assessments during the Compound **I** washout.

### Planned Doses

• The planned Compound **I** doses of 5 mg, 10 mg, and 20 mg qd may be adjusted based on emerging data.
• The dosage of Compound **II** will be 100 mg qd (once daily) and Compound **III-d** will be 150 mg qd.

| Treatment Period (4 weeks) | | Washout Period (18 ± 3 days) | | |
|---|---|---|---|---|
| Screening Period (4 weeks) | TC-20 mg (Cpd I/Cpd II/Cpd III-d) | N = 18 | Cpd II/Cpd III-d | |
| | TC-10 mg (Cpd I/Cpd II/Cpd III-d) | N = 18 | | Safety Follow-up Period (4 weeks) |
| | TC-5 mg (Cpd I/Cpd II/Cpd III-d) | N=9 | | |
| | Triple Placebo | N=9 | Dual Pbo | |

### Part 2 (Optional): Subjects with F508del/ F508del genotype

All subjects are required to complete the Compound **II**/ Compound **III** Run-in Period to establish a reliable on-treatment (Cpd **II**/Cpd **III**) baseline. The 4-week Washout Period is included to evaluate the effect on PD and efficacy endpoints as subjects step down from triple combination to Cpd **II**/Cpd **III,** after the Compound **I** washout.

### Planned Doses

- Part 2 will evaluate the same Compound **I** dose (20 mg qd) used in Part 1. This dose may be adjusted downward based on emerging data from Part D of Study 001.
- The dosage of Compound **II** will be 100 mg qd and Compound **III-d** will be 150 mg qd.
- The dosage of Compound **II**/Compound **III** will be Compound **II** 100 mg qd/ Compound **III** 150 mg every 12 hours (q12h)

| | Run-in Period (4 weeks) | Treatment Period (4 weeks) | | Washout Period (4 weeks) | |
|---|---|---|---|---|---|
| Screening Period (4 weeks) | Cpd II/Cpd III | TC-20 mg (Cpd I/Cpd II/Cpd III-d) | N = 18 | Cpd II/Cpd III | Safety Follow-up Period (4 weeks) |
| | | Placebo + Cpd II/Cpd III | N = 9 | | |

### Part 3 (Optional): Subjects with F508del/MF genotypes

The Washout Period (18 ± 3 days) is included to enable a more thorough evaluation of Compound **I** exposure-response relationships by conducting PK and PD assessments during the Compound **I** washout.

### Planned Doses

• Part 3 will evaluate the same Compound **I** dose (20 mg qd) used in Part 1. This dose may be adjusted based on emerging data.
• The dosage of Compound **II**/Compound **III** will be Compound **II** 100 mg qd/ Compound **III** 150 mg every 12 hours (q12h)

| | Treatment Period (4 weeks) | Washout Period (18 ± 3 days) | | |
|---|---|---|---|---|
| Screening Period (4 weeks) | TC-20 mg (Cpd I/Cpd II/Cpd III) | N = 18 | Cpd II/Cpd III | Safety Follow-up Period (4 weeks) |
| | Triple Placebo | N=9 | Dual Pbo | |

### Other Embodiments

The foregoing discussion discloses and describes merely exemplary embodiments of this disclosure. One skilled in the art will readily recognize from such discussion and from the accompanying drawings and claims, that various changes, modifications and variations can be made therein.

## Claims

1. A pharmaceutical composition comprising about 5 mg to about 20 mg of at least one compound chosen from Compound **I** 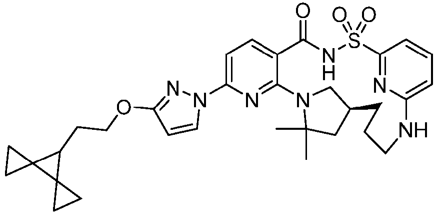
and pharmaceutically acceptable salts thereof, and optionally comprising one or more additional CFTR modulating agents,
wherein "about" means within 20% of the given value or range.

2. The pharmaceutical composition of claim 1 wherein the composition comprises about 5 mg, about 10 mg, or about 20 mg of at least one compound chosen from Compound **I** 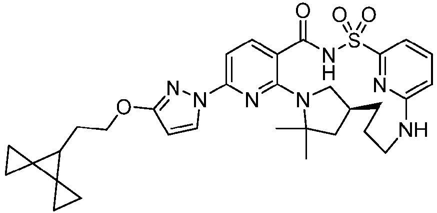 and pharmaceutically acceptable salts thereof.

3. The pharmaceutical composition of claim 1 or claim 2 wherein the composition comprises about 5 mg of at least one compound chosen from Compound **I** 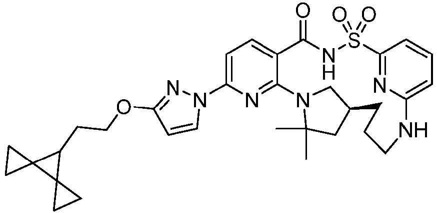 and pharmaceutically acceptable salts thereof.

4. The pharmaceutical composition of any one of the preceding claims, wherein the one or more additional CFTR modulating agents comprise at least one compound chosen from Compound **II:** 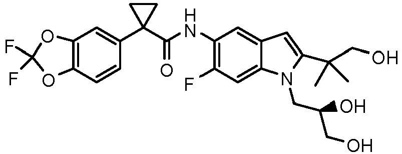 and pharmaceutically acceptable salts thereof.

5. The pharmaceutical composition of any one of the preceding claims, wherein the one or more additional CFTR modulating agents comprise at least one compound chosen from Compound **III-d:** 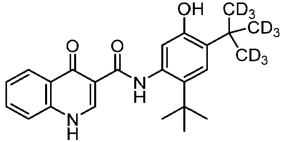 and pharmaceutically acceptable salts thereof.

6. The pharmaceutical composition of any one of the preceding claims, wherein the one or more additional CFTR modulating agents comprise Compound **II** and Compound **III-d.**

7. The pharmaceutical composition of any one of the preceding claims, wherein the pharmaceutical composition is a tablet.

8. The pharmaceutical composition of any one of the preceding claims for use in a method of treating cystic fibrosis.

9. The pharmaceutical composition for use according to claim 8, wherein Compound **I** or a pharmaceutically acceptable salt thereof is administered:
(a) as a single dose, once daily; or
(b) in two doses daily.

10. The pharmaceutical composition for use according to any one of claims 8-9, wherein 50 mg to 150 mg of Compound **II** or an equivalent amount of a pharmaceutically acceptable salt thereof is administered daily.

11. The pharmaceutical composition for use according to any one of claims 8-10, wherein 100 mg of Compound **II** or an equivalent amount of a pharmaceutically acceptable salt thereof is administered daily.

12. The pharmaceutical composition for use according to any one of claims 8-11, wherein 50 mg to 150 mg of Compound **III-d** or an equivalent amount of a pharmaceutically acceptable salt thereof is administered daily.

13. The pharmaceutical composition for use according to any one of claims 8-12, wherein 150 mg of Compound **III-d** or an equivalent amount of a pharmaceutically acceptable salt thereof is administered daily.

14. The pharmaceutical composition for use claim 8 wherein the method of treating cystic fibrosis comprises administering daily to a patient in need thereof:
(a) 50 mg to 100 mg of Compound **II** or an equivalent amount of a pharmaceutically acceptable salt thereof; and
(b) 50 mg to 150 mg of Compound **III-d** or an equivalent amount of a pharmaceutically acceptable salt thereof.

15. The pharmaceutical composition for use according to any one of claims 8-14, wherein the patient is:
(a) heterozygous for the F508del CFTR mutation; or
(b) homozygous for the F508del CFTR mutation.

16. The pharmaceutical composition for use of any one of claims 8-15, wherein the method comprises administering a pharmaceutically acceptable salt of Compound **I**.

## Patentansprüche

1. Pharmazeutische Zusammensetzung, umfassend etwa 5 mg bis etwa 20 mg mindestens einer Verbindung, die aus Verbindung I 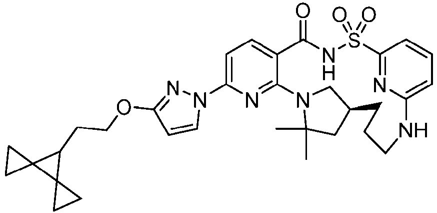
und pharmazeutisch unbedenklichen Salzen davon ausgewählt ist, und gegebenenfalls umfassend ein oder mehrere zusätzliche CFTR-modulierende Mittel,
wobei "etwa" innerhalb von 20 % des angegebenen Werts oder Bereichs bedeutet.

2. Pharmazeutische Zusammensetzung nach Anspruch 1, wobei die Zusammensetzung etwa 5 mg, etwa 10 mg oder etwa 20 mg mindestens einer Verbindung, die aus Verbindung I 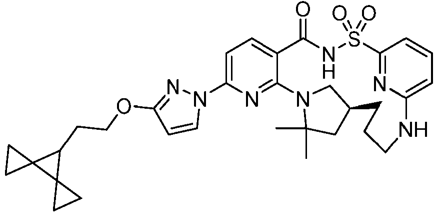 und pharmazeutisch unbedenklichen Salzen davon ausgewählt ist, umfasst.

3. Pharmazeutische Zusammensetzung nach Anspruch 1 oder Anspruch 2, wobei die Zusammensetzung etwa 5 mg mindestens einer Verbindung, die aus Verbindung I 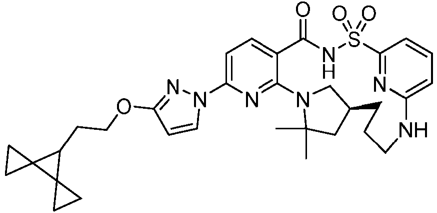 und pharmazeutisch unbedenklichen Salzen davon ausgewählt ist, umfasst.

4. Pharmazeutische Zusammensetzung nach einem der vorhergehenden Ansprüche, wobei das eine oder die mehreren zusätzlichen CFTR-modulierenden Mittel mindestens eine Verbindung, die aus Verbindung II: 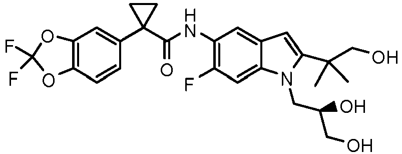 und pharmazeutisch unbedenklichen Salzen davon ausgewählt ist, umfasst.

5. Pharmazeutische Zusammensetzung nach einem der vorhergehenden Ansprüche, wobei das eine oder die mehreren zusätzlichen CFTR-modulierenden Mittel mindestens eine Verbindung, die aus Verbindung III-d: 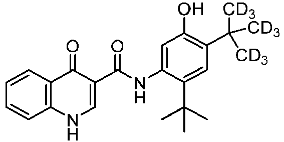 und pharmazeutisch unbedenklichen Salzen davon ausgewählt ist, umfasst.

6. Pharmazeutische Zusammensetzung nach einem der vorhergehenden Ansprüche, wobei das eine oder die mehreren zusätzlichen CFTR-modulierenden Mittel Verbindung II und Verbindung III-d umfassen.

7. Pharmazeutische Zusammensetzung nach einem der vorhergehenden Ansprüche, wobei es sich bei der pharmazeutischen Zusammensetzung um eine Tablette handelt.

8. Pharmazeutische Zusammensetzung nach einem der vorhergehenden Ansprüche zur Verwendung bei einem Verfahren zur Behandlung von zystischer Fibrose.

9. Pharmazeutische Zusammensetzung zur Verwendung nach Anspruch 8, wobei Verbindung I oder ein pharmazeutisch unbedenkliches Salz davon
(a) als Einzeldosis einmal täglich oder
(b) in zwei Dosen täglich verabreicht wird.

10. Pharmazeutische Zusammensetzung zur Verwendung nach einem der Ansprüche 8-9, wobei 50 mg bis 150 mg Verbindung II oder eine äquivalente Menge eines pharmazeutisch unbedenklichen Salzes davon täglich verabreicht werden bzw. wird.

11. Pharmazeutische Zusammensetzung zur Verwendung nach einem der Ansprüche 8-10, wobei 100 mg Verbindung II oder eine äquivalente Menge eines pharmazeutisch unbedenklichen Salzes davon täglich verabreicht werden bzw. wird.

12. Pharmazeutische Zusammensetzung zur Verwendung nach einem der Ansprüche 8-11, wobei 50 mg bis 150 mg Verbindung III-d oder eine äquivalente Menge eines pharmazeutisch unbedenklichen Salzes davon täglich verabreicht werden bzw. wird.

13. Pharmazeutische Zusammensetzung zur Verwendung nach einem der Ansprüche 8-12, wobei 150 mg Verbindung III-d oder eine äquivalente Menge eines pharmazeutisch unbedenklichen Salzes davon täglich verabreicht werden bzw. wird.

14. Pharmazeutische Zusammensetzung zur Verwendung nach Anspruch 8, wobei das Verfahren zur Behandlung von zystischer Fibrose das tägliche Verabreichen von Folgendem an einen Patienten, bei dem diesbezüglicher Bedarf besteht, umfasst:
(a) 50 mg bis 100 mg Verbindung II oder einer äquivalenten Menge eines pharmazeutisch unbedenklichen Salzes davon; und
(b) 50 mg bis 150 mg Verbindung III-d oder einer äquivalenten Menge eines pharmazeutisch unbedenklichen Salzes davon.

15. Pharmazeutische Zusammensetzung zur Verwendung nach einem der Ansprüche 8-14, wobei der Patient:
(a) für die F508del-CFTR-Mutation heterozygot oder
(b) für die F508del-CFTR-Mutation homozygot ist.

16. Pharmazeutische Zusammensetzung zur Verwendung nach einem der Ansprüche 8-15, wobei das Verfahren das Verabreichen eines pharmazeutisch unbedenklichen Salzes von Verbindung I umfasst.

## Revendications

1. Composition pharmaceutique comprenant environ 5 mg à environ 20 mg d'au moins un composé choisi parmi le composé I 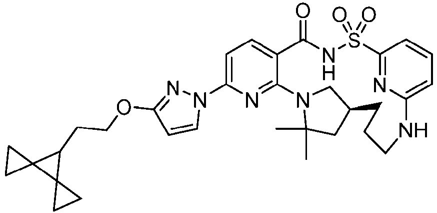
et ses sels pharmaceutiquement acceptables, et comprenant éventuellement un ou plusieurs agents modulateurs de CFTR supplémentaires,
« environ » signifiant dans les 20 % de la valeur ou de la plage donnée.

2. Composition pharmaceutique selon la revendication 1, dans laquelle la composition comprend environ 5 mg, environ 10 mg ou environ 20 mg d'au moins un composé choisi parmi le composé I 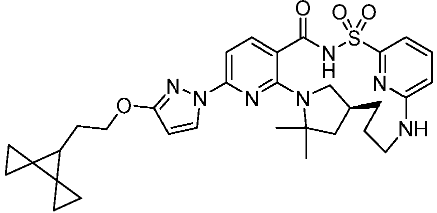 et des sels pharmaceutiquement acceptables de celui-ci.

3. Composition pharmaceutique selon la revendication 1 ou la revendication 2, dans laquelle la composition comprend environ 5 mg d'au moins un composé choisi parmi le composé I 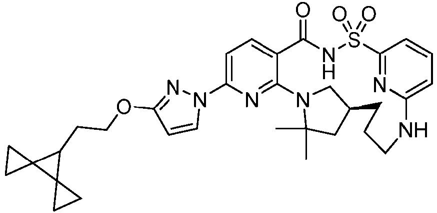 et des sels pharmaceutiquement acceptables de celui-ci.

4. Composition pharmaceutique selon l'une quelconque des revendications précédentes, dans laquelle le ou les agents modulateurs de CFTR supplémentaires comprennent au moins un composé choisi parmi le composé II : 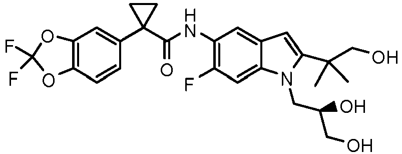 et des sels pharmaceutiquement acceptables de celui-ci.

5. Composition pharmaceutique selon l'une quelconque des revendications précédentes, dans laquelle le ou les agents modulateurs de CFTR supplémentaires comprennent au moins un composé choisi parmi le composé III-d : 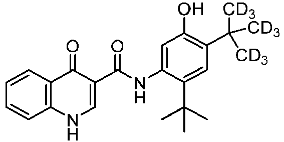 et des sels pharmaceutiquement acceptables de celui-ci.

6. Composition pharmaceutique selon l'une quelconque des revendications précédentes, dans laquelle le ou les agents modulateurs de CFTR supplémentaires comprennent le composé II et le composé III-d.

7. Composition pharmaceutique selon l'une quelconque des revendications précédentes, dans laquelle la composition pharmaceutique est un comprimé.

8. Composition pharmaceutique selon l'une quelconque des revendications précédentes pour une utilisation dans un procédé de traitement de la mucoviscidose chez un patient.

9. Composition pharmaceutique destinée à être utilisée selon la revendication 8, dans laquelle le composé I ou un sel pharmaceutiquement acceptable de celui-ci est administré :
(a) en une dose unique, une fois par jour ; ou
(b) en deux doses quotidiennement.

10. Composition pharmaceutique destinée à être utilisée selon l'une quelconque des revendications 8 à 9, dans laquelle 50 mg à 150 mg du composé II ou une quantité équivalente d'un sel pharmaceutiquement acceptable de celui-ci sont administrés quotidiennement.

11. Composition pharmaceutique destinée à être utilisée selon l'une quelconque des revendications 8 à 10, dans laquelle 100 mg du composé II ou une quantité équivalente d'un sel pharmaceutiquement acceptable de celui-ci sont administrés quotidiennement.

12. Composition pharmaceutique destinée à être utilisée selon l'une quelconque des revendications 8 à 11, dans laquelle 50 mg à 150 mg du composé III-d ou une quantité équivalente d'un sel pharmaceutiquement acceptable de celui-ci sont administrés quotidiennement.

13. Composition pharmaceutique destinée à être utilisée selon l'une quelconque des revendications 8 à 12, dans laquelle 150 mg du composé III-d ou une quantité équivalente d'un sel pharmaceutiquement acceptable de celui-ci sont administrés quotidiennement.

14. Composition pharmaceutique pour utilisation selon la revendication 8, dans laquelle le procédé de traitement de la mucoviscidose comprend l'administration quotidienne à un patient qui en a besoin de :
(a) 50 mg à 100 mg du composé II ou une quantité équivalente d'un sel pharmaceutiquement acceptable de celui-ci ; et
(b) 50 mg à 150 mg du composé III-d ou une quantité équivalente d'un sel pharmaceutiquement acceptable de celui-ci.

15. Composition pharmaceutique pour utilisation selon l'une quelconque des revendications 8 à 14, le patient étant :
(a) hétérozygote pour la mutation de CFTR F508del ; ou
(b) homozygote pour la mutation de CFTR F508del .

16. Composition pharmaceutique destinée à être utilisée selon l'une quelconque des revendications 8 à 15, dans laquelle le procédé comprend l'administration d'un sel pharmaceutiquement acceptable du composé I.
